# EUROPEAN PATENT SPECIFICATION

(11) **EP 1 964 929 B1**
(45) Date of publication and mention of the grant of the patent: **15.02.2012**
(21) Application number: 06841799.7
(22) Date of filing: 12.12.2006
(51) Int. Cl.: C12Q 1/68, G01N 33/58

(54) **HLA-B GENOTYPING METHOD AND KIT BASED ON REAL-TIME PCR**
HLA-B-GENOTYPISIERUNGSVERFAHREN UND -KIT AUF ECHTZEIT-PCR-BASIS
PROCEDE ET KIT DE GENOTYPAGE HLA-B PAR PCR EN TEMPS REEL

(30) Priority: 13.12.2005 ES 200503110
(43) Date of publication of application: 03.09.2008
(73) Proprietor: Universitat Autònoma de Barcelona, 08193 Bellaterra (ES); Banc de Sang i Teixits, E-08035 Barcelona (ES)
(72) Inventor: JUAN OTERO, Manuel, E-08940 Cornellà de Llobregat (Barcelona) (ES); CASAMITJANA PONCES, Natàlia, E-08005 Barcelona (ES); PALOU RIVERA, Eduardo, E-08012 Barcelona (ES); PUJOL BORRELL, Ricardo, E-08005 Barcelona (ES); FANER CANET, María Rosa, E-08035 Barcelona (ES)
(74) Representative: ZBM Patents
(86) International application number: PCT/ES2006/070192
(87) International publication number: WO 2007/068782

(56) References cited:
- WO-A1-2005/059174
- WO-A2-2005/059505
- WO-A2-2005/059505
- US-A- 5 334 499
- CASAMITJANA N ET AL: "Development of a new HLA-DRB real-time PCR typing method" HUMAN IMMUNOLOGY, NEW YORK, NY, US LNKD- DOI:10.1016/J.HUMIMM.2004.08.178, vol. 66, no. 1, 16 September 2004 (2004-09-16), pages 85-91, XP004695148 ISSN: 0198-8859
- GELSTHORPE AR ET AL: "HIGH-THROUGHPUT CLASSI HLA GENOTYPING USING FLUORESCENCE RESONANCE ENERGY TRANSFER (FRET) PROBES ANDSEQUENCE-SPECIFIC PRIMER-POLYMERASE CHAIN REACTION (SSP-PCR)" TISSUE ANTIGENS, MUNKSGAARD, COPENHAGEN, DK LNKD- DOI:10.1034/J.1399-0039.1999.540611.X, vol. 54, no. 6, 1 January 1999 (1999-01-01), pages 603-614, XP008072919 ISSN: 0001-2815
- FANER ET AL: "Real-Time PCR Using Fluorescent Resonance Emission Transfer Probes for HLA-B Typing" HUMAN IMMUNOLOGY, NEW YORK, NY, US LNKD- DOI:10.1016/J.HUMIMM.2006.02.038, vol. 67, no. 4-5, 7 April 2006 (2006-04-07), pages 374-385, XP005446150 ISSN: 0198-8859
- FANER R. ET AL.: 'HLA-B27 Genotyping by Fluorescent Resonance Emission Transfer (FRET) Probes in Real-Time PCR' HUMAN IMMUNOL. vol. 65, no. 8, August 2004, pages 826 - 838, XP004546057
- BAST G.: 'FOCIS Abstract Supplement' CLINICAL IMMUNOL. no. SUPPL. 1, April 2005, pages S3 - S282, XP004863152
- FANER R. ET AL.: 'Real-Time PCR Using Fluorescent Resonance Emission Transfer Probes for HLA-B Typing' HUMAN IMMUNOL. vol. 67, April 2006 - May 2006, pages 374 - 385, XP005446150
- NEOH S-H. ET AL.: 'Rapid Detection of the Factor V Leiden (1691G>A) and Haemochromatosis (845G>A) Mutation by Fluorescence Resonance Energy Transfer (FRET) and Real-Time PCR' J. CLIN. PATHOL. vol. 52, 1999, pages 766 - 769, XP001016133
- PRADEAU K. ET AL.: 'Multiplex Real-Time PCR Assay for Simultaneous Quantitation of Human Cytomegalovirus and Herpesvirus-6 in Polymorphonuclear and Mononuclear Cells of Transplant Recipients' J. VIROL. METHODS vol. 132, 2006, pages 77 - 84, XP005287826

## Description

This invention is related to the field of medicine in general and, specifically, to medical research, molecular biology, immunology, forensic medicine and diagnostics. In particular, the invention provides a method for determining the genotype (that is, the set of specific alleles) of an individual.

### BACKGROUND ART

In transplants between immunogenetically different individuals, immunity is established in the transplant and a rejection reaction against the graft is induced. There are antigens which induce a particularly intense immunity in the transplant as targets of this reaction, which are called major histocompatibility antigens. These antigens are controlled by a group of genes called major histocompatibility complex (MHC), known in humans as the human leukocyte antigen system (HLA). These genes which encode HLA molecules are amongst the most variable genes in humans: each variant encodes molecules which bind different peptides. These genes are the same in all the cells of a particular individual, but differ from one person to another. It has been extensively proven that HLA matching of patient and unrelated donour significantly reduces transplant rejection and graft-versus-host disease, and, consequently, reduces transplant-related mortality (cf. N. Flomenberg et al., "Impact of HLA class I and class II high-resolution matching on outcomes of unrelated donor bone marrow transplantation: HLA-C mismatching is associated with a strong adverse effect on transplantation outcome", Blood 2004, vol. 104, pp. 1923-30).

HLA comprises several genes grouped in a 4-Mb segment in the short arm of chromosome 6. The HLA region comprises six major loci that structurally encode homologous proteins classified in HLA class I (HLA-A, B, Cw) and class II (HLA-DR, DQ, DP), which present antigens to two different subtypes of cells. HLA class I molecules present antigens to T-cells that express the CD8 cell-surface glycoprotein. HLA glycoproteins are highly polymorphic and, in the case of the HLA-B locus, the polymorphism is mainly located in exons 2 and 3. To date, 699 allelic variants of this locus are known, grouped into 35 serological specificities, which means that this locus is the most polymorphic of HLA loci. The allelic variations of the HLA-B locus are the most important in the selection of the transplant within class I molecules.

HLA genotyping by polymerase chain reaction (PCR) techniques has become an alternative to serological methods that is widely used in clinical practise. The most commonly used PCR-based typing methods are PCR with sequence-specific primers (PCR-SSP) and PCR with sequence-specific oligonucleotides (PCR-SSO). In the PCR-SSP method, the gene sequence is determined by the amplification of the hypervariable region of the target HLA antigen in order to determine the type of HLA antigen (e.g. cf. M. Bunce et al., "Phototyping: comprehensive DNA typing for HLA-A, B, C, DRB1, DRB3, DRB4, DRB5 & DQB1 by PCR with 144 primer mixes utilizing sequence-specific primers (PCR-SSP)", Tissue Antigens 1995, vol. 46, pp. 355-67). In the PCR-SSO method, a membrane is prepared (whereon the DNA amplified by the HLA gene specific primers is immobilised) and the specific oligonucleotide probes for the respective type of HLA are hybridised for typing (e.g. cf. R.K. Saiki et al., "Analysis of enzymatically amplified beta-globin and HLA-DQ alpha DNA with allele-specific oligonucleotide probes", Nature 1986, vol. 324, pp. 163-6).

The HLA-B genotyping kits currently available in the market are based on these two methods, PCR-SSP and PCR-SSO. But although these methods have been effective in routine laboratory practise, they require a time-consuming post-PCR processing that is a big source of contamination. Moreover, PCR-SSP requires a large number of PCR reactions and is difficult to automate, which limits its throughput capabilities. On the other hand, although PCR-SSO is capable of a higher throughput, its processing times are longer and it does not allow for differentiation between motifs present on cis-trans strands of the DNA template.

In order to overcome these limitations, several alternatives have been proposed in the last three years, but they are still not in the market: microarray technology (cf. C. Consolandi et al., "Polymorphism analysis within the HLA-A locus by universal oligonucleotide array", Hum. Mutat. 2004, vol. 24, pp. 428-34), pyrosequencing (cf. S. Ringquist et al., "HLA class II DRB high resolution genotyping by pyrosequencing: comparison of group specific PCR and pyrosequencing primers", Hum. Immunol. 2004, vol. 65, pp. 163-74), single nucleotide extension technique (cf. M. Han et al., "Multiplex single nucleotide extension: a robust and high throughput method for HLA-A locus typing", Hum. Immunol. 2003, vol. 64, pp. 1111-22) and real-time PCR. Applications of real-time PCR have been described for HLA-B27 (cf. C. Tiemann et al., "Rapid DNA typing of HLA-B27 allele by real-time PCR using lightcycler technology", Clin. Lab. 2001, vol. 47, pp. 131-4; K. Sylvain et al., "Rapid screening for HLA-B27 by a TaqMan-PCR assay using sequence-specific primers and a minor groove binder probe, a novel type of TaqMan trade mark probe", J. Immunol. Methods 2004, vol. 287, pp. 179-86; WO 2005/059505) and for HLA-DRB typing (cf. WO 2005/059174).

### SUMMARY OF THE INVENTION

The problem to be solved by this invention is to provide tools to achieve a level of description of the HLA-B locus which makes it possible to better address the study of compatibility in transplants. HLA typing laboratories have to analyse a large number of samples and need to increase the typing resolution in order to have valid clinical results that determine transplant compatibility in an individual.

The solution is based on the detailed work of the inventors (see the examples further below), who have found a method based on real-time PCR with specific primers and probes that makes it possible to obtain a high degree of subtyping of the complete HLA-B locus.

Previously, as already noted, protocols based on real-time PCR with SYBR^{®} green I or with FRET probes have been proposed for typing other targets, including HLA-DRB and HLA-B27. However, similar techniques to achieve a high level of subtyping of the entire HLA-B locus have not been described. The kits currently available in the market for the description of this locus are based on reverse PCR-SSO (e.g. Inno-LiPA HLA-B Update Plus kit from Innogenetics; Dynal RELI SSO HLA-B Typing kit from Dynal Biotech-Oxoid S.A.), PCR-SSO Luminex (e.g. LifeMatch HLA-B Typing Kit from Tepnel Lifecodes-Diagnostica Longwood; Kit LabType SSO B Locus from One Lambda-Rafer) and PCR-SSP (e.g. Dynal Biotech / Pel-Freez; Genovision Olerup; One Lambda; Protrans).

Thus, one aspect of this invention relates to a method for genotyping the alleles of the HLA-B locus from a nucleic acid sample as defined in the appendant claims.

Normally, the container will be a tube or vial suitable for PCR. The primers and the probes of this invention have sequences that are designed to amplify specific regions within the intron 1-exon 2-intron 2-exon 3-intron 3 of the HLA-B locus. Both the primer and probe sequences specified in this description and the complementary sequences thereof will be useful for this invention.

In step (ii), fluorescence signals are detected by means of probes labelled with a fluorescent label. In the art there are several ways to fluorescently label the probes. One of the most widely used at present is probe labelling with fluorochromes of the donour label-acceptor label type, which induces the phenomenon called FRET (acronym of "Förster or fluorescence resonance energy transfer"). The FRET phenomenon entails the intervention of two probes, whose sequences are specific for the DNA template that is amplified. Each probe is labelled with a different fluorophore. When the probe with the donour label is excited by a light source, it transfers its energy to the second probe labelled as the acceptor, instead of emitting fluorescence. Both probes are designed to hybridise in their specific targets such that both fluorophores are in close proximity; thus, resonance energy transfer only occurs when both probes are hybridised to the target when they are very close to each other. The fluorescence resonance energy transfer is the magnitude which makes it possible to follow amplimer production in real-time PCR. The FRET amount depends on the amount of hybridised probes and the latter is proportional to the amount of amplimer whereto they may bind and, consequently, to the amount of amplimer produced. The following labels are used for this purpose: fluorescein, LC-RED 640™, LC-RED 705™ and FAM from Tib-Mol Biol. The assays may be performed using multiple labels with different emission wavelengths.

Other fluorescently labelled probes which have been recently developed by Biotools are Lion probes. They are based on the generation of a detectable and/or quantifiable signal mediated by a 3'-5' nuclease activity. The substrate nucleic acid that is to be identified is placed in contact with at least one probe designed such that said probe is capable of hybridising with the substrate nucleic acid, leaving one or more bases unpaired in the 3' end of the probe, or adjacent bases. The structure of the double-strand nucleic acid with unpaired bases in the 3' end of the oligonucleotide chain is the substrate for the 3'-5' nuclease activity, also present, which splits the probe's unpaired bases, as well as the bases in the 3' position of the unpaired area, generating a measurable signal. The probe chain has double labelling with a quencher fluorophore and a reporter fluorophore.

Other fluorescently labelled probes known in the art are Taqman probes from Applied Biosystems, Foster City, CA, USA. Taqman probes use the 5' fluorogenic exonuclease activity of Taq polymerase in order to measure the number of target sequences in the samples. These probes are longer oligonucleotides than the primers (20-30 bases long with a Tm value 10 °C higher) which contain a fluorescent label normally on 5', and a quencher label (normally TAMRA), generally on 3'. When it is irradiated, the excited fluorescent label transfers energy to the closest quencher label molecule instead of emitting fluorescence (FRET phenomenon). Thus, the proximity between the reporter and the quencher prevents the emission of any fluorescence as long as the probe is intact. TaqMan probes are designed to hybridise to an internal region of a PCR product. When the polymerase replicates, its 5' exonuclease activity releases the probe. This ends the activity of the quencher (non-FRET) and the reporter label begins to emit fluorescence, which increases in each cycle proportionally to the probe release rate. Other fluorescently labelled probes known in the state of the art are Molecular Beacons probes, whose fluorescent chemistry is identical to that of Taqman probes, but have a self-complementary sequence that folds them in a characteristic fashion. Other fluorescently labelled probes known in the art are Scorpion probes, whose fluorescent chemistry is identical to that of Taqman probes, but which may have two different molecular structures: Scorpion uni-probe, with a loop conformation similar to Molecular Beacons probes, and Scorpion bi-probe, with a duplex conformation. Other fluorescently labelled probes known in the art are Simple probes, which consist of a single probe labelled only with a fluorochrome called LED that only emits fluorescence when it is bound to DNA.

The person skilled in the art will be able to adapt the method of the invention in accordance with each of the specified probes.

In general, the nucleic acid in the sample will be DNA, normally genomic DNA. However, the method of this invention may be used with other nucleic acids, such as cloned DNA or, for certain reactions, messenger RNA, and the nucleic acid may be single-strand or double-strand. Real-time PCR is performed using universal thermal cycling parameters and the PCR reaction conditions known by a person skilled in the art. Although assay run times are shorter in LightCycler™ than in other thermocyclers, the person skilled in the art will be able to easily adapt the method of the invention to other RT-PCR machines.

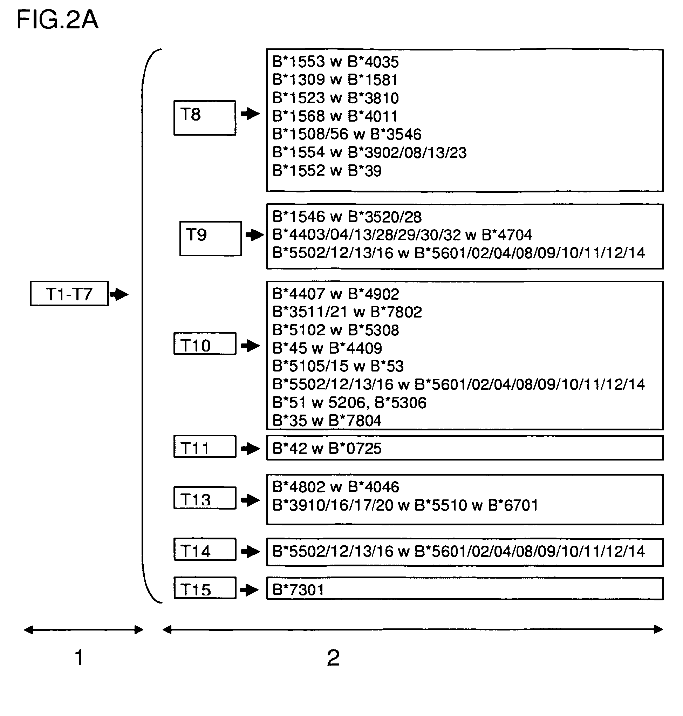

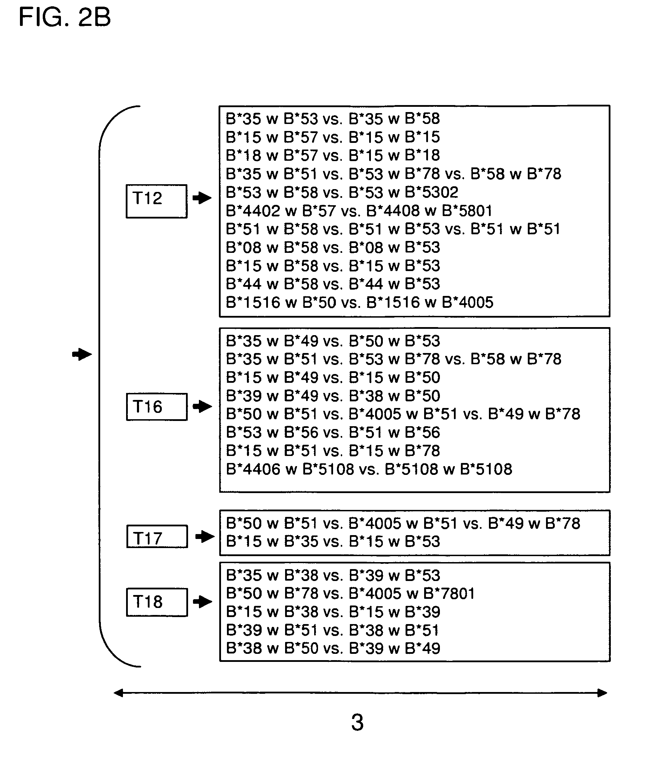

The probes for the battery of containers comprise the probes with sequences SEQ ID NO: 36 and SEQ ID NO: 40-50. FIG. 2, step 1, is an example of the battery of containers specified above. In this case, the battery consists of seven tubes with the primers and probes specified above, which make it possible to differentiate the major allele groups of HLA-B.

In a more particular embodiment, the battery of containers additionally comprises one or more of the following second containers, each container comprising the specified primers:
container 1: primers with sequences SEQ ID NO: 1-3;
container 2: primers with sequences SEQ ID NO: 10-12;
container 3: primers with sequences SEQ ID NO: 13-14;
container 4: primers with sequences SEQ ID NO: 4-7;
container 5: primers with sequences SEQ ID NO: 22-23;
container 6: primers with sequences SEQ ID NO: 20-21.

In a more particular embodiment, each of the above-mentioned second containers additionally comprises the specified probes:
container 1: probes with sequences SEQ ID NO: 32-33, or
container 1: probes with sequences SEQ ID NO: 38-39;
container 2: probes with sequences SEQ ID NO: 40-41;
container 3: probes with sequences SEQ ID NO: 32-33;
container 4: probes with sequences SEQ ID NO: 34-35;
container 5: probes with sequences SEQ ID NO: 33 and SED ID NO: 51;
container 6: probes with sequences SEQ ID NO: 42-43.

Therefore, the battery of containers of the invention may be formed by the containers which allow to differentiate the major allele groups of HLA-B and, additionally, by the above-mentioned second containers. The second containers make it possible to solve the allele confusions in the homozygous hybridisation pattern. An example of second containers are those specified in FIG. 2 step 2. In this example, container 3 would correspond to T11. Different combinations of second containers may be selected depending on the allele confusions that need to be solved.

In a more particular embodiment, the battery of containers additionally comprises one or more of the following third containers, each container comprising the specified primers:
container 7: primers with sequences SEQ ID NO: 8-9;
container 8: primers with sequences SEQ ID NO: 24-25;
container 9: primers with sequences SEQ ID NO: 26-27;
container 10: primers with sequences SEQ ID NO: 15 and SEQ ID NO: 27.

Finally, in a more particular embodiment, each of the above-mentioned third containers additionally comprises the specified probes:
container 7: probes with sequences SEQ ID NO: 36-37;
container 8: probes with sequences SEQ ID NO: 46-47;
container 9: probes with sequences SEQ ID NO: 36-37;
container 10: probes with sequences SEQ ID NO: 36 and SEQ ID NO: 50.

Therefore, the battery of containers of the invention may consist of the containers which allow to differentiate the major allele groups of HLA-B, the second containers which allow to solve the allele confusions in the homozygous hybridisation pattern and, additionally, by the above-mentioned third containers. The third containers allow to solve the allele confusions in the heterozygous hybridisation pattern. An example of third containers are those specified in FIG. 2 step 3. In this example, container 7 would correspond to T12. Different combinations of third containers may be selected depending on the allele confusions that need to be solved.

In another particular embodiment of the invention, two primers for (3-globin gene with SEQ ID NO: 28-29 are added as a control in step (i) of the genotyping method. In a more particular embodiment, the probes with sequences SEQ ID NO: 52-53 are also added in order to detect the amplification of the β-globin gene. Instead of a control based on the β-globin gene, it may be necessary to add two primers for SCYA4 gene with SEQ ID NO: 30-31 to some containers, as a control in step (i). In a more particular embodiment, the probes with sequences SEQ ID NO: 54-55 are also added in order to detect the amplification of the SCYA4 gene. This occurs, for example, in tube T5 of the examples described below. The controls may be external (in a different tube), although they are preferably internal (in the same tube).

In a preferred embodiment, the probes are fluorescently labelled with fluorochromes of the donour label-acceptor label type, which lead to the FRET energy transfer phenomenon explained above.

Another aspect of the invention is related to a kit for genotyping the alleles of the HLA-B locus from a nucleic acid sample, by means of the method defined in any of the forms disclosed above, which comprises the fluorescence pattern, the primers and the probes, as defined above. The kit may optionally comprise instructions to determine the HLA-B alleles of a sample in accordance with the kit components.

The terms "genotyping" and "typing" are used as synonymous in this description and refer to any test that reveals the specific alleles inherited by an individual, which is particularly useful in situations wherein more than one genotype combination may produce the same clinical presentation. In this description, the term "locus" means the position occupied by each HLA gene (e.g. the B locus of HLA). The term "allele" refers to one of the two or more alternative forms of a gene, which differ in the genetic sequence and lead to observable differences in hereditary characters (phenotype), and are found on the same site in a chromosome. In this case, the HLA-B locus is distributed in different allele groups (e.g. B*35 or B*37) which contain several alleles (e.g. B*3502, B*3508).

The method and the kit of this invention entail many advantages in relation to the HLA typing methods known in the art. The main advantages are the greater speed (65 minutes, including the interpretation); the ease of automation, since only eighteen tubes are necessary in order to obtain a good level of resolution (typing of 300 groups); reduction of the total cost per test thanks to the ease of automation and the simplicity; a surprisingly high degree of allele definition is achieved; and the risk of sample contamination is reduced because the amplified products always remain in the tubes and no post-PCR step is necessary.

The method and the kit of the invention demonstrate great robustness when real laboratory samples are assayed. They allow for the reproducibility, precision and simplicity required for clinical diagnosis: 1) the Tm values show an interassay variation below 1°C and an interassay variation below 0.5 °C in most cases; 2) all the PCR reactions may be performed in 50 cycles and under the same conditions.

Except as otherwise defined, the technical and scientific terms have the same meaning as that commonly understood by a person skilled in the art whereto the invention belongs. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration, and are not intended to be limiting of the present invention.

### BRIEF DESCRIPTION OF THE DRAWINGS

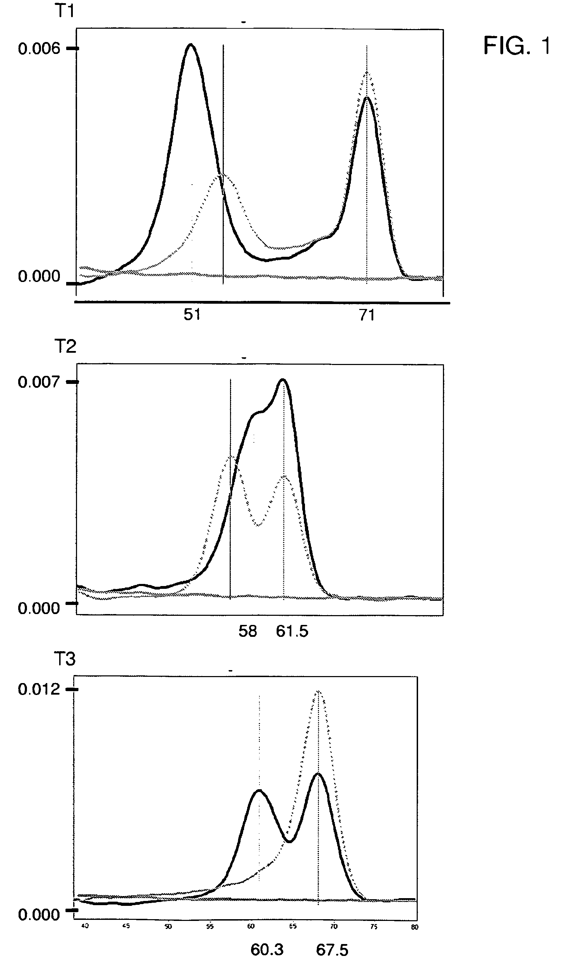

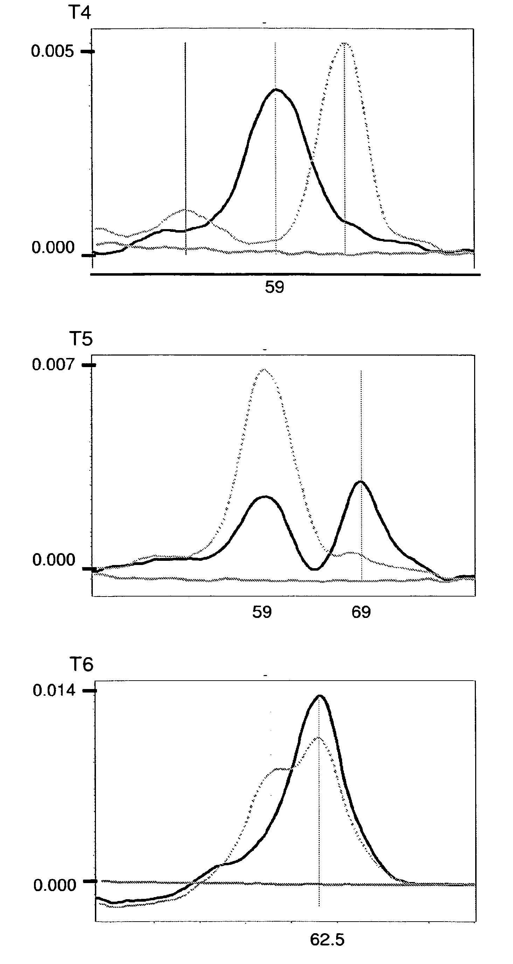

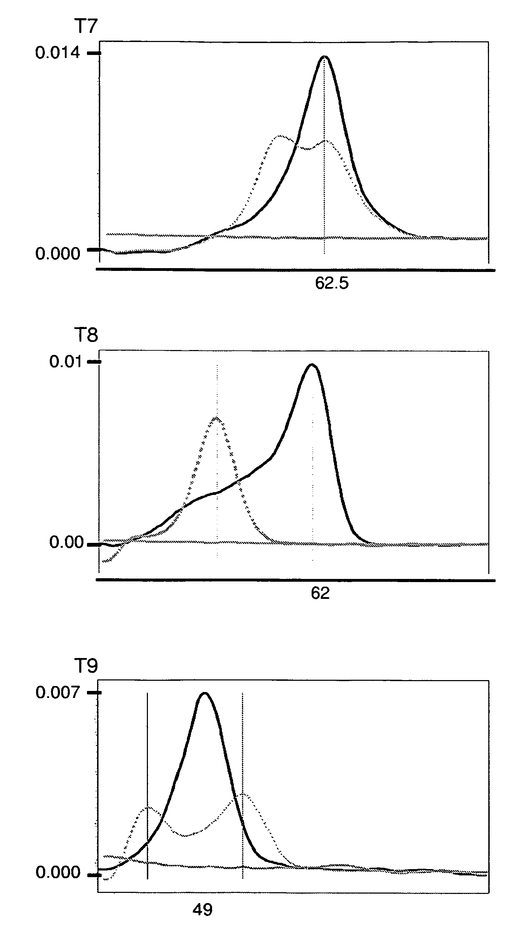

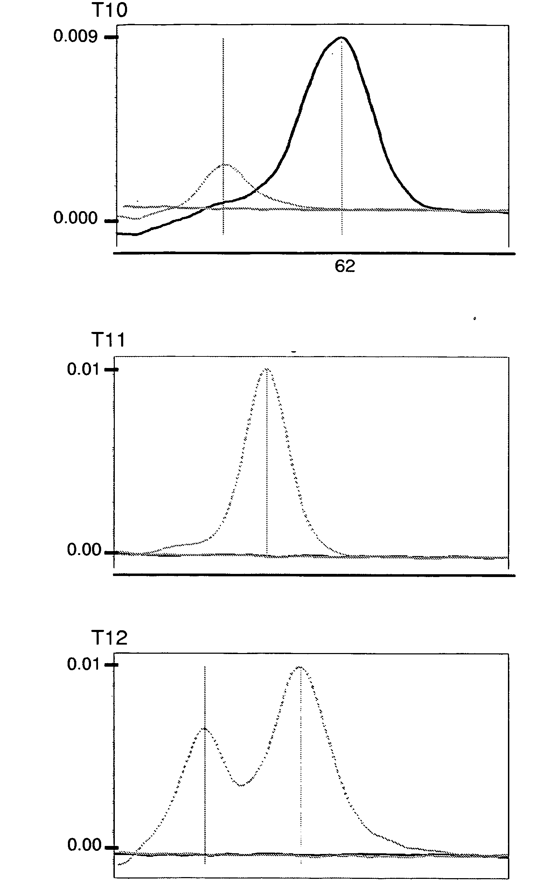

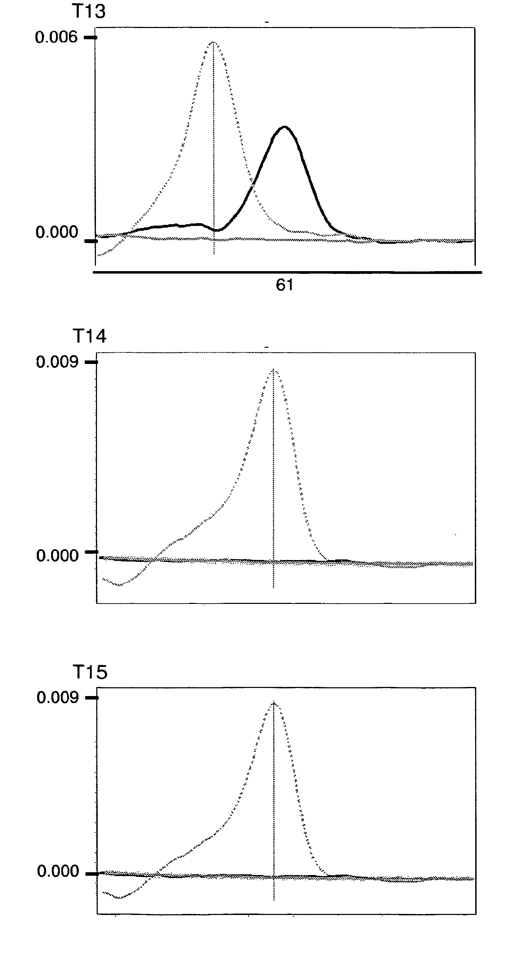

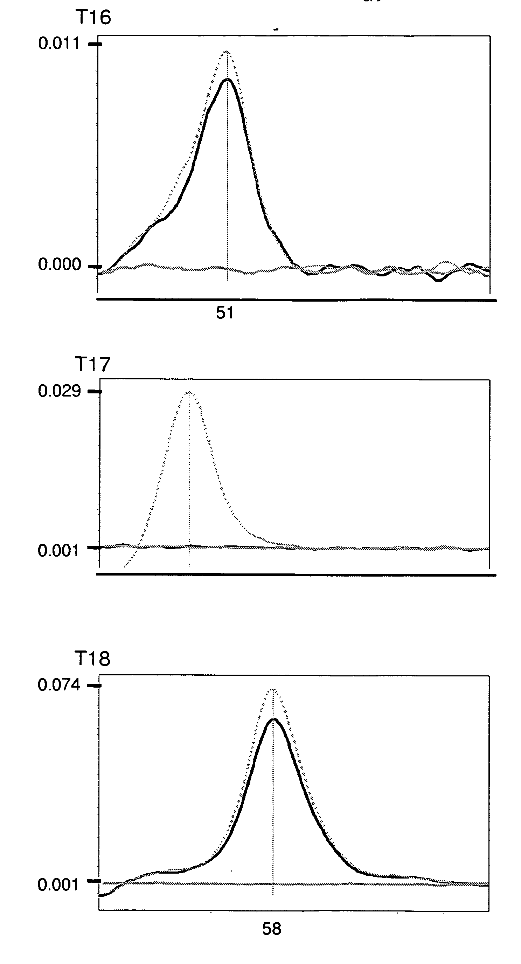

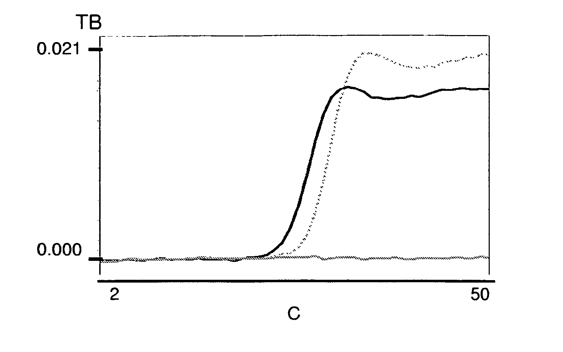

FIG. 1 shows an example of the results of the 18 reaction tubes used for typing of a sample. The typing result of the example sample are alleles B^{*}14/51. T1 means tube number 1 and the same nomenclature is valid up to tube 18 (T18). TB means beta-globin tube. Each graph T1-T18 represents fluorescence in 640/Back 530 versus temperature. The temperature resulting from the example sample in each tube is specified. In those tubes wherein the alleles of the example sample are not amplified, the temperature is not indicated, since the sample is negative in those cases. The grey continuous line indicates the negative control sample (reaction mixture without DNA), the grey broken line indicates the positive control sample (a DNA sample with a known typing that is amplified in said tube) and the black continuous line indicates the example sample with unknown typing. In the case of TB, fluorescence in 705/Back 530 versus cycles is represented.

FIG. 2 shows the typing scheme in three steps. The first step, indicated by a 1, is used to differentiate the major allele groups. In the second step, indicated by a 2, the allele confusions in the homozygous hybridisation pattern are solved. In the third step, indicated by a 3, the allele confusions in the heterozygous hybridisation pattern are solved. A "w" means "with" and "vs." means versus. For example, in T8, the B*1553 confusion is solved with B*4035.

### DETAILED DESCRIPTION OF PARTICULAR EMBODIMENTS

### Preparation of the samples

All the samples used for both set-up and validation of the method were previously typed in the laboratory by means of a standard PCR-SSO, PCR-SSP or sequence-based typing (SBT) methodology. In all cases, the genomic DNA was obtained from samples using the QIAamp DNA blood mini kit (Quiagen GmbH, Hilden, Germany).

### Method set-up and construction of the fluorescence pattern

### Real-time PCR reactions

All the real-time PCR reactions were performed with the LightCycler™ or LightCycler 2.0™ equipment (Roche, Mannheim, Germany), combining the amplification with specific primers (sequences specified in TABLE 1) with the detection by specific probes labelled with fluorochromes (sequences specified in TABLE 2). All the primers were designed to have a Tm between 60-62 °C; all the anchor probes were designed to have a Tm between 70-75 °C and all the sensor probes a Tm in principle between 60-68 °C, although in the case of P20s a higher Tm was required in order to include all the possibilities of that region. All the primers were purchased from Sigma-Genosys (London, UK) and all the hybridisation probes were purchased from Tib MoiBiol™ (Mannheim, Germany). A set of 18 reaction tubes was used fcr each sample in order to define all the HLA-B alleles. In order to develop all the reactions disclosed, 31 primers were used: two pairs for the two different genes in the control sample (beta-globin and SCYA4), and the remaining 27 for the 18 HLA-B specific reactions (some of them were used in different combinations in order to generate different amplifications). Each reaction contained two pairs of the primer and probe set: a set with HLA-B locus primers and FRET probes for the B locus labelled with LC-Red 640™, and a second set with control gene (normally beta-globin) primers and FRET probes for this internal control labelled with LC-Red 705™. As a consequence, in each tube, the signal obtained in F2/F1 (or 640/530 in the LightCycler 2.0™ equipment) was from the specific HLA-B locus reaction, and the signal obtained in F3/F1 (or 705/530 in the LightCycler 2.0™ equipment) was from the control gene. The SCYA4 gene control primers were used in a single reaction, wherein the complementarities between the beta-globin primers and probes with the specific HLA-B locus primers and probes (tube 18; Pbx1-R1as primers; P18s-P1 a probes) affected the reaction in such a way that a very low signal of HLA-B amplification (or of the beta-globin reaction, depending on the tested primer and probe concentrations) was obtained. All the reactions were developed in a final volume of 10 µl, for each reaction. The details of each mixture (primers and probes used, as well as specific reagent concentrations) are listed in TABLE 3. In all the PCR reactions, Taq polymerase, dNTPs and reaction buffer were added using 1 µl of LightCycler Fast Start Master Hybridization probes™ (x10), up to 9 µl of each reaction mixture; a volume of 1 gl of DNA was added at a concentration of 0.1 µg/µl. The real-time amplification protocol for all the reactions was: 10 min of initial denaturation and activation of the Fast Start™ enzyme at 95°C, followed by 50 amplification cycles (5 seconds of denaturation at 95 °C, 6 seconds of hybridisation at 62°C and 10 seconds of extension at 72°C). The melting curves were generated at 95 °C for 0 seconds, 72°C for 20 seconds, 65 °C for 15 seconds, 42°C for 270 seconds, 39°C for 0 seconds (all at a ramp rate of 20 °C/s) and 80°C for 0 seconds (ramp rate of 0.05 °C/s; acquisition mode: continuous), followed by a 30-second cooling step at 40 °C.

### Analysis and interpretation of the data

In order to perform the fluorogenic typing of HLA-B, PCR amplification of a sample was conducted in parallel in all the mixtures of 18 primers and probes. By analysing the presence of melting curves in the F2/F1 channel (640/530), the specificity of the HLA-B locus PCR product was determined. TABLE 6 lists the HLA-B specificities, and also shows the different Tms which allow to differentiate these specificities. Analysis of the F3/F1 channel (705/530) during the amplification step made it possible to detect the control PCR product, which is shown by the increase in F3 fluorescence in the samples. Interpretation of the HLA typing results obtained by real-time PCR was performed using a probe hit table (TABLE 6) containing the homozygous hybridisation patterns of each HLA-B allele and using Polygene 2.0, the software developed to analyse ambiguities (see below).

### Software analysis

In order to define the oligonucleotides (primers and probes), and due to the need to direct them to the HLA-B polymorphic sequences, manual analysis of the HLA-A, -B and -C sequences was performed using the alignment tools of the IMGT/HLA web (Robinson and Marsh 2000). The Oligo^{®} v4 software was used to predict the tendency of the primers and probes to generate artifacts and the meltcalc99free.xlm^{®} application (Schutz and von Ahsen 1999) was used to calculate the Tms of the probes and to calculate the theoretical melting temperature obtained from all the different mismatched hybridisations. In order to predict the ambiguous typing results obtained from heterozygous samples for HLA-B alleles, the Polygene 2.0 software was developed. In order to obtain the genotyping of each sample, taking into consideration heterozygous situations, the different Tm results were coded in binary code, where, for each Tm, 1 meant positive and 0 meant negative. The coded number was analysed by means of Polygene 2.0. This software compares all the possible pairs of typing results predicted for the known HLA-B alleles, including their frequency in each allele [calculated as the mean of all the known data about this allele in the Caucasoid population (cf. D. Middleton et al., "New allele frequency database: http://www.allelefrequencies.net", Tissue Antigens 2003, vol. 61, pp. 403-7) starting from samples typed in 2004 by the National Marrow Donor Program (NMDP) (cf. G.M. Schreuder et al., "HLA dictionary 2004: summary of HLA-A, -B, -C, -DRB1/3/4/5, -DQB1 alleles and their association with serologically defined HLA-A, -B, -C, -DR, and -DQ antigens", Hum. Immunol. 2005, vol. 66(2), pp.170-210)], and determines which allele combinations will generate ambiguous results (when they co-exist in a heterozygous sample), as well as the frequency of each allele combination.

### Results

In order to establish the FRET real-time PCR approach with 18 tubes, a total of 250 samples from bone marrow or solid organ transplant patients, or healthy donours, were selected from the DNA repository of the Banc de Sang i Teixits (Barcelona) in order to represent all the serologically-defined HLA-B alleles available in the Spanish population (in homo- and heterozygous presentation). The samples were selected to represent all the heterozygous situations involving closer Tm profiles in the same reaction; in this way, their Tm was specified, allowing for the accurate assignment of ambiguous heterozygous results. For each reaction, different concentrations of both sets of primers involved (B locus and control primers) and the MgCl₂ concentration were assayed to determine the best combination for obtaining the greatest specific melting signal for the HLA-B locus alleles in each case. In those reactions wherein the conditions so required, DMSO was added after evaluating the concentration required in said reactions. When the primers or the probes designed did not perform as expected, new oligonucleotides were designed which included modifications with respect to the original sequence (cf. TABLES 1 and 2). Since the main parameter to be analysed was the melting step, all the probes were designed to operate under the same melting conditions, although some of them produced higher fluorescence levels than others. In fact, this variability between reactions indicates different PCR efficiencies for the primers and probes individual mixes. In order to overcome this problem, the maximum and minimum fluorescence were measured for each reaction (TABLE 4). In general, the minimum fluorescence corresponds to 10 ng of DNA (the minimum amount of sample tested per reaction). If lower fluorescences were observed, they were considered to be PCR artifacts.

HLA-B typing by patterns of 18 real-time PCR Tm results: When the methodology was used in a single assay, the results of the 18 reactions had to be considered (TABLE 6). 61 Tms were experimentally defined and 47 theoretical, not experimentally determined, Tms were added which corresponded to HLA-B specificities that are not common amongst the population. In two reactions (tube 2 and tube 4), the HLA-B probes used for the monitoring did not produce a fluorescence signal with some of the amplified alleles due to the large number of mismatches with the sensor probe. They were considered to be non-amplified alleles. Similarly, in tube 4, where it is possible to detect a Tm of 50 °C, some of the samples which theoretically had to produce this Tm produced a very weak fluorescence signal, whereas other samples did not produce any signal. These Tm results were not taken into account for the final allele assignment for each sample analysed.

HLA-B typing in consecutive PCR rounds: initial HLA-B typing groups by 7 reactions: resolution of ambiguities by additional reaction as the second rt-PCR round: The results of the 18 reactions are not always necessary to produce a typing. Therefore, in order to optimise the procedure, a flow chart was designed to perform the typing in several steps, using the optimal minimum number of reactions in each case. In fact, seven of the eighteen reactions (tubes 1 to 7) allow to classify the B alleles in 188 groups, providing an initial assignment for many of the results. Five of these seven reactions (tubes 1 to 5, which use the same primers but different probes) amplify exon 2 of all the HLA-B alleles except for B*7301. In the case of the third exon, two additional reactions were designed (tubes 6 to 7) in order to amplify a large number of alleles. Thus, these seven initial reactions made it possible to characterise an allele by the analysis of 26 experimentally-defined melting curves (theoretically, they could define 21 additional Tms, which were not analysed because they corresponded to very uncommon alleles). The following eleven amplifications were divided into two groups, leading to two additional amplification rounds. The second round was used to resolve ambiguities in the case of homozygous allele combinations and the third round was used to resolve ambiguities in heterozygous allele combinations.

Therefore, in order to use the minimum possible number of reactions, the typing was performed using the first seven reactions (tubes 1-7) for all the samples, and subsequently, depending on the result obtained, using the necessary reactions to resolve the observed ambiguities, as shown in FIG. 3. Although the reactions were differentiated into two steps, to resolve the homozygous ambiguities (step 2) and the heterozygous ambiguities (step 3), both steps may be simultaneously performed in a single second PCR assay.

Interpretation of HLA-B real-time genotyping data and definition of indeterminations: Interpretation of the HLA-B typing results obtained by real-time PCR was performed using a probe hit table containing the homozygous hybridisation patterns for each HLA-B allele (cf. TABLE 6) and the Polygene 2.0 software. The computer analysis found fours pairs of alleles that were not resolvable by this methodology (the relative frequency is included in parentheses): B*3537 (0.0010443%) versus B*7804 (0.0041772%); B*5401 (0.65895%) versus B*5507 (0.0020886%); B*4047 (0%) versus B*4431 (0%); B*5104 (0.0062658%) versus B*5306 (0%). The Polygene 2.0 software also allowed to detect allele redundancies and assign a specific allele typing (all these data in relation to the expected frequency in the population).
Furthermore, Polygene 2.0 allowed to determine whether the co-existence of different HLA-B alleles could generate typing indeterminations. It was shown that only very rare alleles within the population produced not resolvable HLA assignments (cf. TABLE 5 ).

### Validation of the method

### Analysis of 200 samples

In order to verify the technique's capacity to produce results in clinical samples (once all the reaction conditions were established), the technique was blind-tested with 200 randomly selected clinical samples from solid organ or bone marrow transplant patients, or healthy donours, which were previously genotyped by PCR-SSO. The samples were distributed into seven sets of LightCycler rounds which included, in addition to the samples to be typed, a negative sample (reaction mixture without clinical sample) and a known positive sample (the same for the seven tubes) in each reaction. The analysis of the Tm values of this positive sample allowed to calculate the assay's reproducibility to assess the interassay Tm variation; the intra-assay variability was evaluated using positive samples in the same round. In 17 reactions, the (intra- and interassay) Tm variability was less than ±0.5 °C; in one reaction (tube 2), the Tm of 54 °C had a variability of ±0.75 °C (these high variations were not observed when assessing other Tm values present in this reaction). In all cases, the typing achieved by the real-time procedure agreed with the previous typing methods. An example of HLA-B typing of a DNA sample using this method is shown in FIG. 1.

### PCR-SSO (sequence-specific oligonucleotides)

The set of samples used both to establish and to validate the technique were typed using the conventional PCR-SSO methodology (Dynal Rely SSO HLA-B typing kit, Dynal Biotech, Bromborough, UK).

### PCR-SBT (sequence-based typing)

In order to validate the technique with the 200 samples, and when the technique provided a higher resolution than the previous typing methodology (PCR-SSO), those real-time PCR samples that produced the highest resolution were re-typed by PCR-SBT. This PCR-SBT was performed as described by S. Pozzi et al. ("HLA-B locus sequence-based typing", Tissue Antigens 1999, vol. 53, pp. 275-81), using FastStart™ (Roche) polymerase and BigDye™ Terminator Cycle Sequencing v2.0 (AbiPrism - PE Biosystems, Foster City, Califomia).

**TABLE 1: Primers for HLA-B typing by real-time PCR.**

| Primer | Sequence | Position |
|---|---|---|
| R8s | SEQ ID NO: 1 | 737 |
| R8as1 | SEQ ID NO: 2 | 846 |
| R8as2 | SEQ ID NO: 3 | 846 |
| R13s | SEQ ID NO: 4 | 279 |
| R13as1 | SEQ ID NO: 5 | 414 |
| R13as2 | SEQ ID NO: 6 | 414 |
| R13as3 | SEQ ID NO: 7 | 414 |
| R12s | SEQ ID NO: 8 | 243 |
| R12as | SEQ ID NO: 9 | 416 |
| R10s | SEQ ID NO: 10 | 729 |
| R10as1 | SEQ ID NO: 11 | 941 |
| R10as2 | SEQ ID NO: 12 | 941 |
| R11s | SEQ ID NO: 13 | 745 |
| R11as | SEQ ID NO: 14 | 985 |
| Pbx1 | SEQ ID NO: 15 | 138 |
| R1as | SEQ ID NO: 16 | 515 |
| R6s | SEQ ID NO: 17 | 781 |
| Pbint3 | SEQ ID NO: 18 | 1037 |
| R7s | SEQ ID NO: 19 | 846 |
| R15s | SEQ ID NO: 20 | 279 |
| R15as | SEQ ID NO: 21 | 409 |
| R14s | SEQ ID NO: 22 | 729 |
| R14as | SEQ ID NO: 23 | 941 |
| R16s | SEQ ID NO: 24 | 419 |
| R16as | SEQ ID NO: 25 | 729 |
| R17s | SEQ ID NO: 26 | 292 |
| Bw4as | SEQ ID NO: 27 | 454 |
| Pc03 | SEQ ID NO: 28 | 199 |
| Pc04 | SEQ ID NO: 29 | 270 |
| 4o1s | SEQ ID NO: 30 | 1335 |
| 442as | SEQ ID NO: 31 | 1548 |

**TABLE 2: Probes for HLA-B typing bv real-time PCR.**

| Probe | Sequence | Labelling |
|---|---|---|
| P6s | SEQ ID NO: 32 | 3' Fluos |
| P6a | SEQ ID NO: 33 | 3' LC-Red 640 |
| P9s | SEQ ID NO: 34 | 5' Fam |
| P9a | SEQ ID NO: 35 | 3' LC-Red 640 |
| P1a | SEQ ID NO: 36 | 3' LC-Red 640 |
| P1s | SEQ ID NO: 37 | 5' Fam |
| P8s | SEQ ID NO: 38 | 3' Fluos |
| P8a | SEQ ID NO: 39 | 5' LC-Red 640 |
| P14s | SEQ ID NO: 40 | 3' Fluos |
| P14a | SEQ ID NO: 41 | 5' LC-Red 640 |
| P73s | SEQ ID NO: 42 | 3' Fluos |
| P73a | SEQ ID NO: 43 | 5' LC-Red 640 |
| P2s | SEQ ID NO: 44 | 5' Fam |
| P2a | SEQ ID NO: 45 | 3' LC-Red 640 |
| P20s | SEQ ID NO: 46 | 3' Fluos |
| P20a | SEQ ID NO: 47 | 5' LC-Red 640 |
| P19s | SEQ ID NO: 48 | 3' Fluos |
| P19a | SEQ ID NO: 49 | 5' LC-Red 640 |
| P18s | SEQ ID NO: 50 | 5' Fam |
| P5s | SEQ ID NO: 51 | 3' Fluos |
| CTW LC | SEQ ID NO: 52 | 5' LC-Red 705 |
| CTW FL | SEQ ID NO: 53 | 3'Fluos |
| C2a | SEQ ID NO: 54 | 3' Fluos |
| C2s | SEQ ID NO: 55 | 5' LC-Red 705 |

**TABLE 3: PCR Reagents. M means primer mix (as) for that reaction, in cases R8, R10 and R13. Mix MR8as is formed by 16.5% of R8asl and 83.5% of R8as2. MR10as is formed by 80% of R10as1 and 20% of R10as2. MR13as is formed by 46.53% of R13as1, 52.47% of R13as2 and 1 % of R13as3. All the tubes contained 1 x of Fast Start Master H.P. T means tube.**

| T | sense primer (S) | µM S | antisense primer (AS) | µM AS | Anchor probe | µM anchor probe | Sensor probe | µM sensor | % DMSO | mM Mg²⁺ |
|---|---|---|---|---|---|---|---|---|---|---|
| 1 | Pbx1 | 0.2 | R1 as | 1.4 | P20A | 0.2 | P20S | 0.2 | 2 | 5 |
| | PC 03 | 0.2 | PC 04 | 0.6 | CTW LC | 0.2 | CTW FL | 0.2 | | |
| 2 | Pbx1 | 0.5 | R1 as | 0.5 | P19a | 0.2 | P19s | 0.4 | 2 | 5 |
| | PC 03 | 0.2 | PC 04 | 0.6 | 2 CTW LC | 0.2 | CTW FL | 0.2 | | |
| 3 | Pbx1 | 0.2 | R1 as | 1.4 | P73a | 0.2 | P73s | 0.2 | 2 | 4 |
| | PC 03 | 0.2 | PC 04 | 0.6 | CTW LC | 0.2 | CTW FL | 0.2 | | |
| 4 | Pbx1 | 0.5 | R1 as | 0.5 | P2a | 0.2 | P2s | 0.2 | 2 | 5 |
| | PC 03 | 0.2 | PC 04 | 0.6 | CTW LC | 0.2 | CTW FL | 0.2 | | |
| 5 | Pbx1 | 0.2 | R1 as | 1.6 | P1a | 0.2 | P18s | 0.2 | 2 | 5 |
| | 4o1s s | 0.6 | 442as | 0.2 | C2a | 0.2 | C2s | 0.2 | | |
| 6 | R6s | 0.2 | Pbint 3 | 0.6 | P14a | 0.2 | P14s | 0.2 | - | 3 |
| | PC 03 | 0.2 | PC 04 | 1.0 | CTW LC | 0.2 | CTW FL | 0.2 | | |
| 7 | R7s | 0.1 | Pbint 3 | 0.5 | P14s | 0.2 | P14a | 0.2 | - | 3 |
| | PC 03 | 0.2 | PC 04 | 1.0 | CTW LC | 0.2 | CTW FL | 0.2 | | |
| 8 | R8s | 0.2 | MR8as | 0.6 | P6a | 0.2 | P6s | 0.2 | - | 3 |
| | PC 03 | 0.4 | PC 04 | 2.0 | CTW LC | 0.2 | CTW FL | 0.2 | | |
| 9 | R8s | 0.2 | MR8as | 0.8 | P8a | 0.2 | P8s | P8s | - | 3.75 |
| | PC 03 | 0.2 | PC 04 | 2.0 | CTW LC | 0.2 | CTW FL | 0.2 | | |
| 10 | R10s | 0.2 | MR10as | 1.0 | P14a | 0.2 | P14s | 0.2 | - | 3 |
| | PC 03 | 0.2 | PC 04 | 1.0 | CTW LC | 0.2 | CTW FL | 0.2 | | |
| 11 | R11s | 0.4 | R11as | 1.0 | P6a | 0.2 | P6s | 0.2 | - | 5 |
| | PC 03 | 0.2 | PC 04 | 1.0 | CTW LC | 0.2 | CTW FL | 0.2 | | |
| 12 | R12s | 0.2 | R12as | 1.0 | P1a | 0.2 | P1s | 0.2 | - | 4 |
| | PC 03 | 0.2 | PC 04 | 1.0 | CTW LC | 0.2 | CTW FL | 0.2 | | |
| 13 | R13s | 0.2 | MR13as | 1.4 | P9a | 0.2 | P9s | 0.2 | - | 3 |
| | PC 03 | 0.2 | PC 04 | 1.0 | CTW LC | 0.2 | CTW FL | 0.2 | | |
| 14 | R14s | 0.2 | R14as | 1.0 | P6a | 0.2 | P5s | 0.2 | - | 5 |
| | PC 03 | 0.2 | PC 04 | 0.6 | CTW LC | 0.2 | CTW FL | 0.2 | | |
| 15 | R15s | 0.1 | R15as | 1.4 | P73a | 0.2 | P73s | 0.2 | - | 4.5 |
| | PC 03 | 0.2 | PC 04 | 1.0 | CTW LC | 0.2 | CTW FL | 0.2 | | |
| 16 | R16s | 0.5 | R16as | 1.5 | P20A | 0.2 | P20S | 0.2 | - | 3 |
| | PC 03 | 0.2 | PC 04 | 0.5 | CTW LC | 0.2 | CTW FL | 0.2 | | |
| 17 | R17s | 0.2 | Bw4as | 0.5 | P1a | 0.2 | P1s | 0.2 | - | 5 |
| | PC 03 | 0.2 | PC 04 | 1.2 | CTW LC | 0.2 | CTW FL | 0.2 | | |
| 18 | Pbx1 | 0.2 | Bw4as | 1.0 | P1a | 0.2 | P18s | 0.2 | - | 4 |
| | PC 03 | 0.2 | PC 04 | 0.6 | CTW LC | 0.2 | CTW FL | 0.2 | | |

**TABLE 4. Maximum and minimum fluorescence observed for each reaction**

| | Minimum fluorescence | Maximum fluorescence |
|---|---|---|
| Tube 1 | 0.002 | 0.01 |
| Tube 2 | 0.002 | 0.013 |
| Tube 3 | 0.003 | 0.012 |
| Tube 4 | 0.002 | 0.007 |
| Tube 5 | 0.002 | 0.007 |
| Tube 6 | 0.002 | 0.011 |
| Tube 7 | 0.002 | 0.007 |
| Tube 8 | 0.0045 | 0.03 |
| Tube 9 | 0.002 | 0.011 |
| Tube 10 | 0.0045 | 0.01 |
| Tube 11 | 0.0045 | 0.017 |
| Tube 12 | 0.001 | 0.006 |
| Tube 13 | 0.017 | 0.021 |
| Tube 14 | 0.003 | 0.09 |
| Tube 15 | 0.0045 | 0.023 |
| Tube 16 | 0.005 | 0.012 |
| Tube 17 | 0.03 | 0.07 |
| Tube 18 | 0.02 | 0.07 |

**TABLE 5. Confusions found in the heterozygous HLA-B situation. Ambiguities found in the heterozygous situation. The combinatorial frequencies of the HLA-B alleles are calculated from the mean of the entire Caucasian population in the National Marrow Donor Program (NMDP column) and the Allele Project (AP column). This figure only shows the indeterminations with NMDP values higher than 0.0005% (5E-6) and a percentage within the group (% in group) greater than 1 %, or AP values with a documented frequency.**

| | | | NMDP | | AP | |
|---|---|---|---|---|---|---|
| No | Allele 1 | Allele 2 | Combination frecuency (% in group) | | Combination frecuency (% in group) | |
| 1 | B*5001 | B*520101 | 5.23E-04 | (81.65%) | 2.63E-04 | (51.45%) |
| | B*4005 | B*520101 | 9.02E-05 | (14.07%) | 9.23E-05 | (18.05%) |
| | B*5002 | B*520101 | 2.43E-05 | (3.79%) | 1.56E-04 | (30.50%) |
| | B*4009 | B*520101 | 2.16E-06 | ( 0.34%) | Nd | ( - ) |
| | B*5004 | B*520101 | 9.24E-07 | ( 0.14%) | Nd | ( - ) |
| 2 | B*0801 | B*4102 | 3.40E-04 | ( 96.33% ) | Nd | ( - ) |
| | B*0801 | B*4103 | 7.82E-06 | ( 2.22% ) | Nd | ( - ) |
| | B*0801 | B*4009 | 4.98E-06 | (1.41% ) | Nd | ( - ) |
| 3 | B*5001 | B*510101 | 3.52E-04 | ( 72.90% ) | 1.94E-03 | ( 61.43% ) |
| | B*4005 | B*510101 | 6.07E-05 | ( 12.56% ) | 6.82E-05 | ( 2.15% ) |
| | B*5001 | B*510201 | 4.04E-05 | ( 8.37% ) | Nd | ( - ) |
| | B*5002 | B*510101 | 1.64E-05 | ( 3.39% ) | 1.15E-03 | ( 36.42% ) |
| | B*4005 | B*510201 | 6.97E-06 | ( 1.44% ) | Nd | ( - ) |
| 4 | B*3701 | B*8101 | 1.23E-04 | ( 51.85% ) | 1.33E-05 | ( 49.23% ) |
| | B*3701 | B*4801 | 1.14E-04 | ( 48.15% ) | 1.38E-05 | ( 50.77% ) |
| 5 | B*3801 | B*390101 | 1.20E-04 | ( 57.77% ) | Nd | ( - ) |
| | B*3801 | B*3905 | 6.19E-05 | ( 29.73% ) | Nd | ( - ) |
| | B*3801 | B*670101 | 2.05E-05 | ( 9.83% ) | Nd | ( - ) |
| | B*3801 | B*3911 | 4.04E-06 | ( 1.94% ) | Nd | ( - ) |
| 6 | B*180102 | B*8101 | 1.18E-04 | ( 50.86% ) | 4.53E-05 | ( 47.86% ) |
| | B*180102 | B*4801 | 1.09E-04 | ( 47.22% ) | 4.68E-05 | ( 49.35% ) |
| | B*1803 | B*8101 | 2.08E-06 | ( 0.90% ) | 1.30E-06 | ( 1.37% ) |
| | B*1803 | B*4801 | 1.93E-06 | ( 0.83% ) | 1.34E-06 | (1.41% ) |
| 7 | B*0801 | B*3910 | 9.03E-05 | ( 60.74% ) | Nd | ( - ) |
| | B*0801 | B*670101 | 5.76E-05 | ( 38.74% ) | Nd | ( - ) |
| 8 | B*4102 | B*4201 | 5.29E-05 | ( 89.33% ) | Nd | ( - ) |
| | B*4102 | B*4202 | 4.17E-06 | ( 7.04% ) | Nd | ( - ) |
| | B*4103 | B*4201 | 1.22E-06 | ( 2.06% ) | Nd | ( - ) |
| | B*4009 | B*4201 | 7.75E-07 | (1.31% ) | Nd | ( - ) |
| 9 | B*5001 | B*7801 | 3.89E-05 | ( 81.65% ) | 4.66E-05 | ( 61.43% ) |
| | B*5002 | B*7801 | 1.81 E-06 | ( 3.79% ) | 2.76E-05 | ( 36.42% ) |
| | B*4005 | B*7801 | 6.71 E-06 | ( 14.07% ) | 1.63E-06 | ( 2.15% ) |
| 10 | B*4501 | B*5108 | 3.87E-05 | ( 81.95% ) | 4.37E-05 | ( 87.22% ) |
| | B*4102 | B*5108 | 8.03E-06 | ( 16.98% ) | 6.27E-06 | ( 12.51 % ) |
| | B*4502 | B*5108 | 6.72E-08 | ( 0.14% ) | 1.36E-07 | ( 0.27% ) |
| 11 | B*5109 | B*5301 | 1.43E-05 | ( 98.89% ) | 2.74E-06 | ( 90.72% ) |
| | B*5109 | B*5302 | 2.34E-08 | ( 0.16% ) | 2.40E-07 | ( 7.95% ) |
| | B*5105 | B*5109 | 1.17E-07 | ( 0.81 % ) | 4.00E-08 | ( 1.33% ) |
| 12 | B*0804 | B*4801 | 4.66E-07 | ( 48.15% ) | 1.19E-06 | ( 50.77% ) |
| | B*0804 | B*8101 | 5.02E-07 | ( 51.85% ) | 1.16E-06 | ( 49.23% ) |
| 13 | B*4801 | B*4801 | 4.06E-05 | ( 48.11 % ) | 8.40E-07 | ( 50.77% ) |
| | B*4801 | B*8101 | 4.37E-05 | ( 51.81 % ) | 8.15E-07 | ( 49.23% ) |
| 14 | B*5105 | B*5301 | 8.33E-06 | ( 98.93% ) | 6.85E-07 | ( 90.72% ) |
| | B*5105 | B*5302 | 1.36E-08 | ( 0.16% ) | 6.00E-08 | ( 7.95% ) |
| | B*5105 | B*5105 | 6.82E-08 | ( 0.81 % ) | 1.00E-08 | ( 1.33% ) |
| 15 | B*1802 | B*4801 | 2.22E-06 | ( 51.85% ) | 2.50E-07 | ( 50.77% ) |
| | B*1802 | B*8101 | 2.06E-06 | ( 48.15% ) | 2.42E-07 | ( 49.23% ) |
| 16 | B*4801 | B*4802 | 2.59E-06 | ( 48.15% ) | 8.33E-08 | ( 50.77% ) |
| | B*4802 | B*8101 | 2.79E-06 | ( 51.85% ) | 8.08E-08 | ( 49.23% ) |

TABLE 6. Fluorescence pattern for the interpretation of results. Fluorescence pattern for each allele observed in the 18 reactions. The first column indicates the alleles, the first row indicates the tube number and the second row for each tube indicates the melting temperatures observed ; hp means not tested; * signal which is not always observed. The black squares indicate a positive signal for the corresponding allele with that melting temperature and the white squares indicate a negative signal for the allele with that melting temperature.

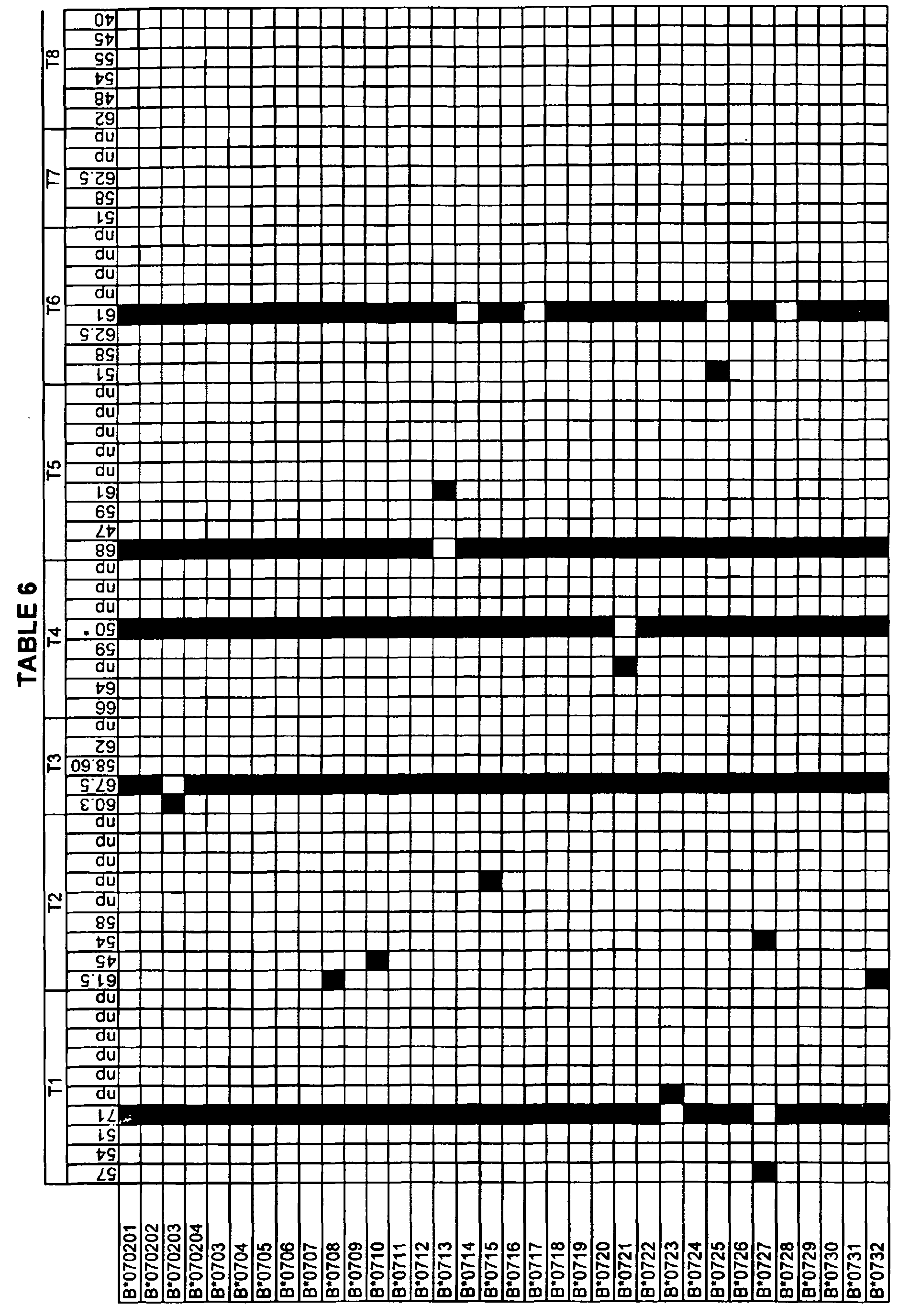

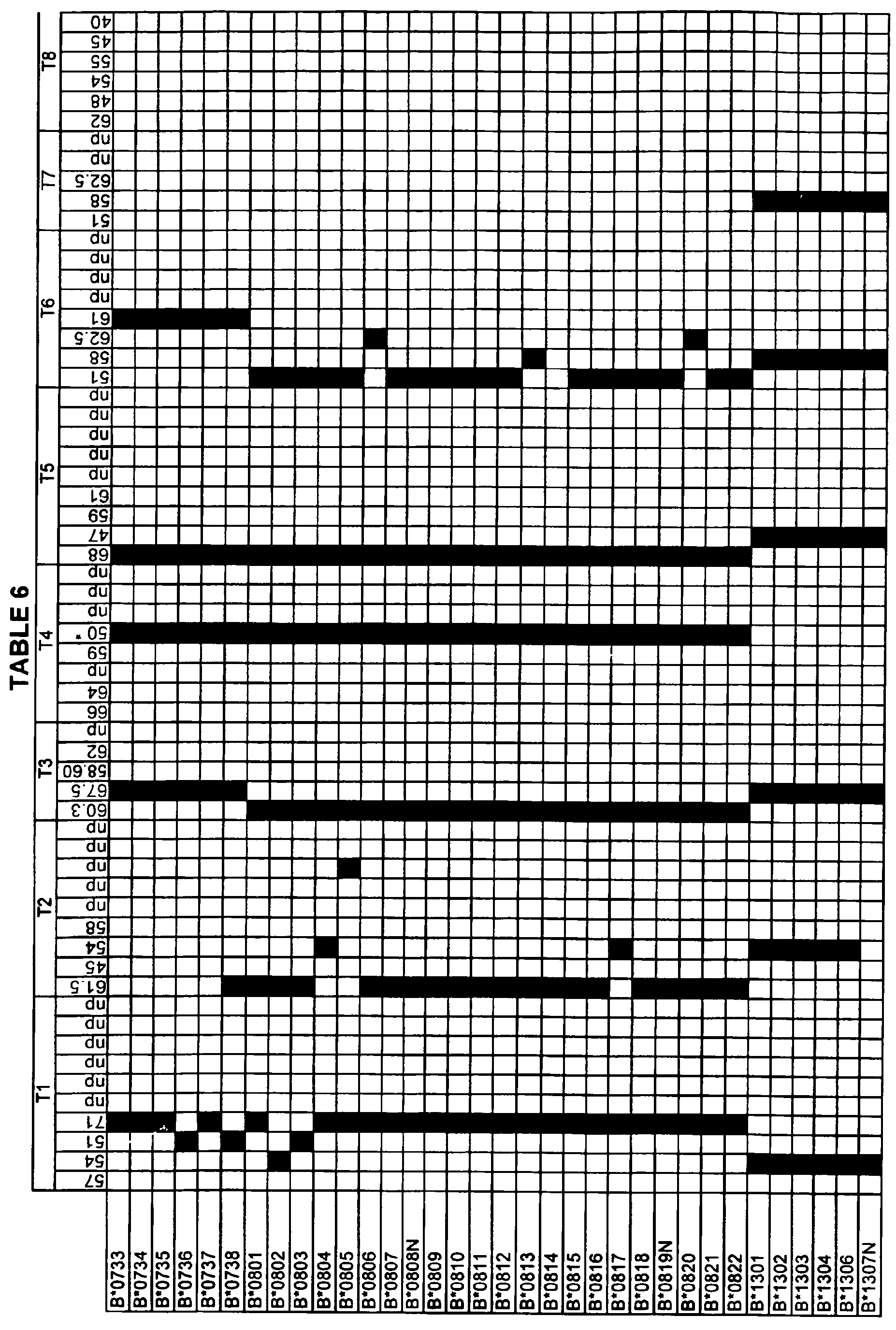

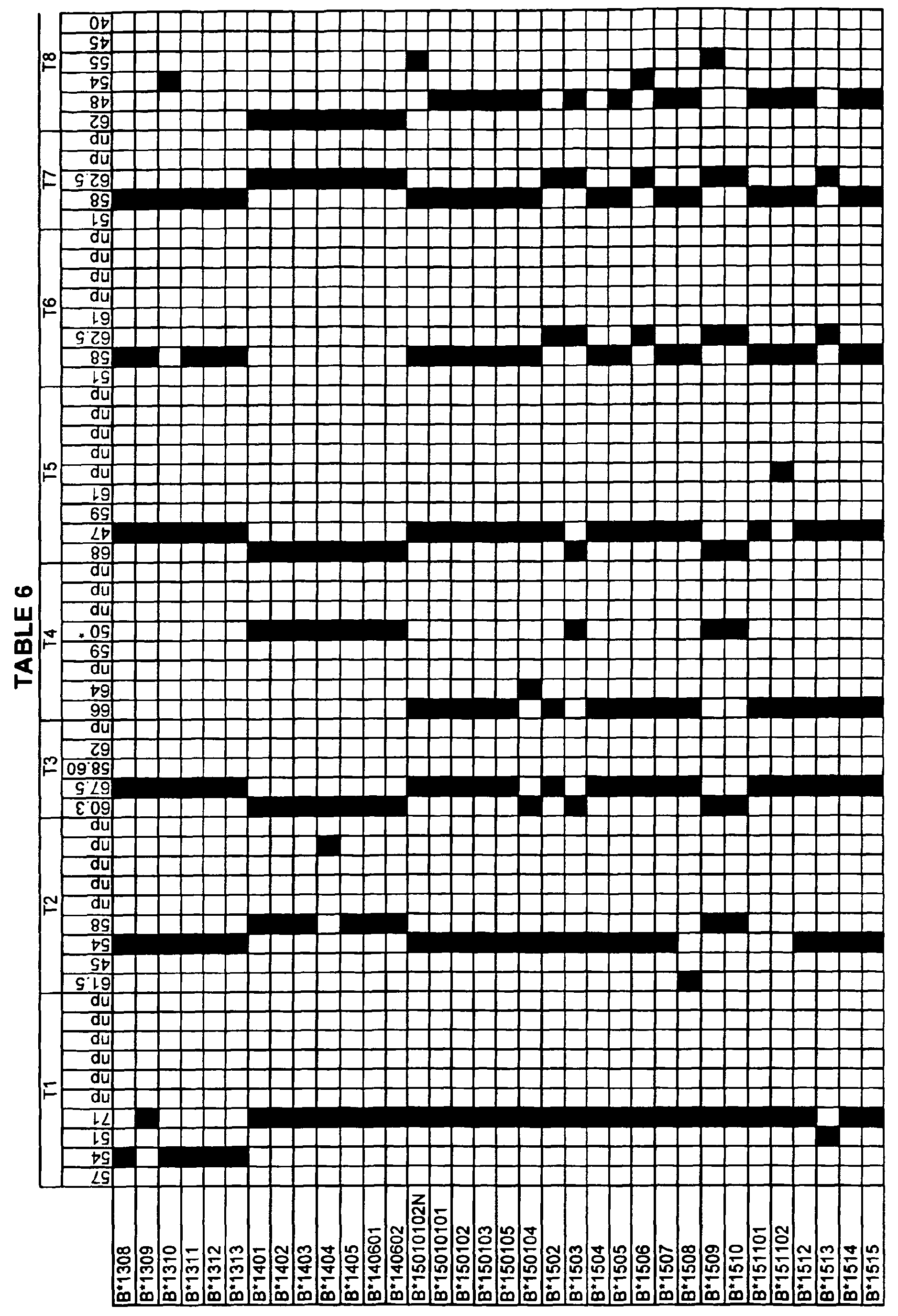

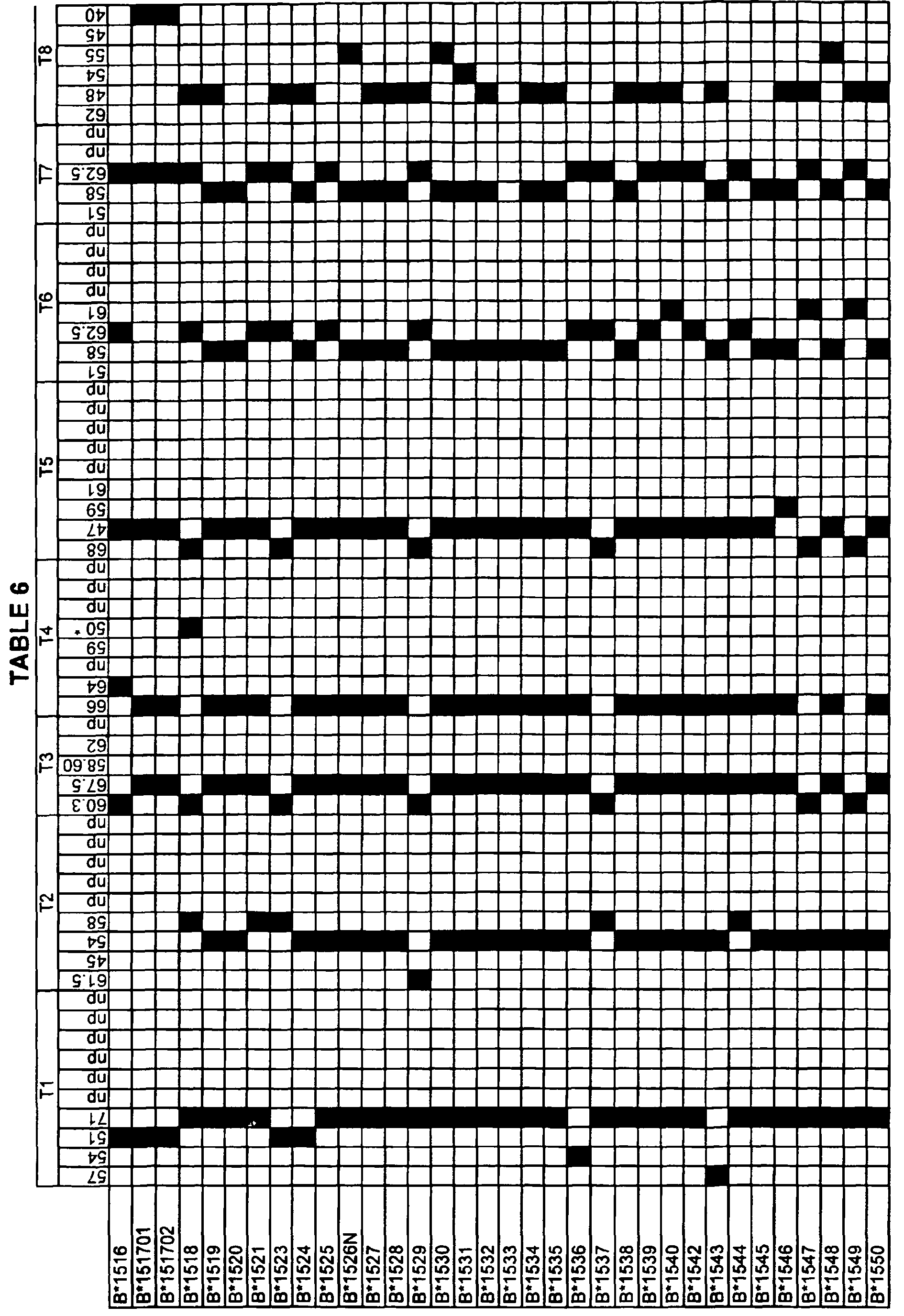

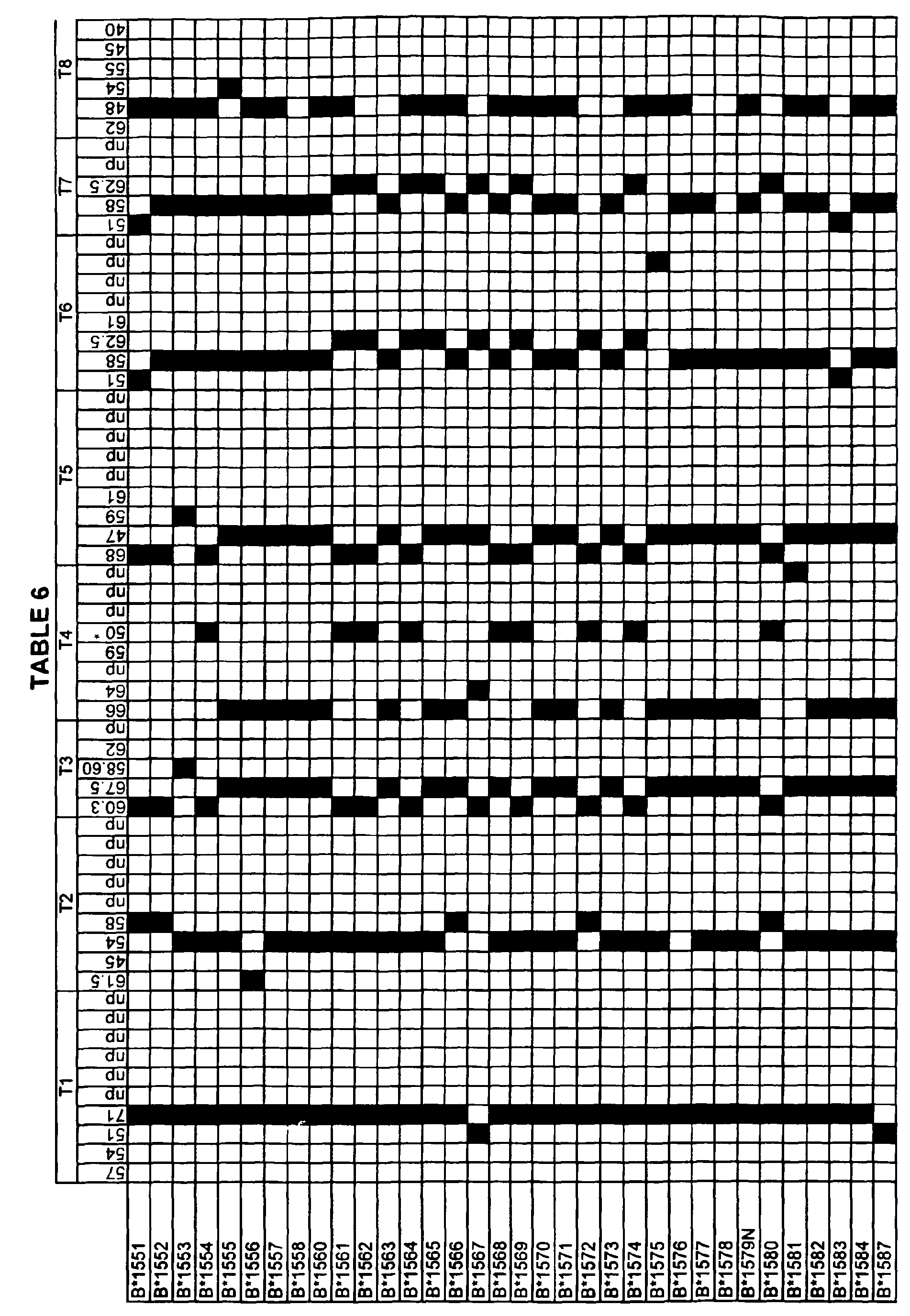

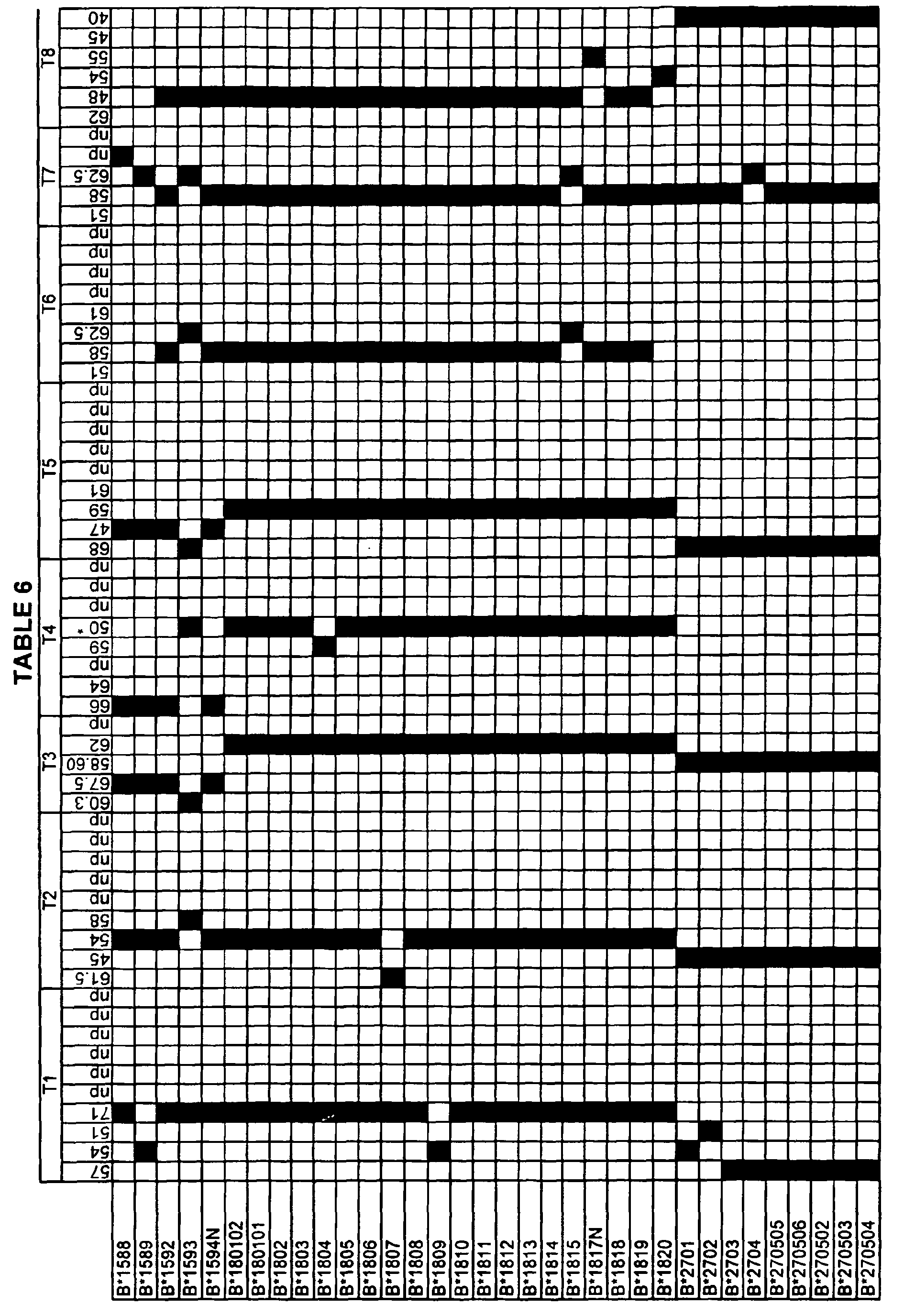

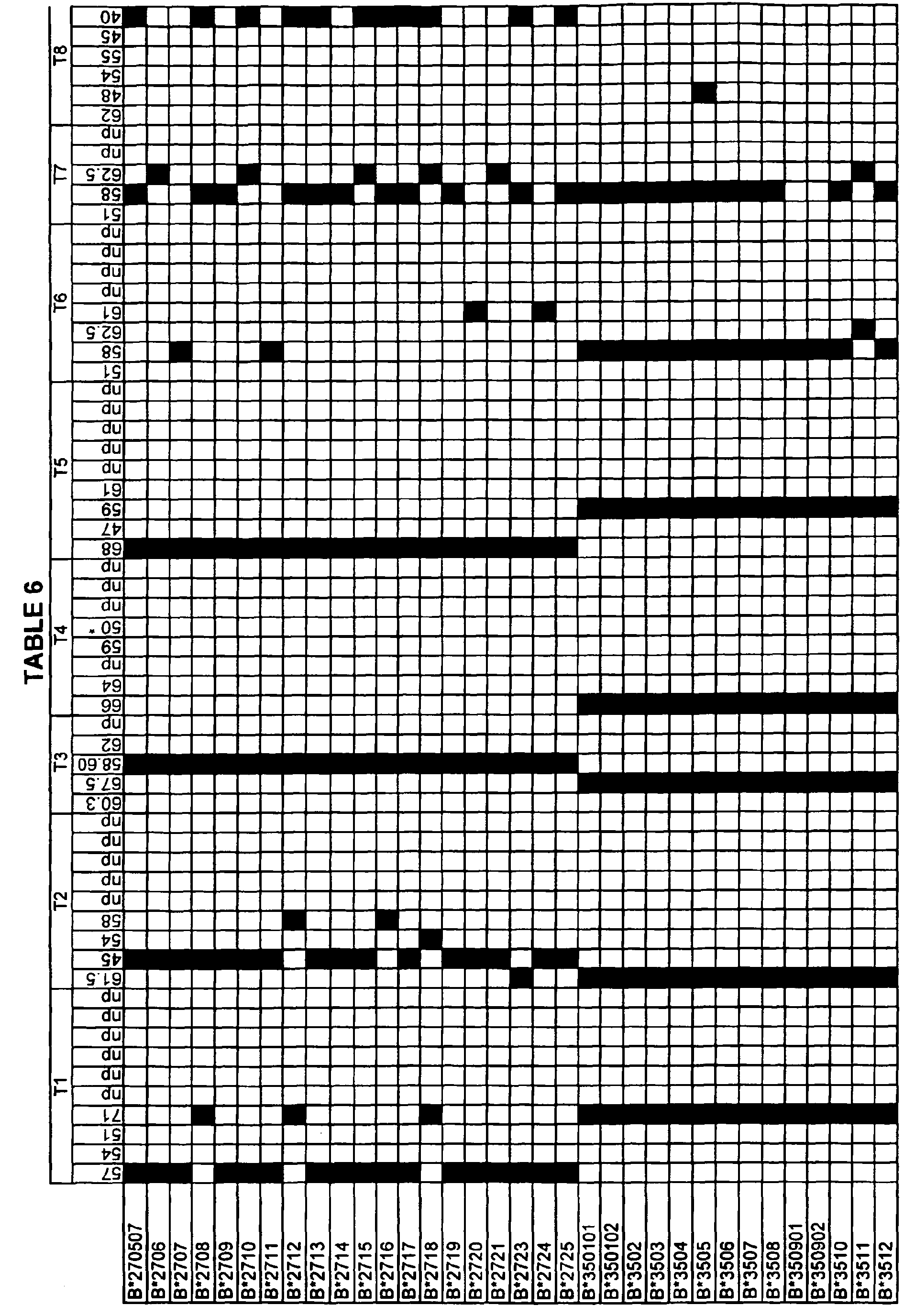

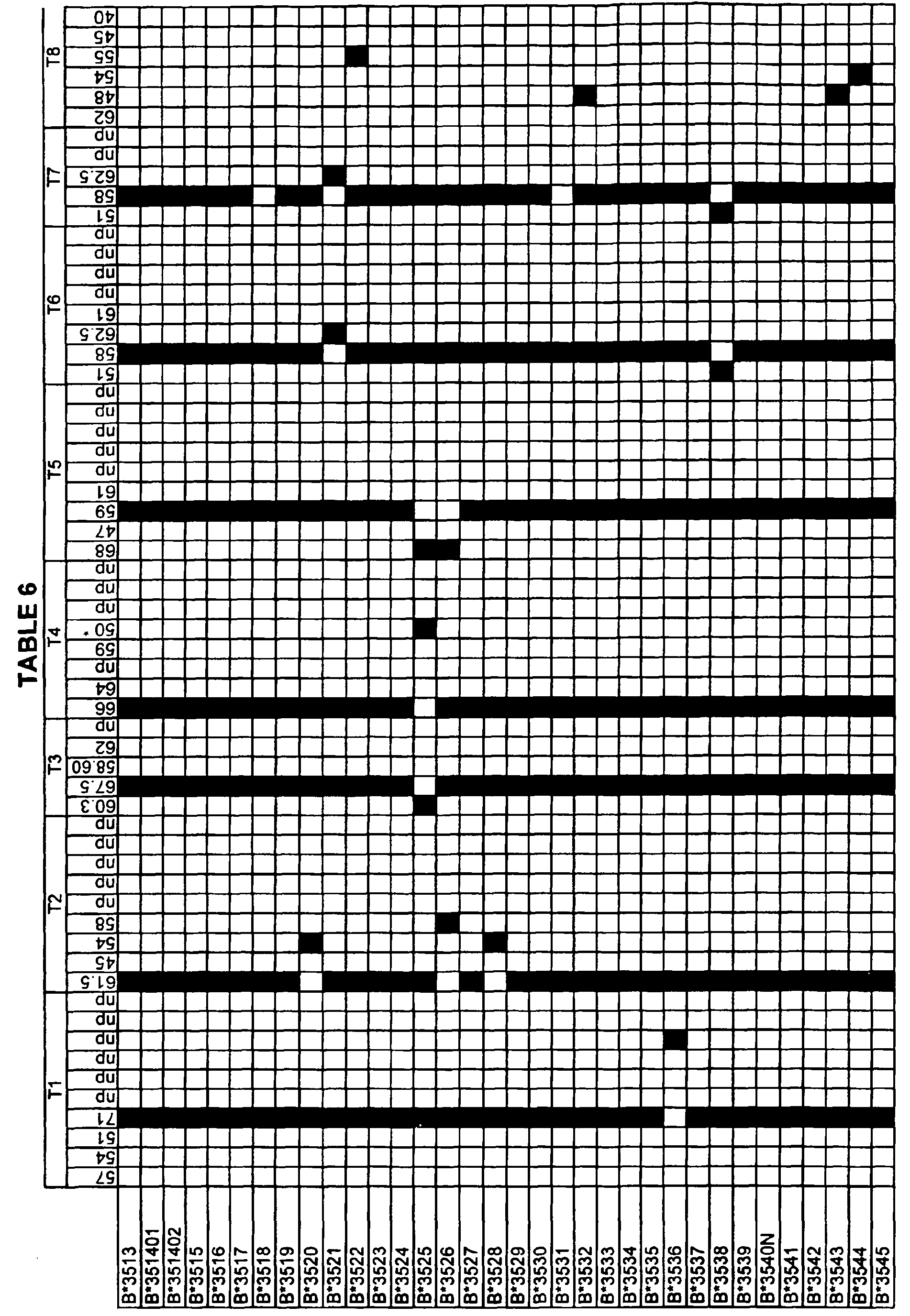

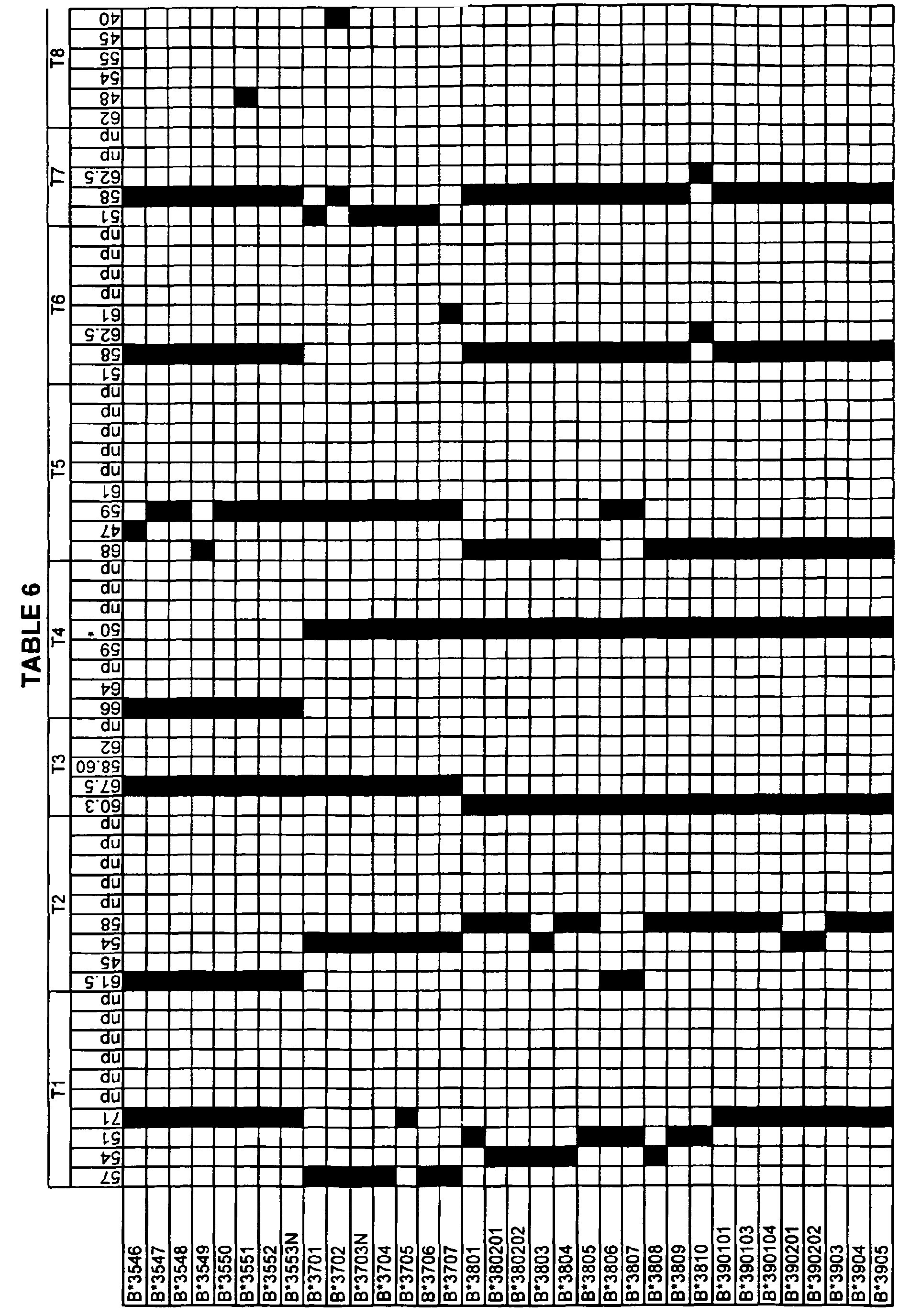

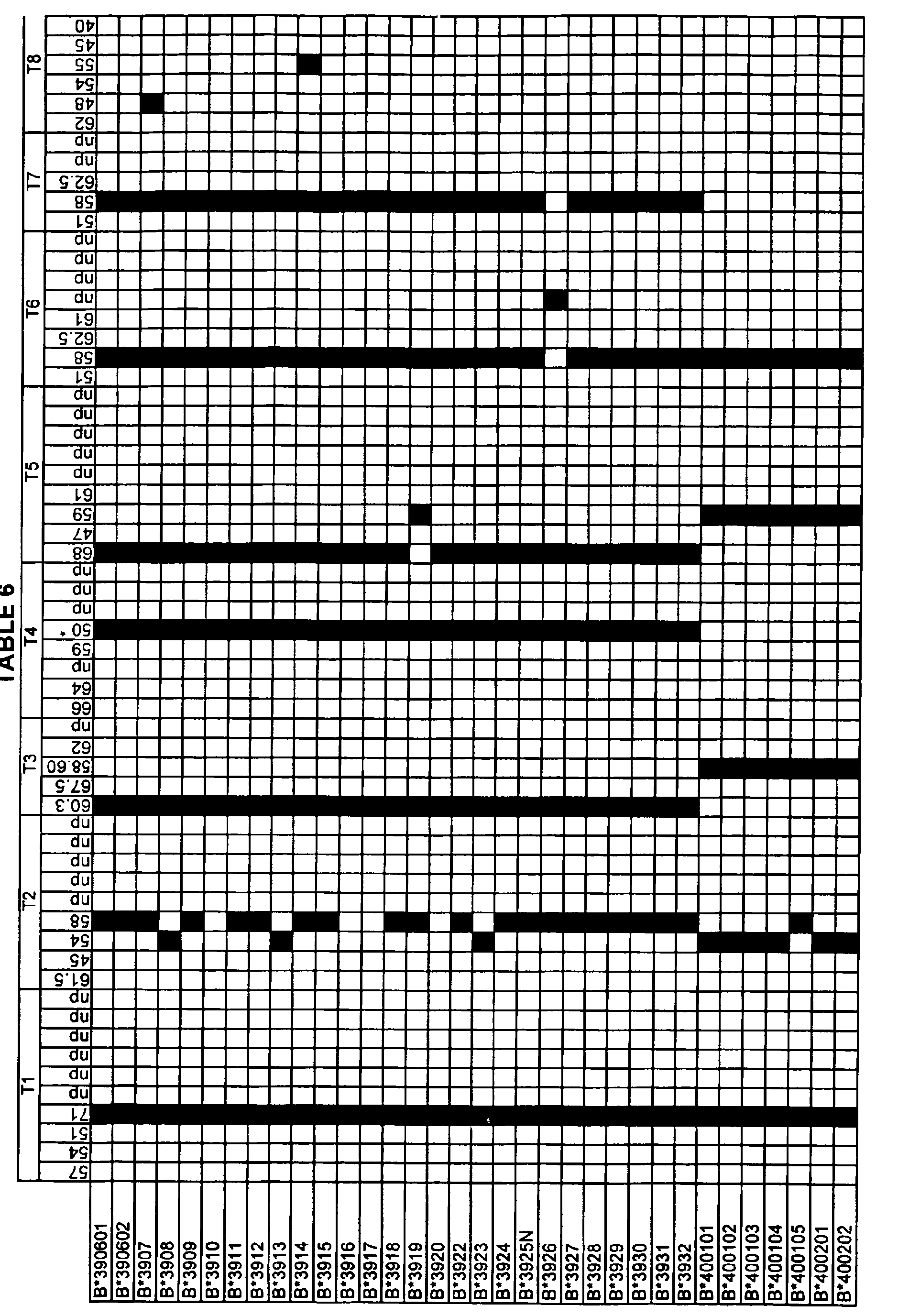

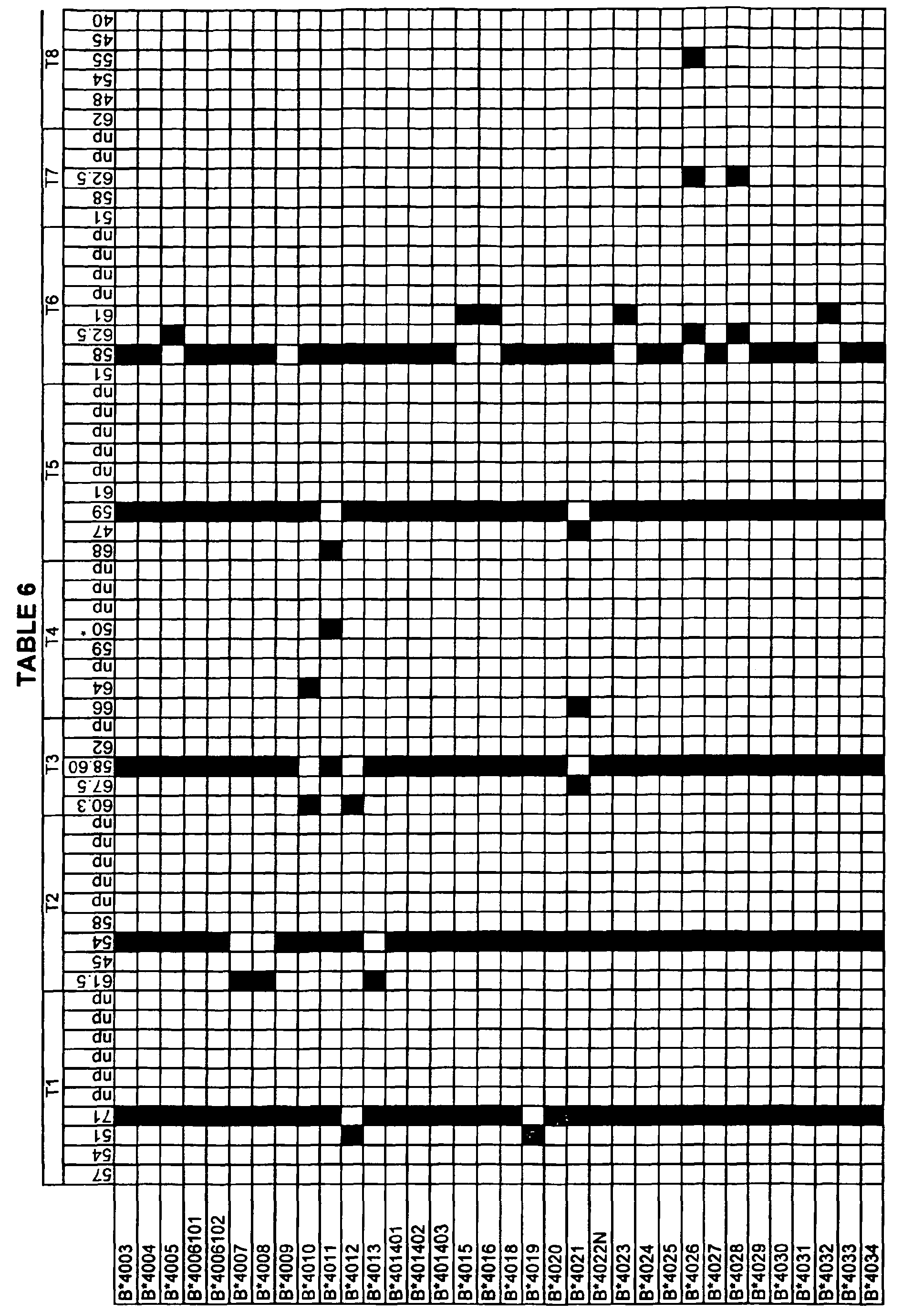

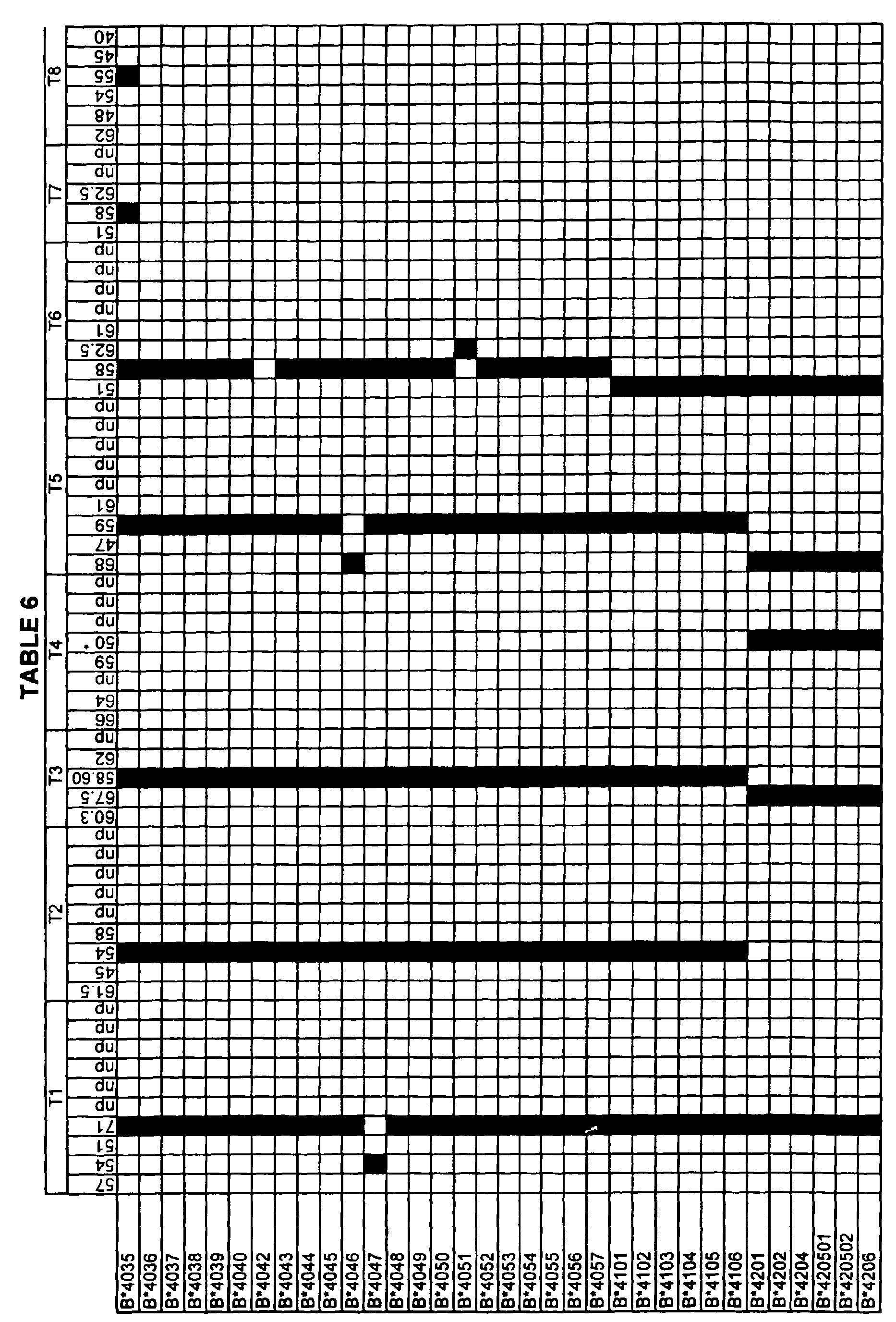

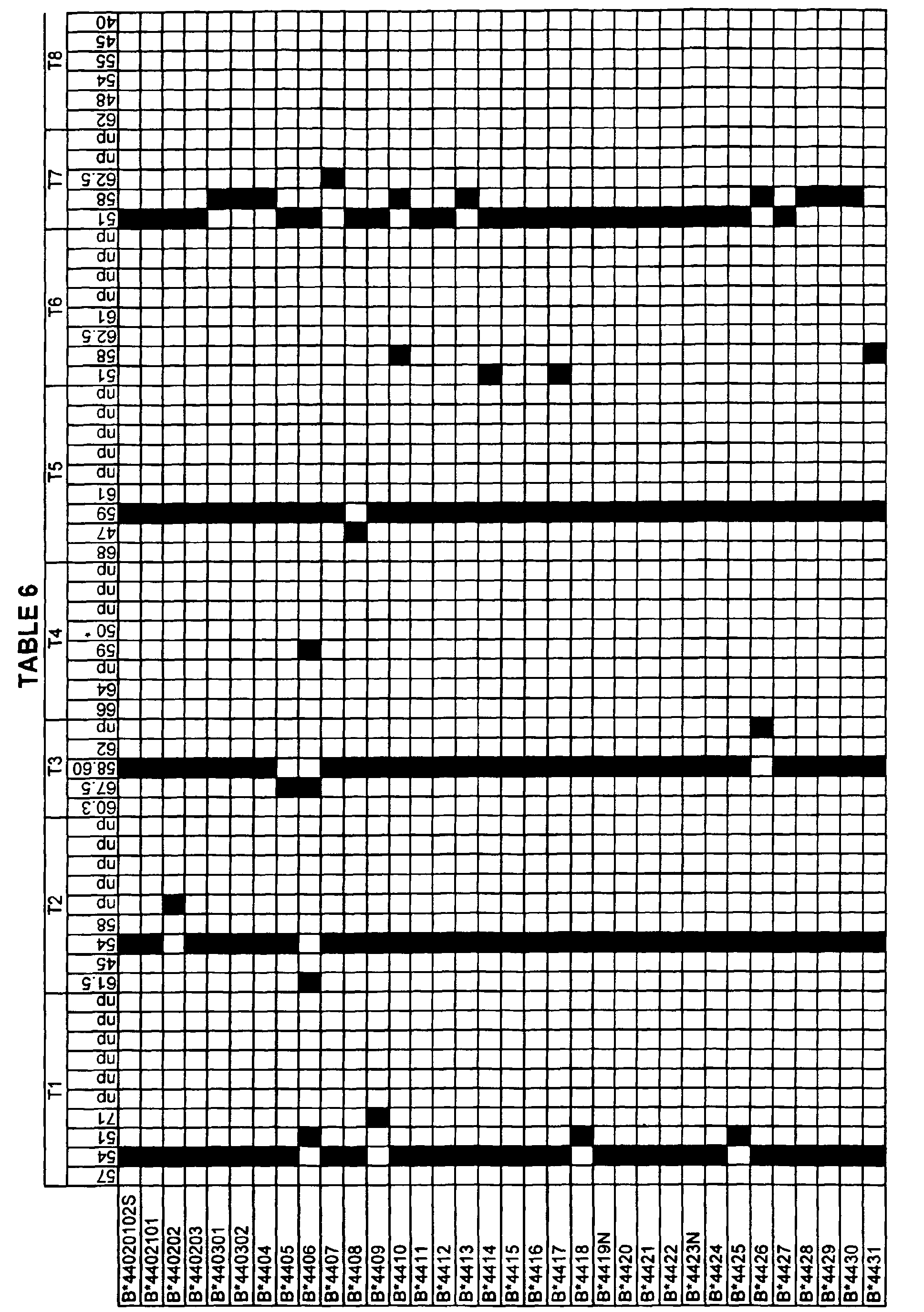

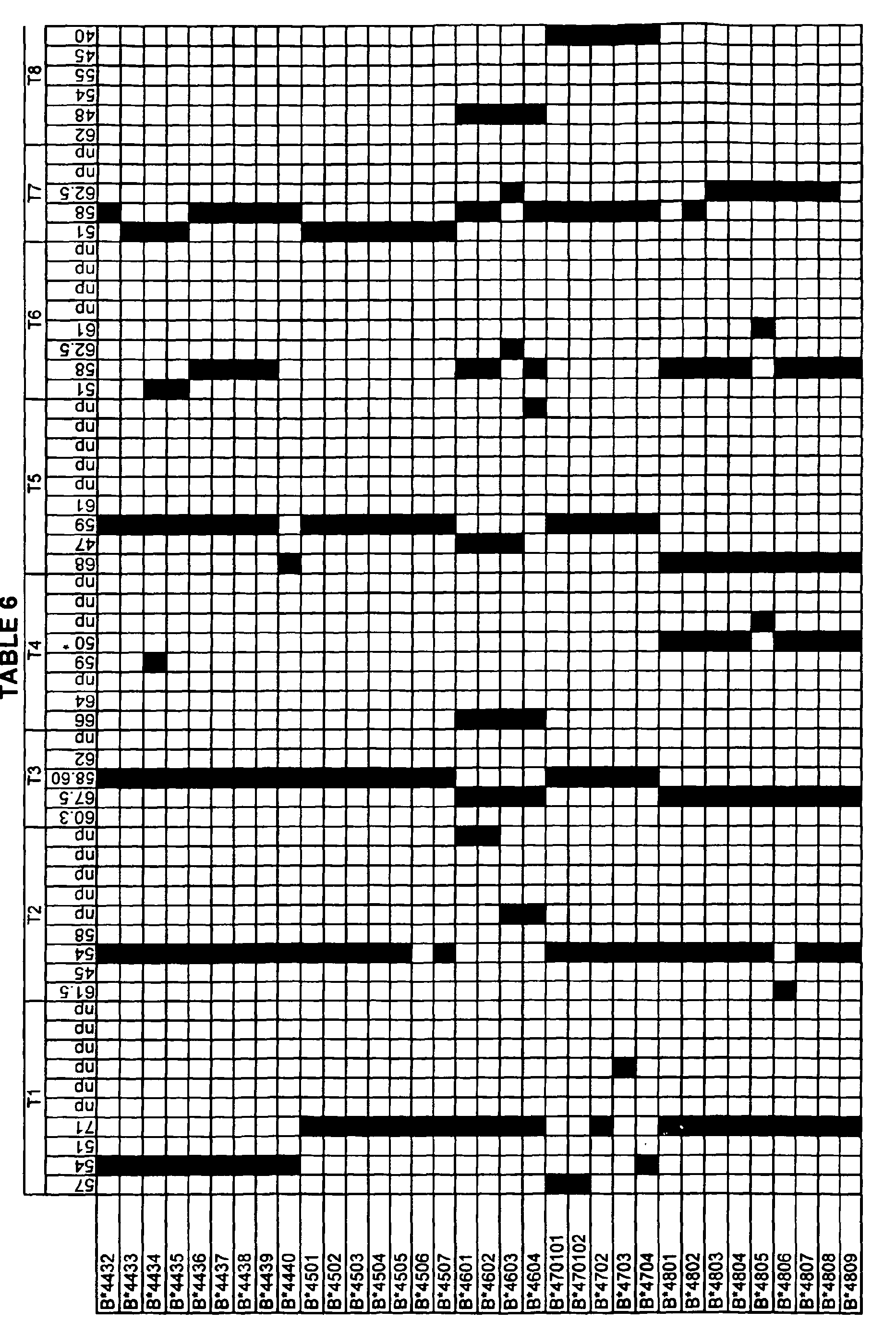

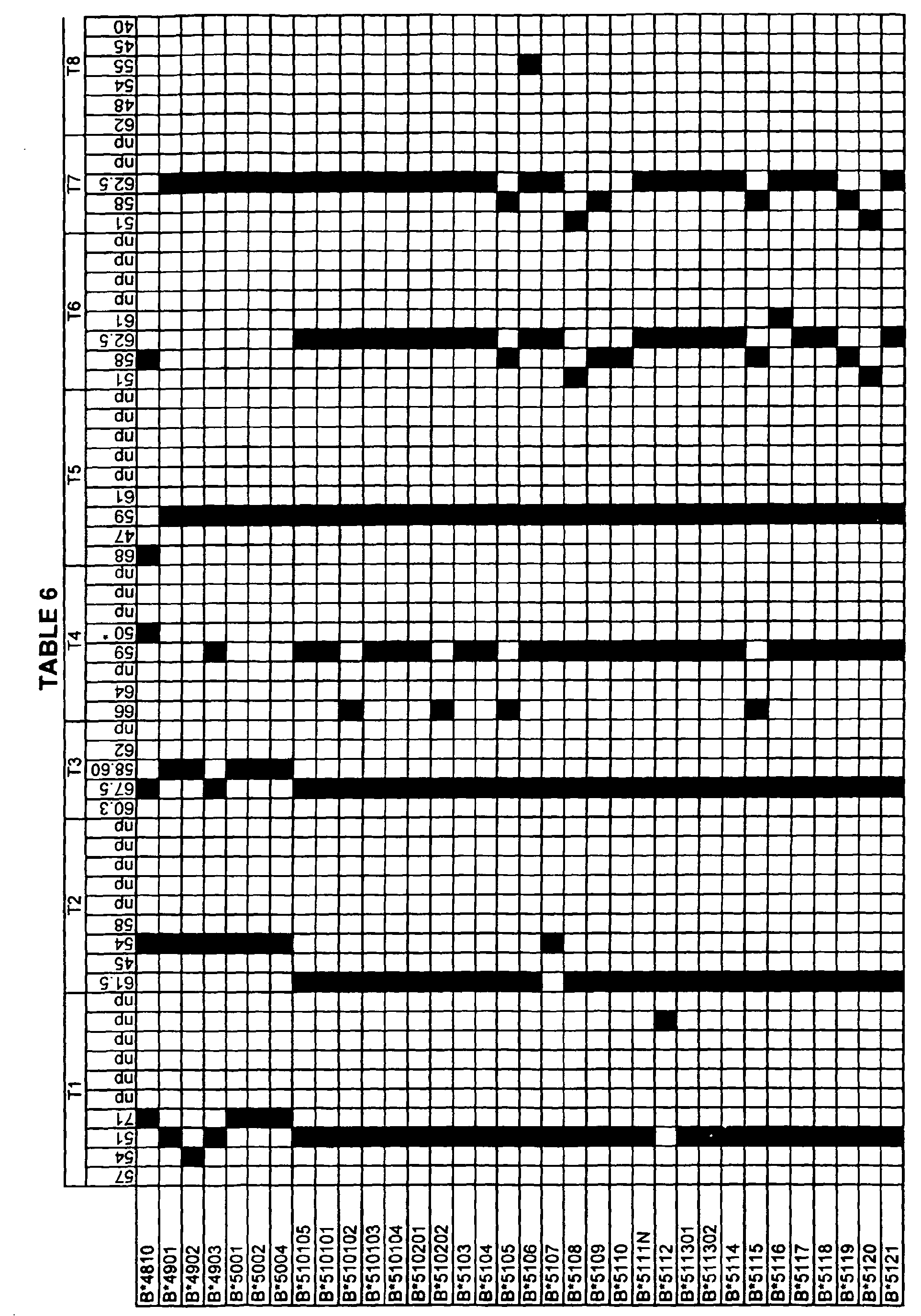

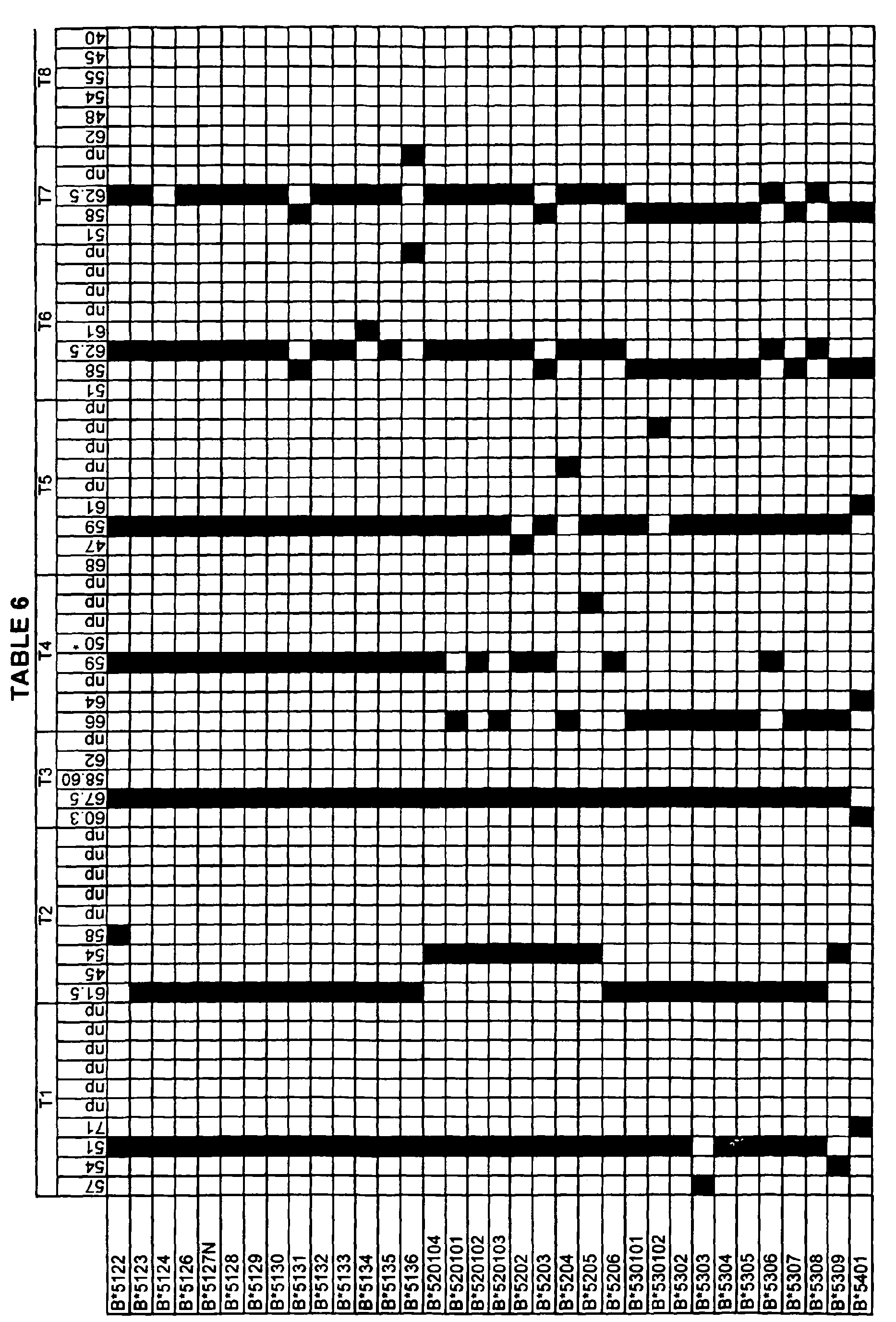

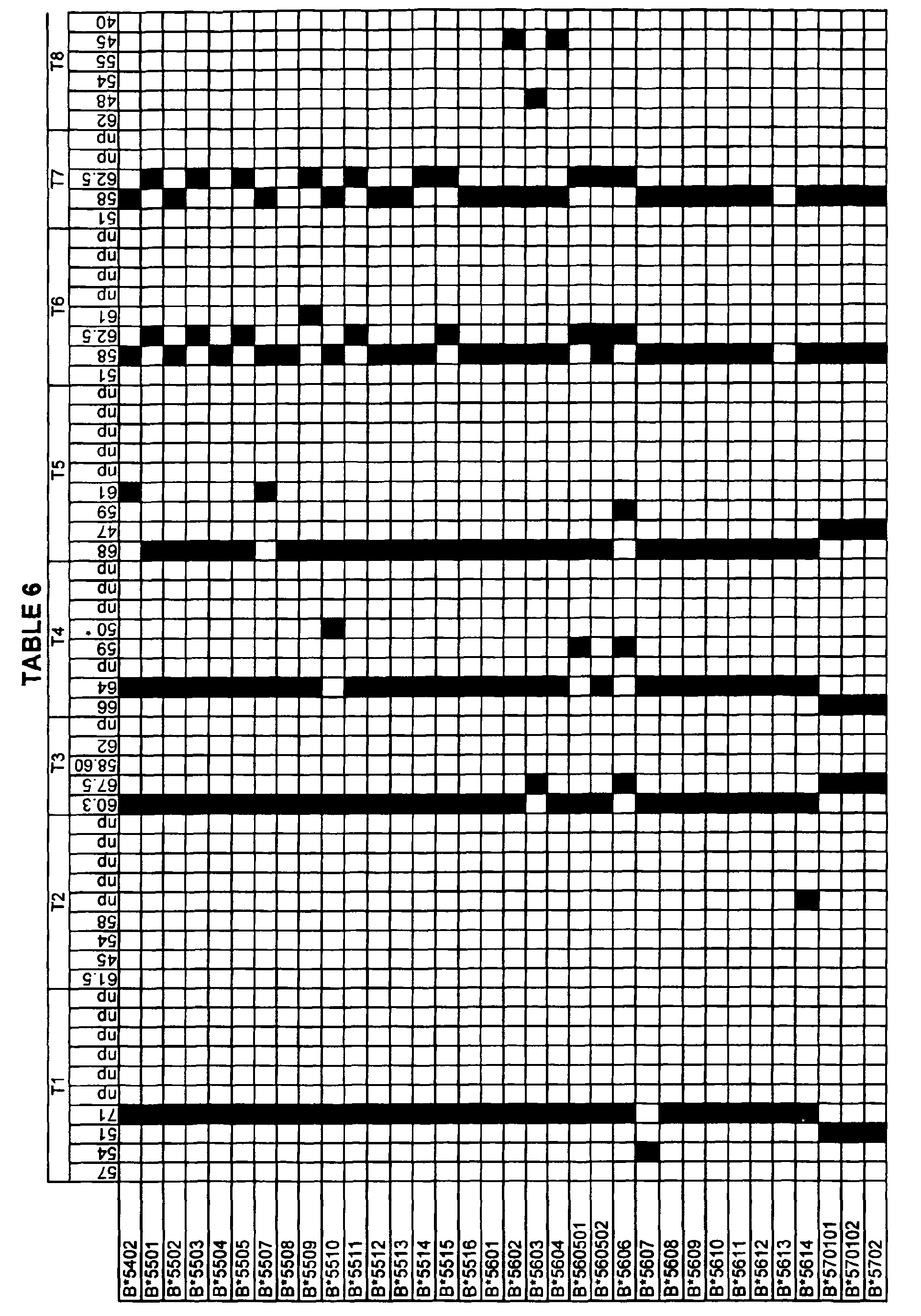

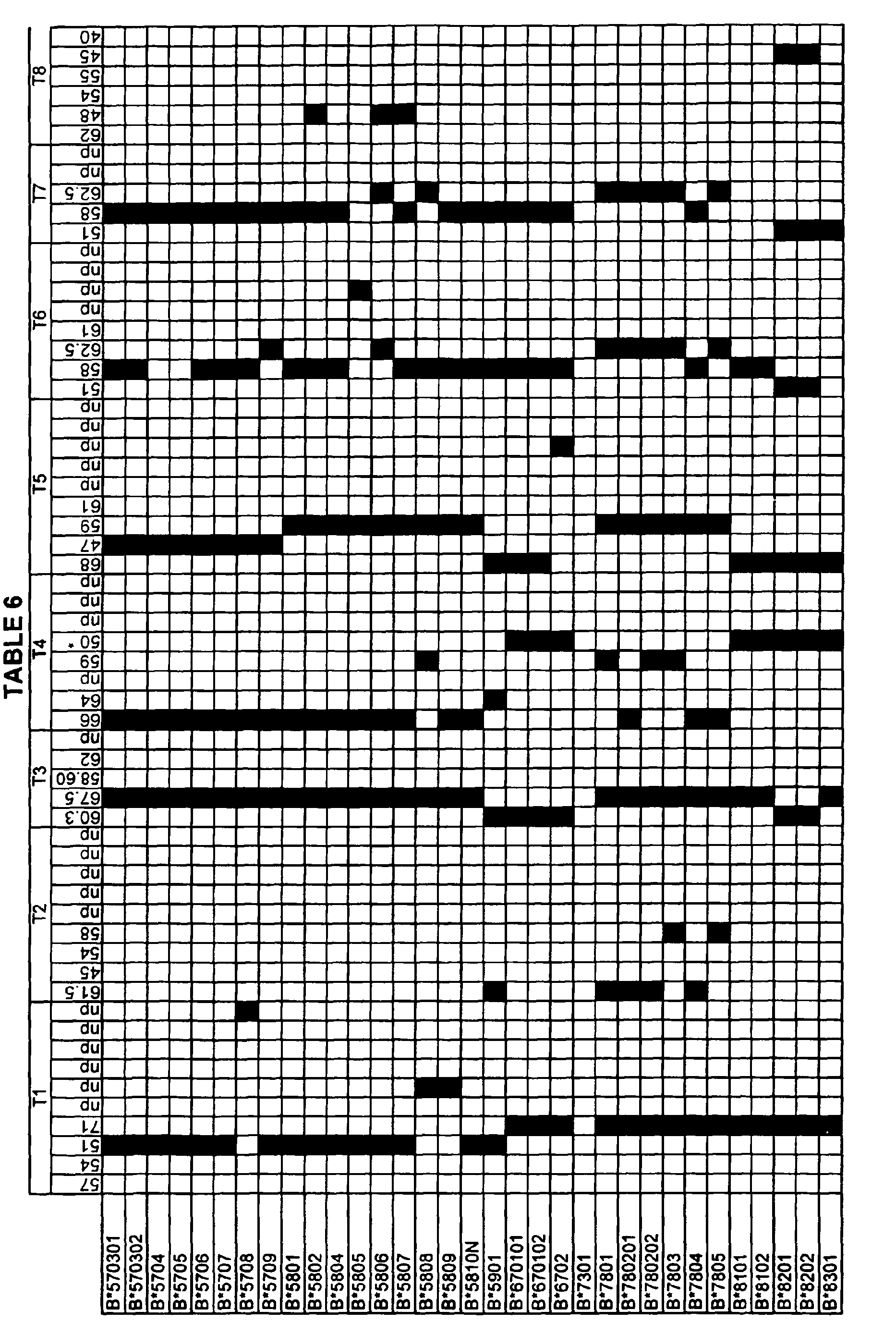

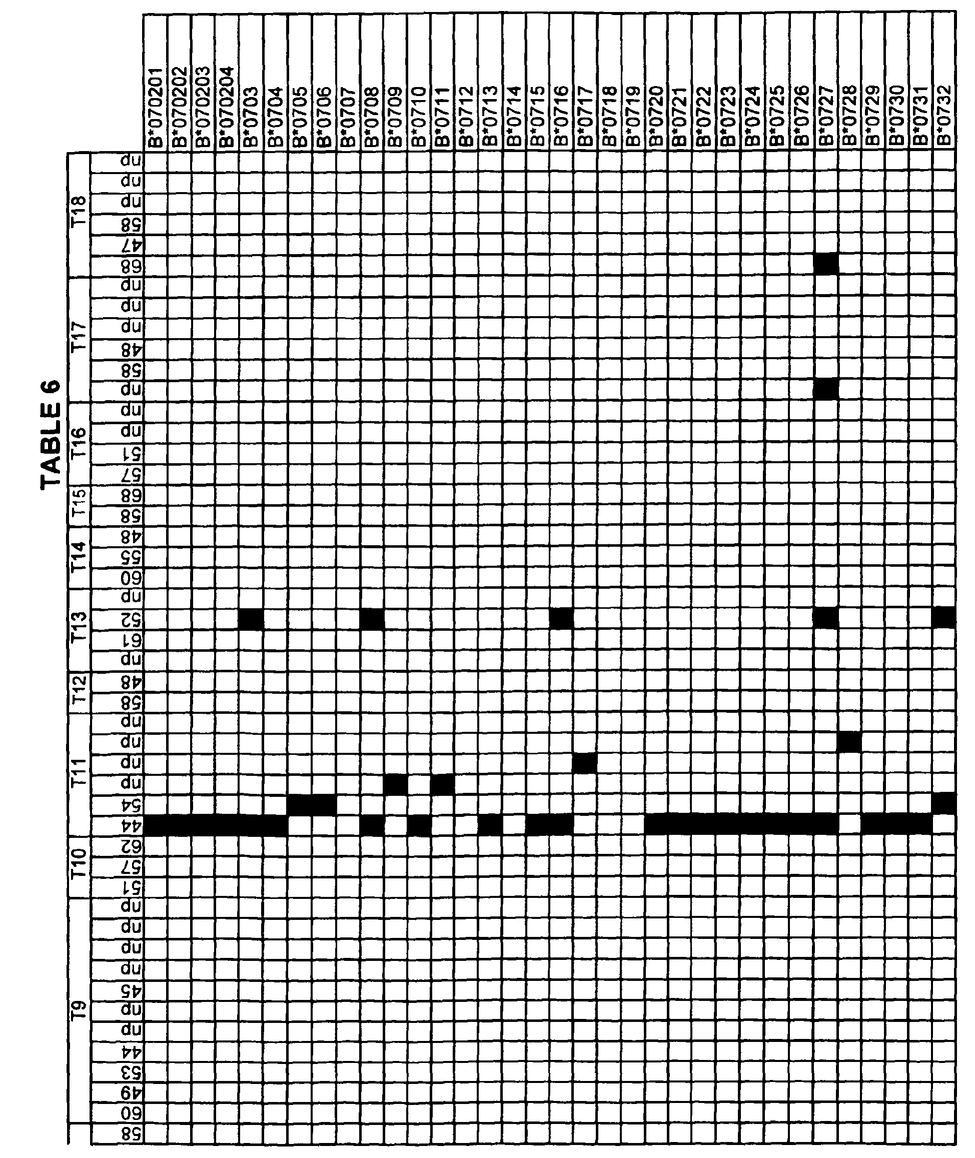

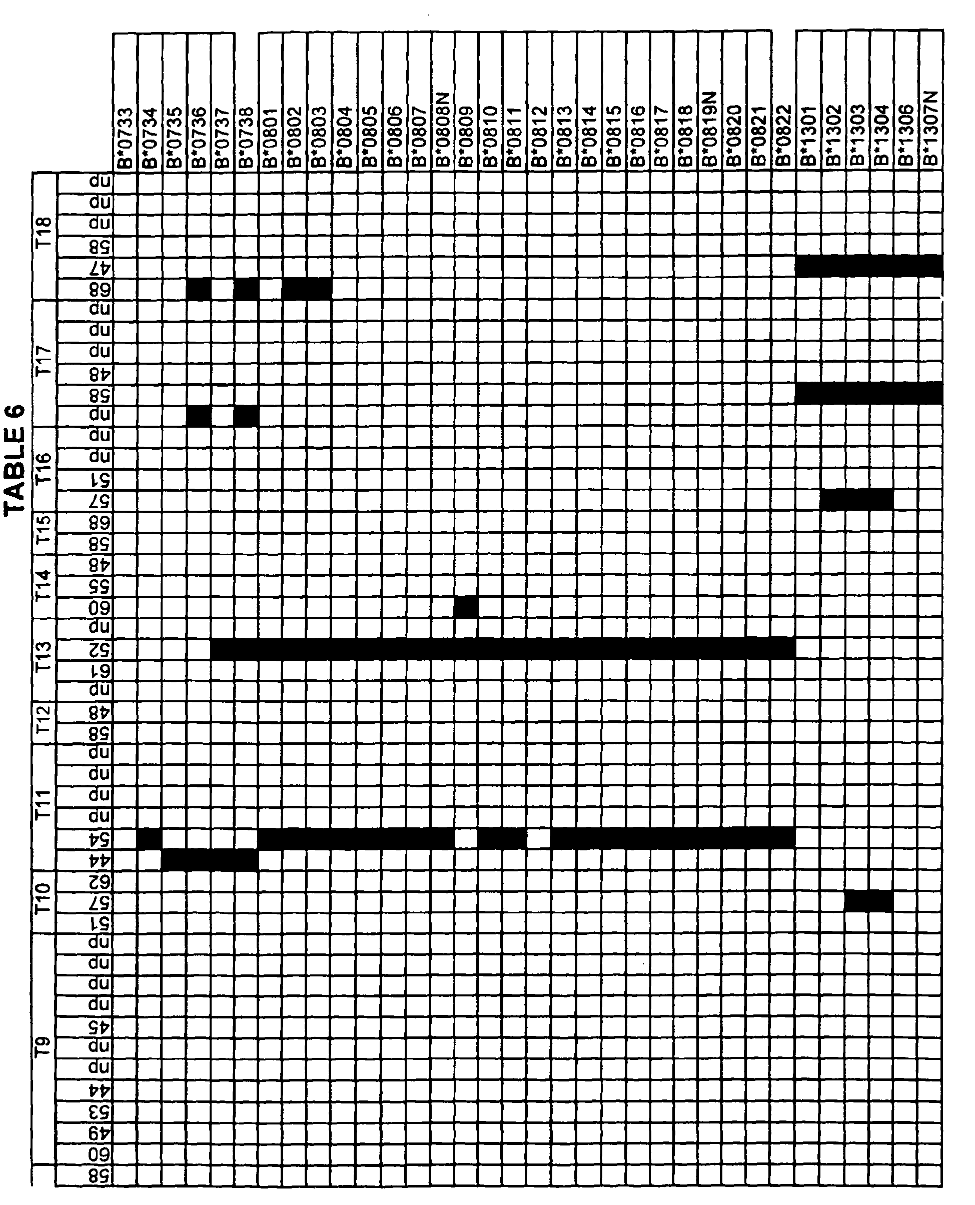

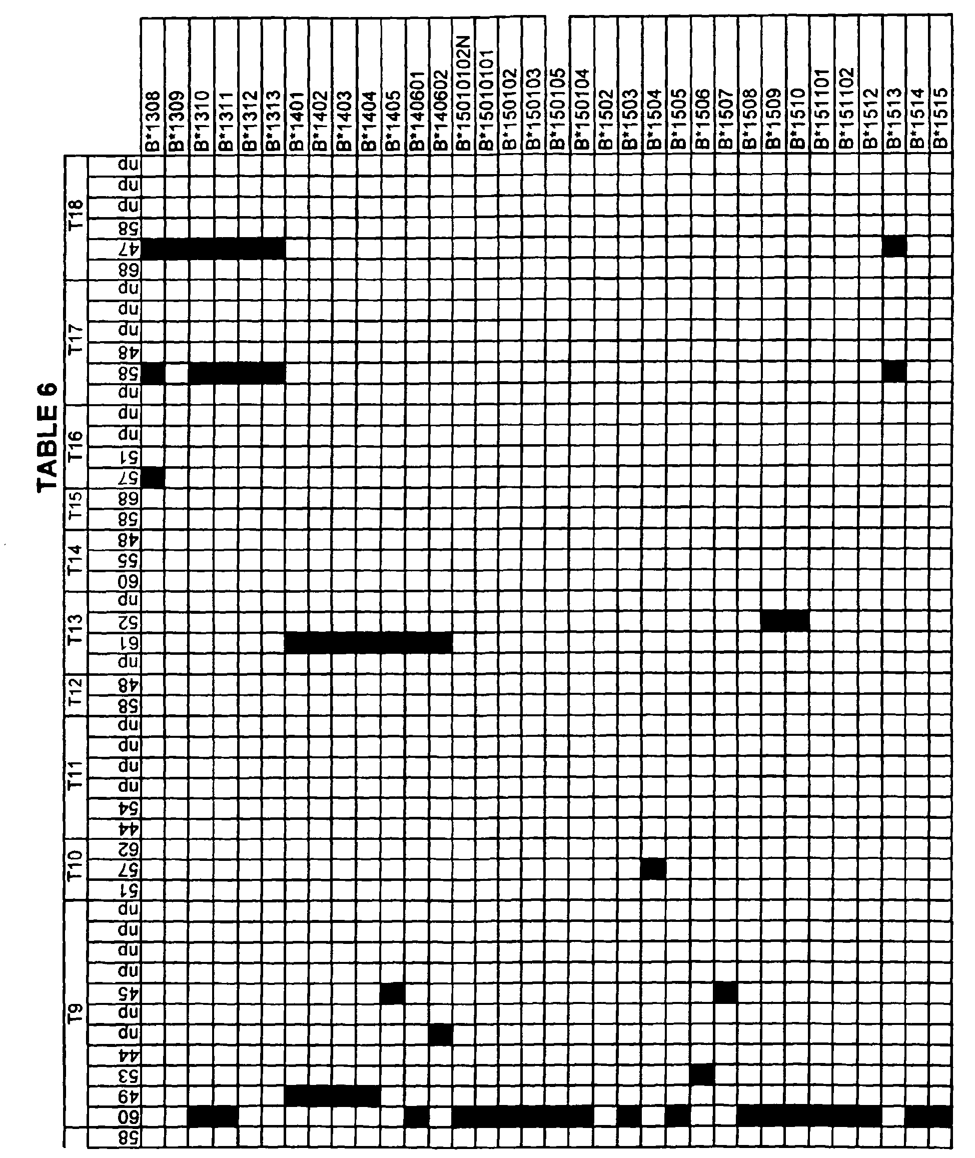

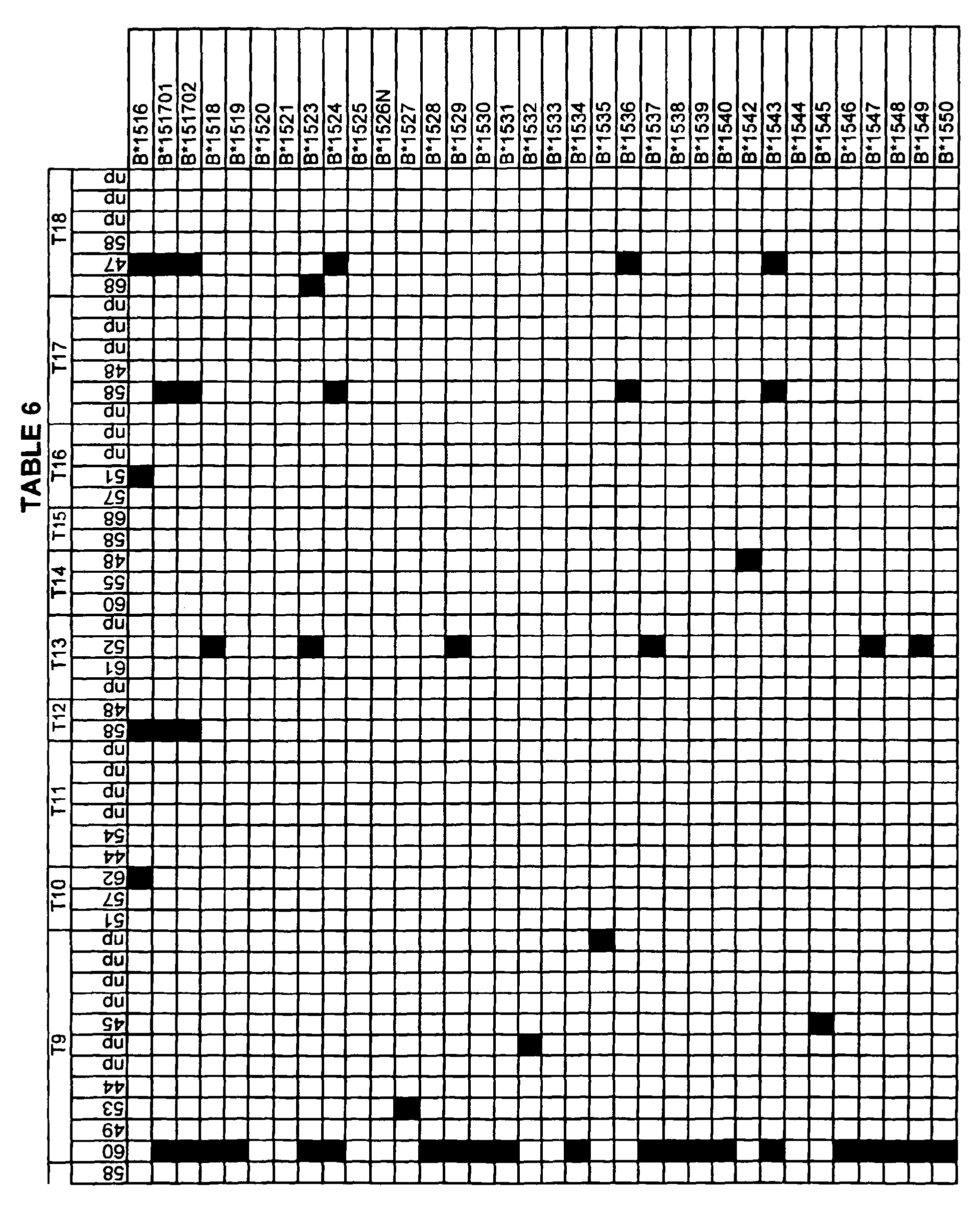

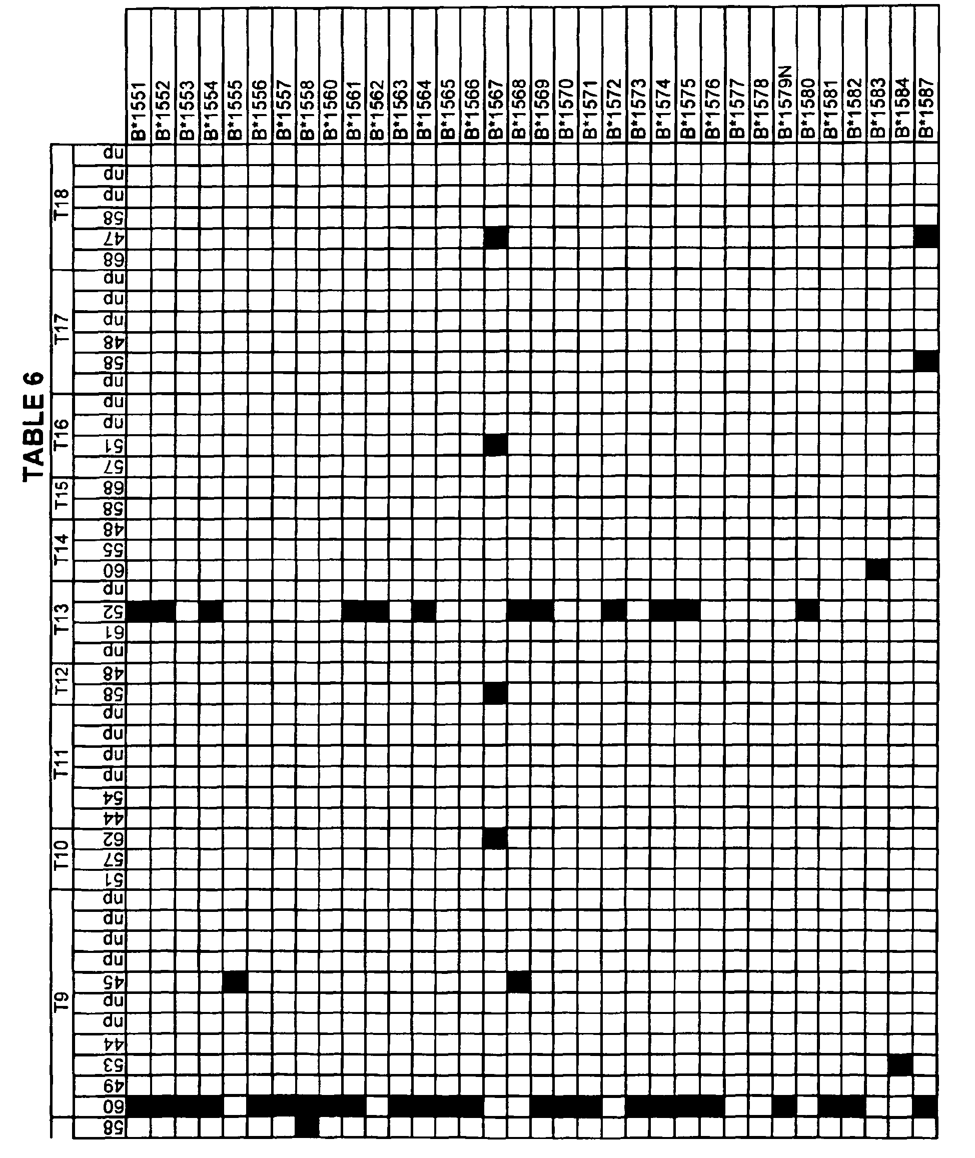

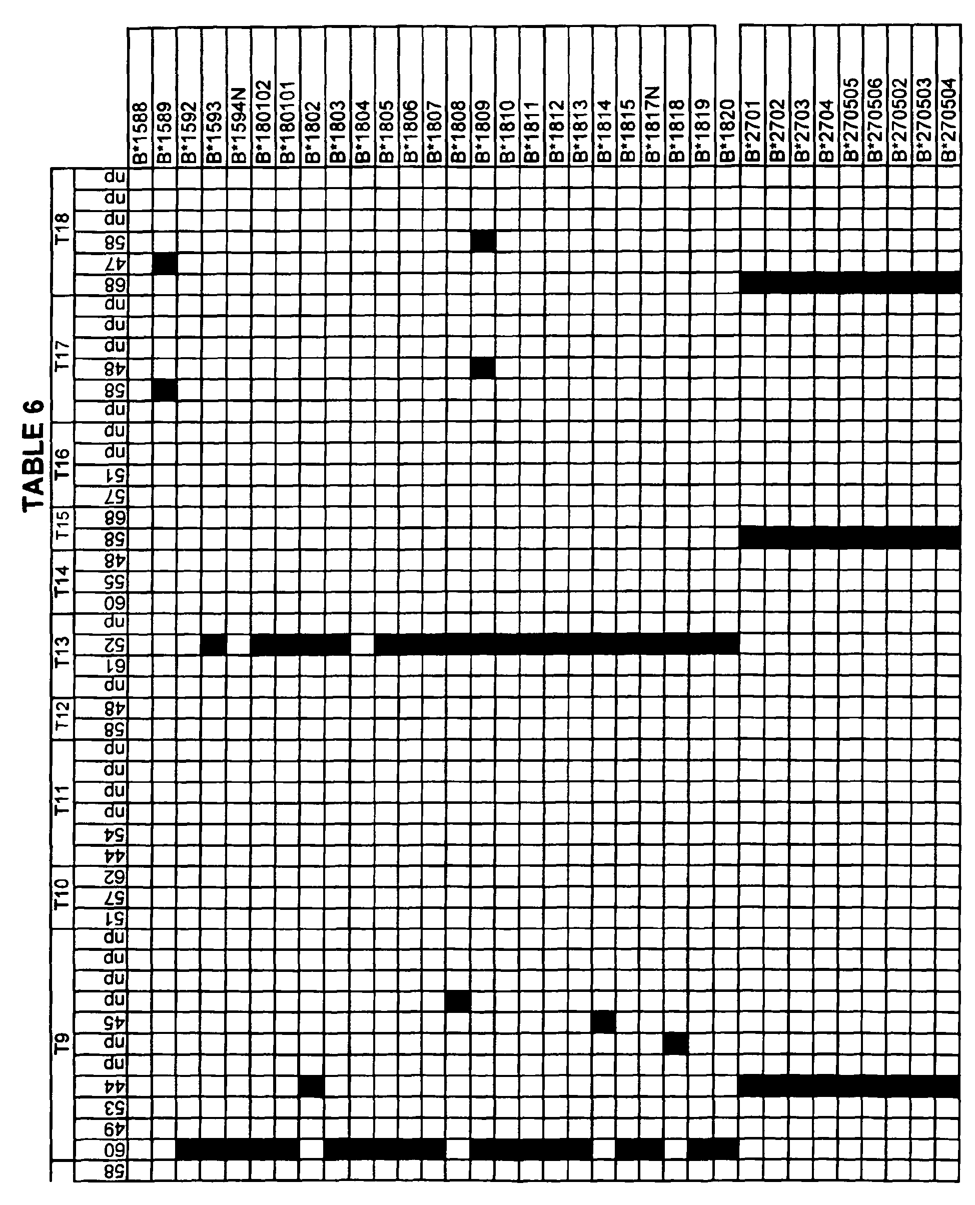

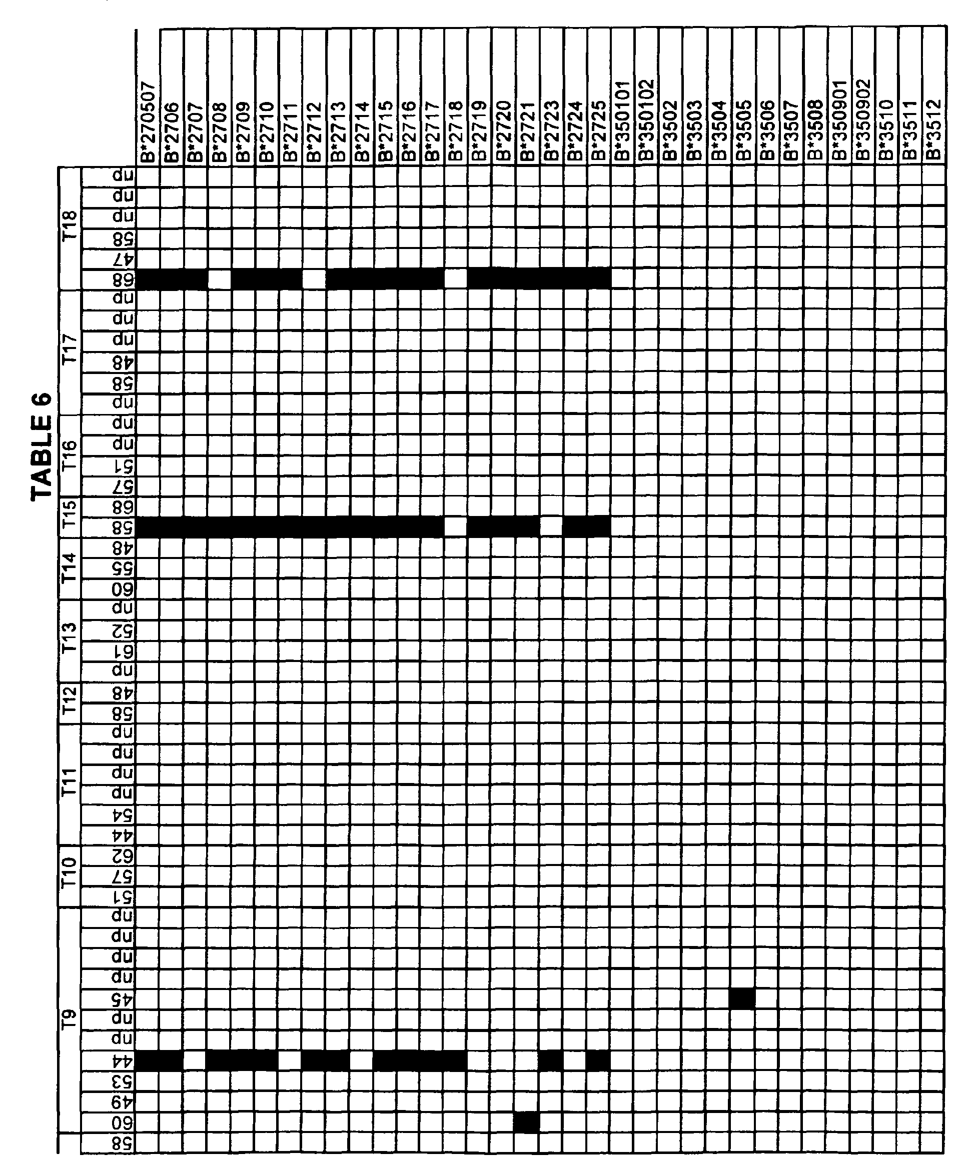

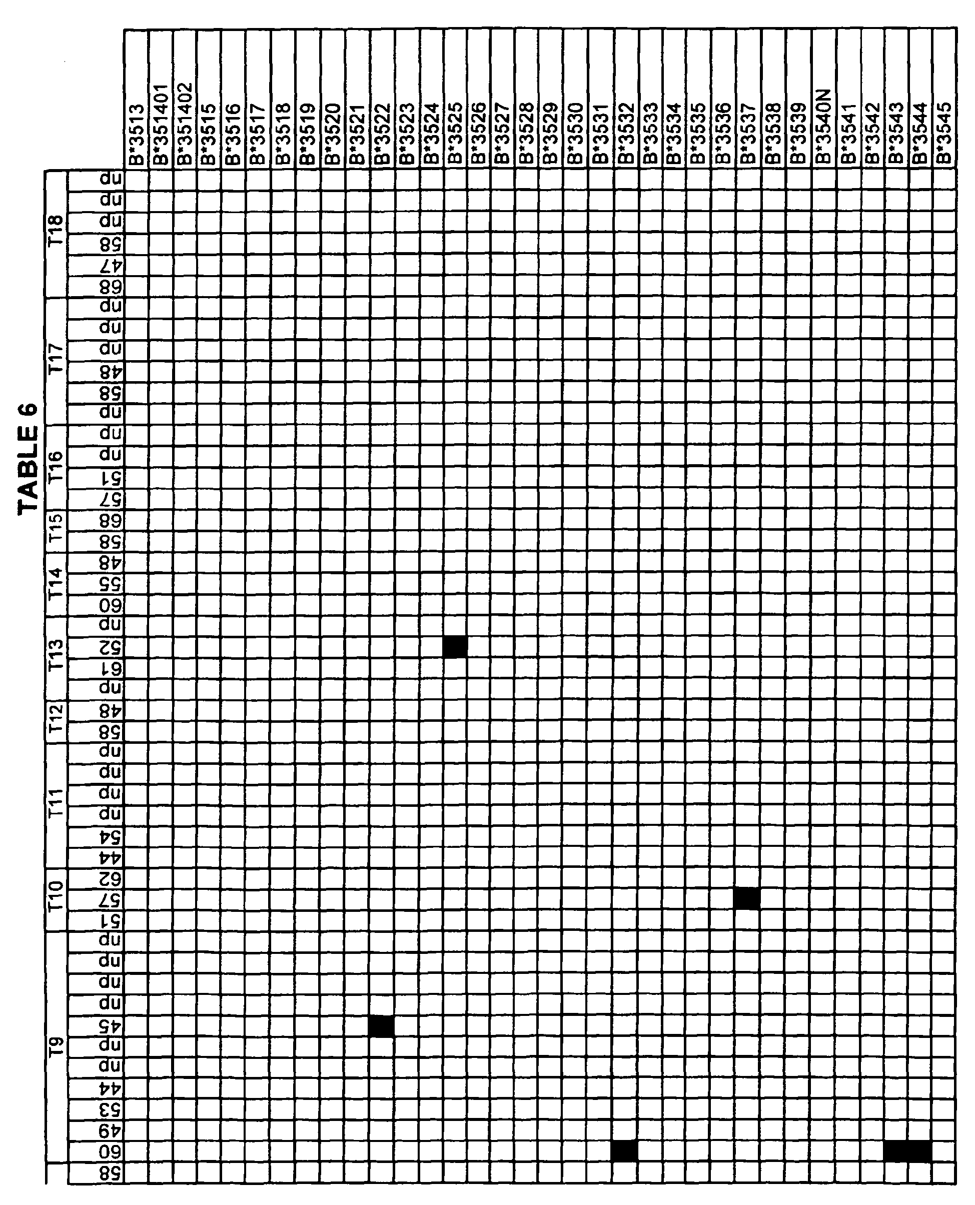

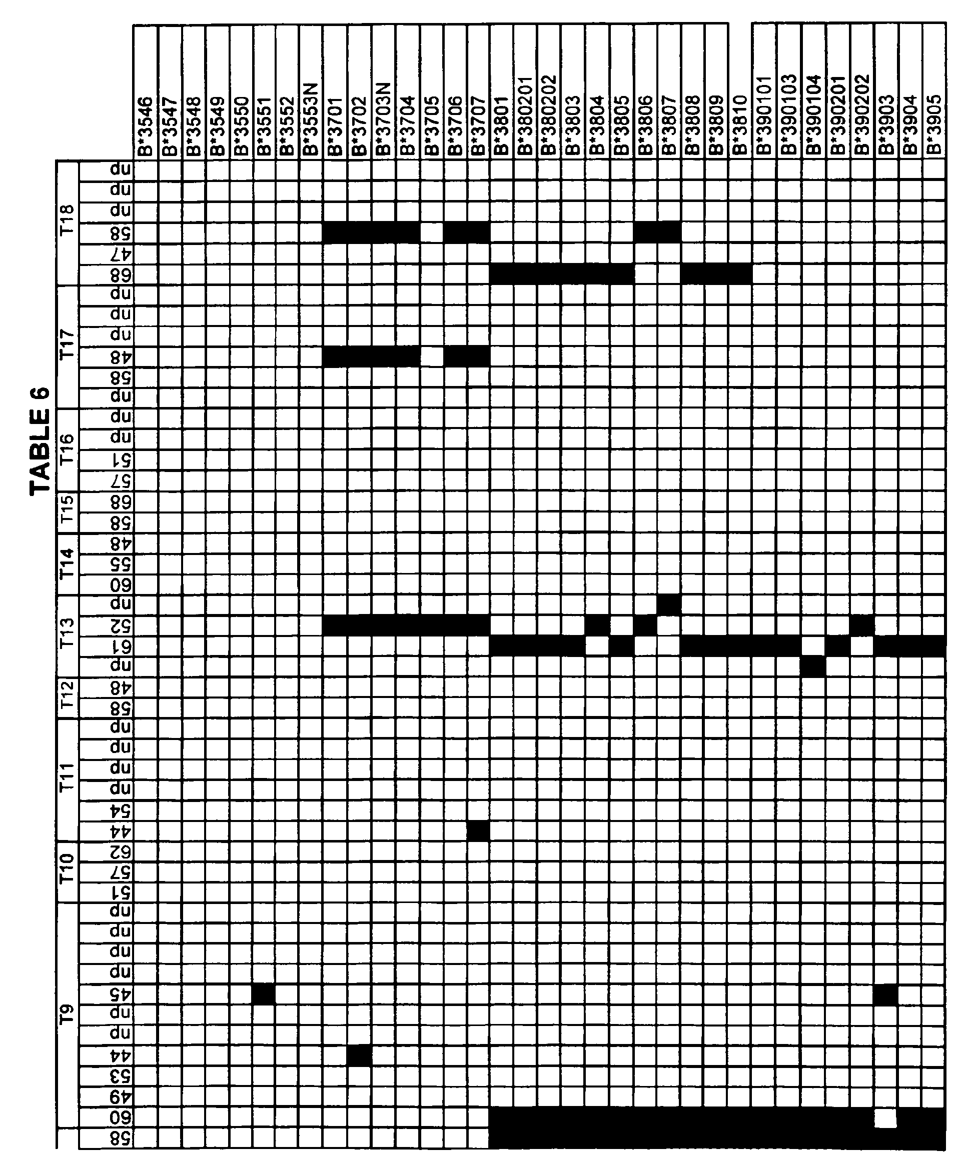

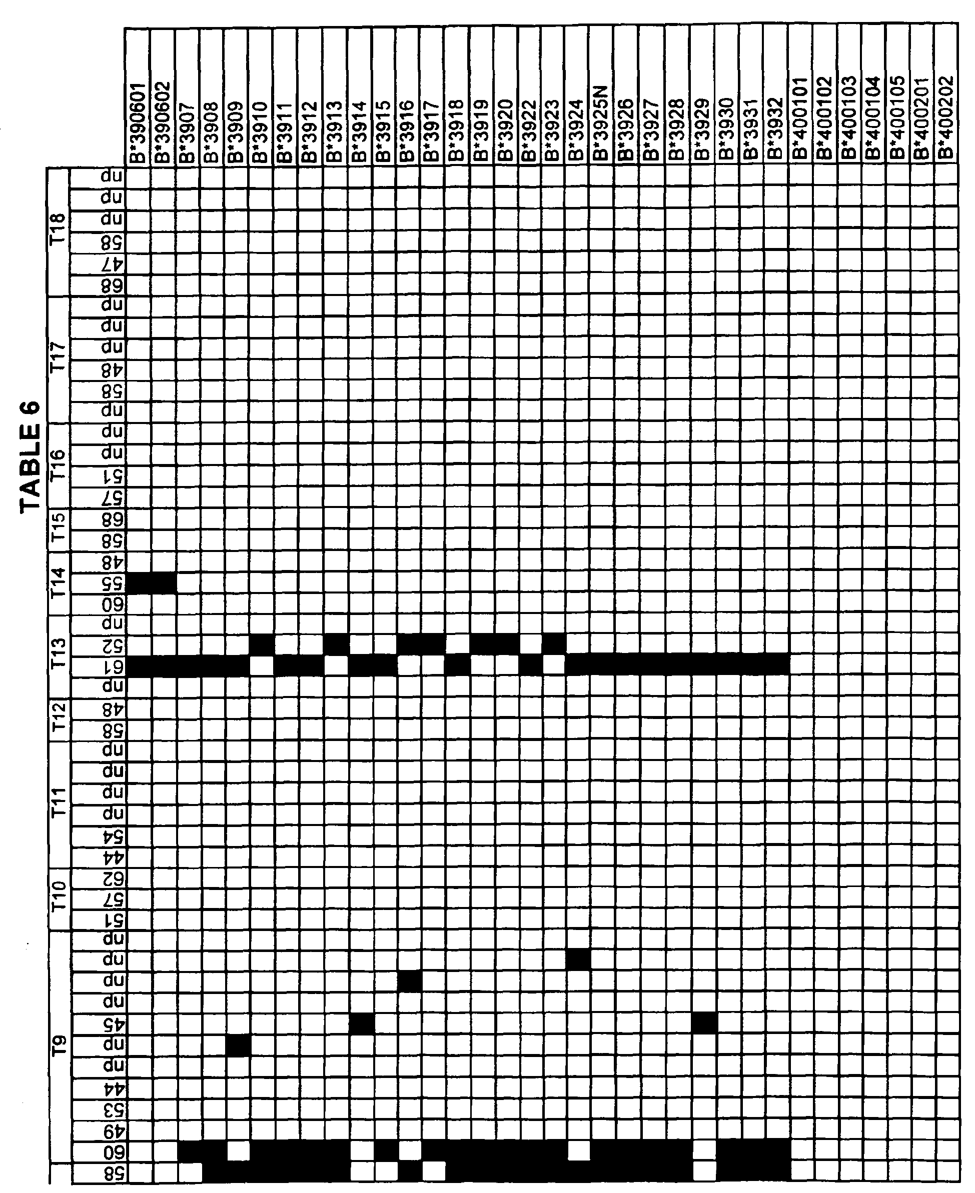

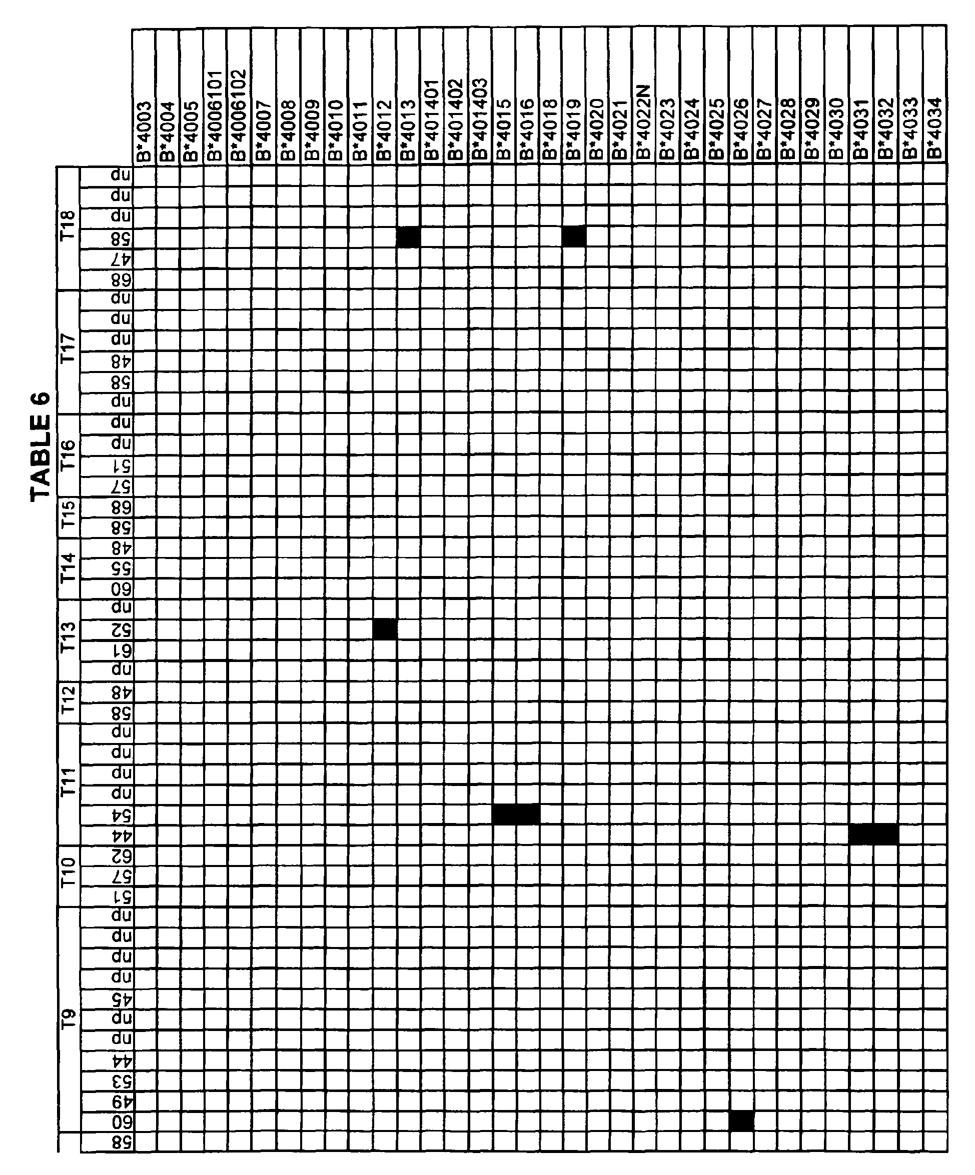

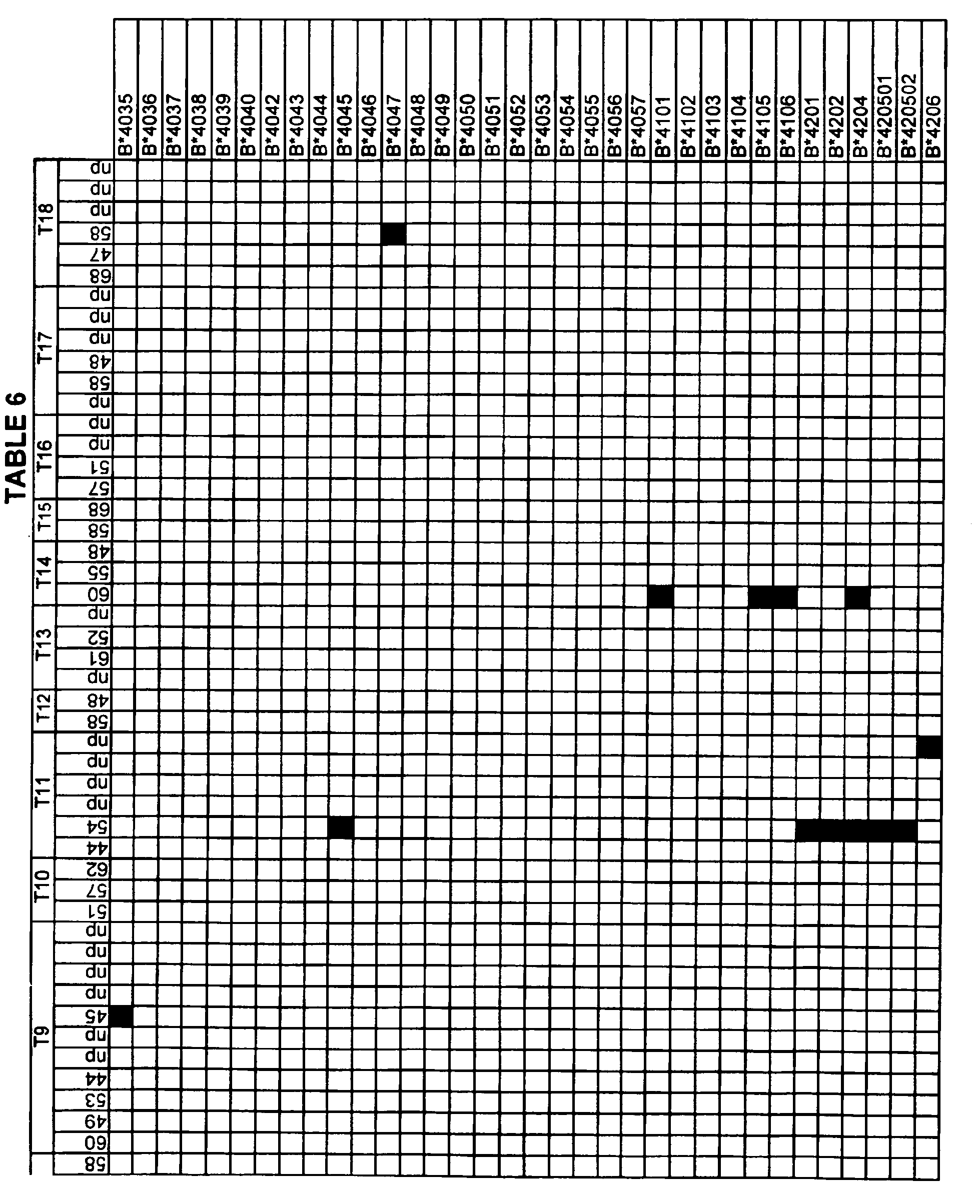

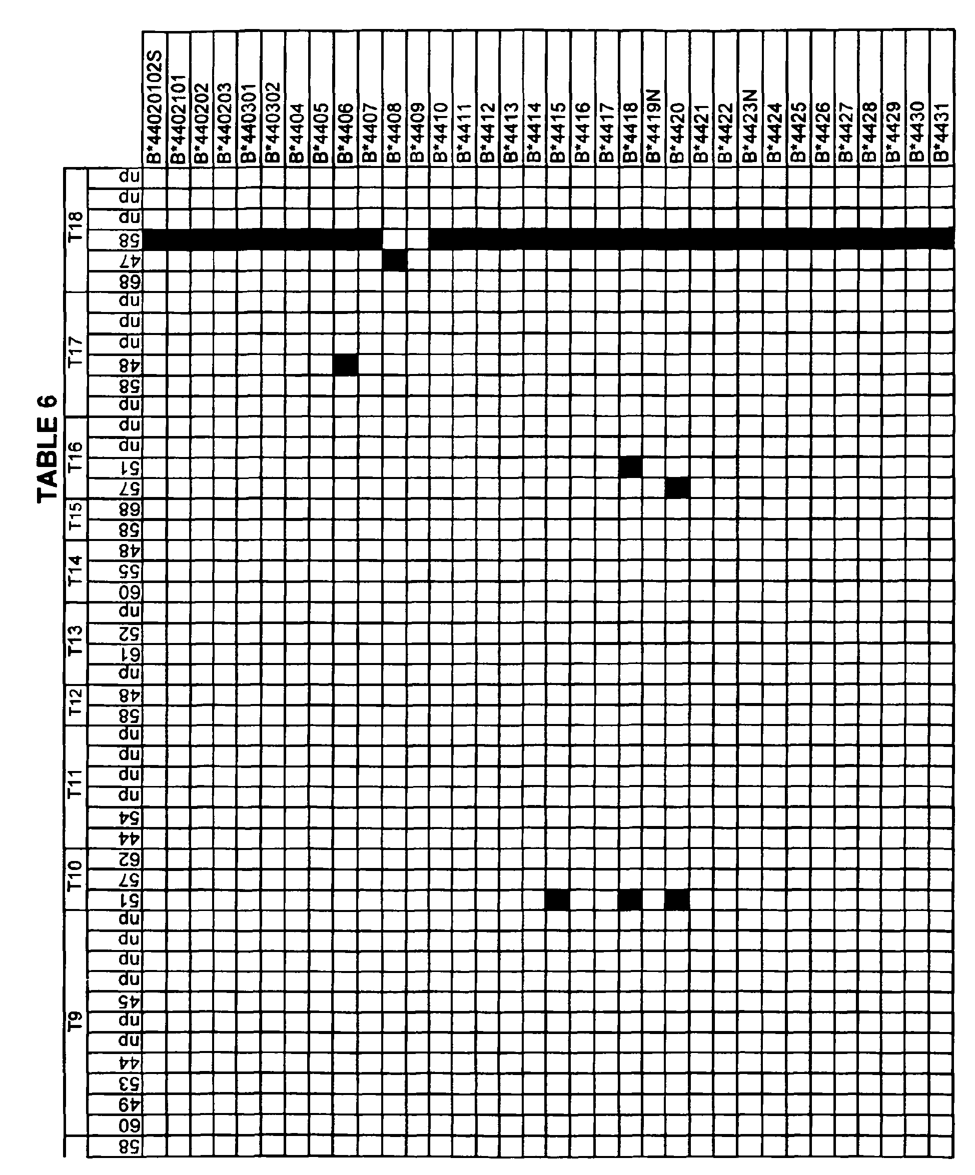

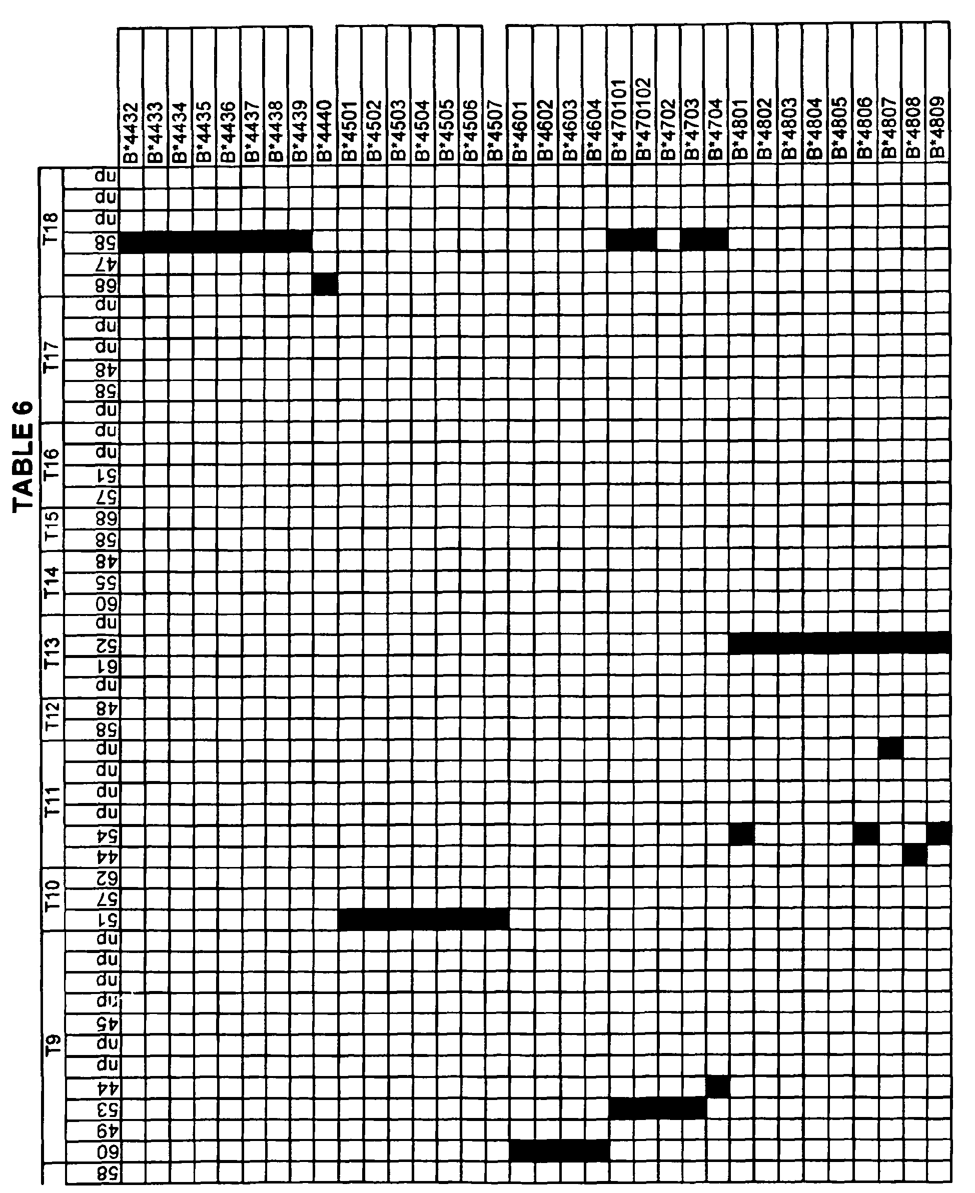

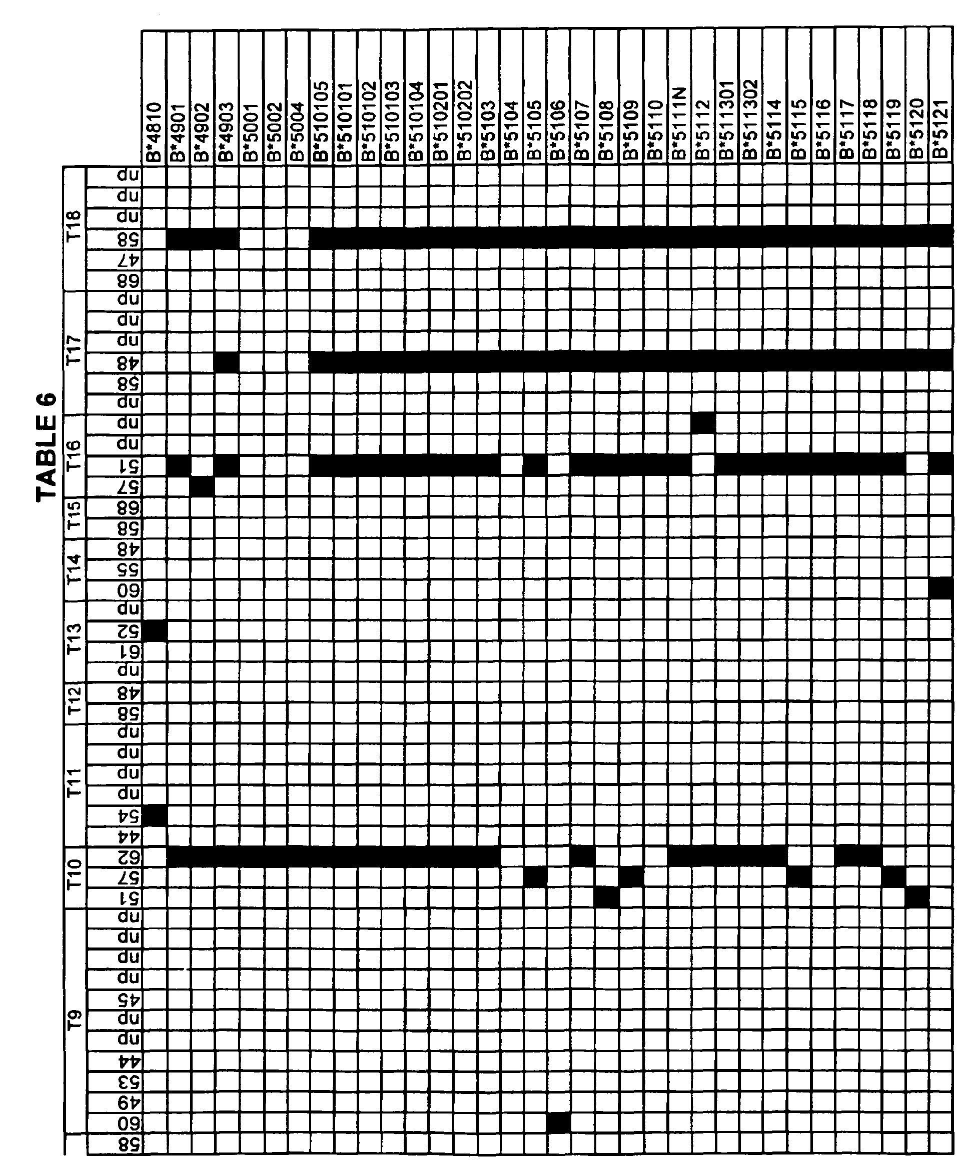

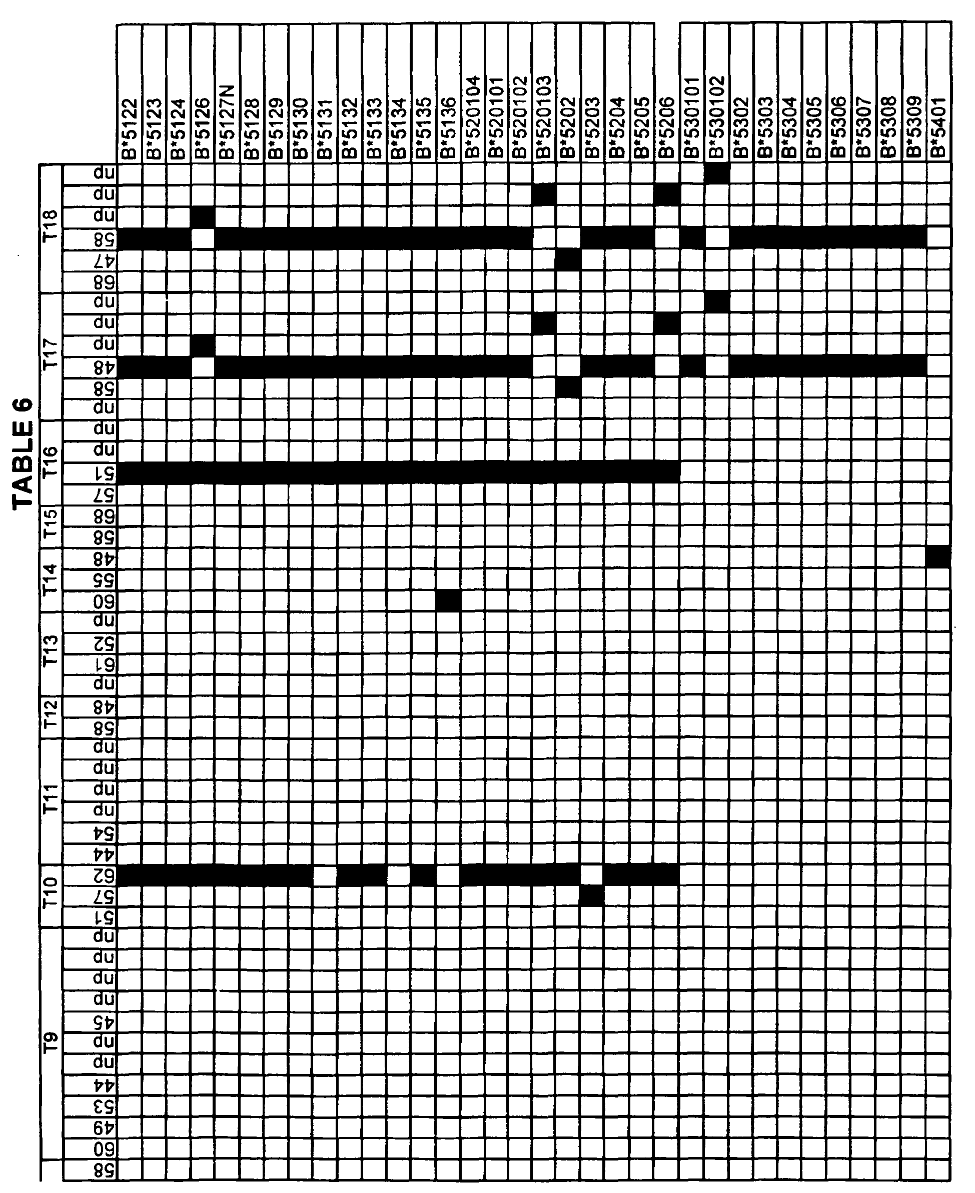

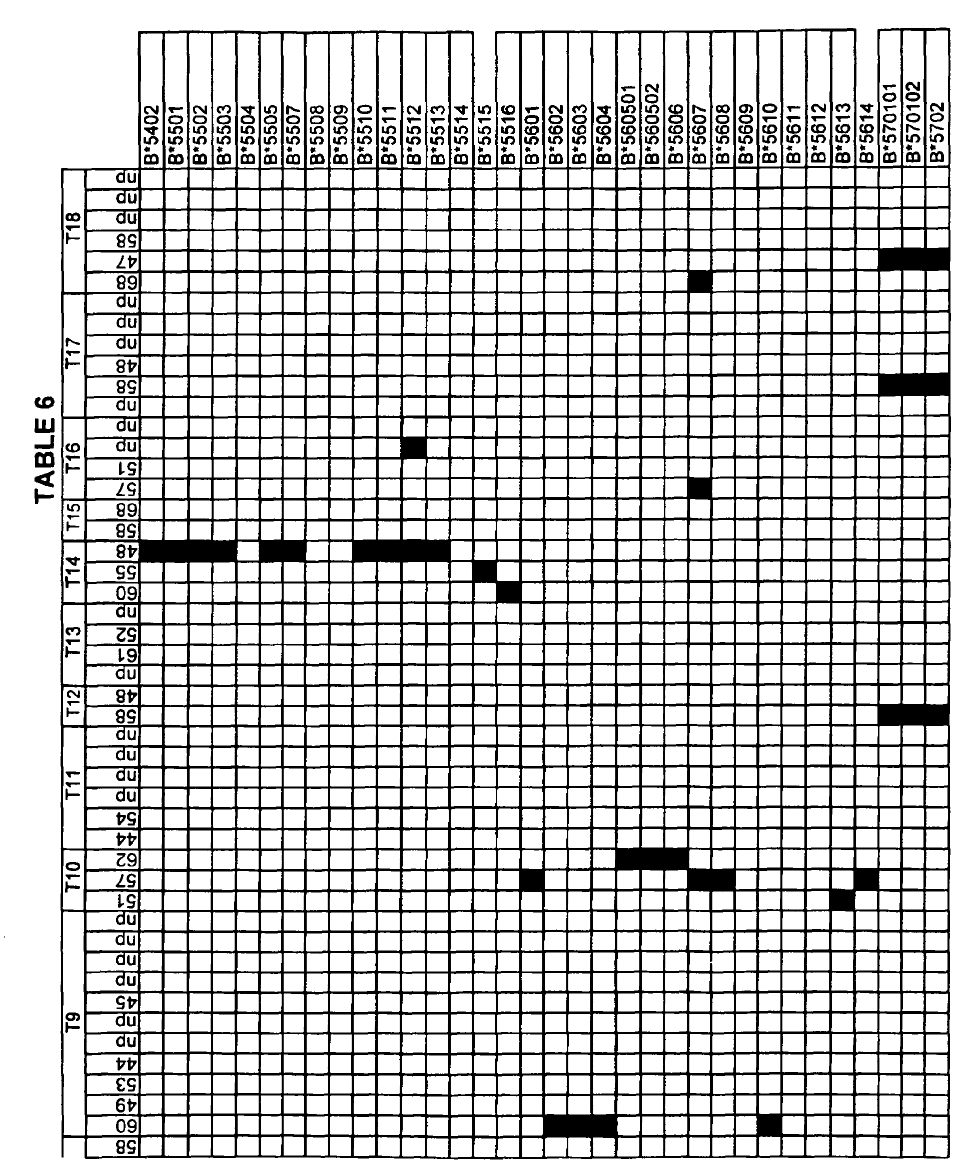

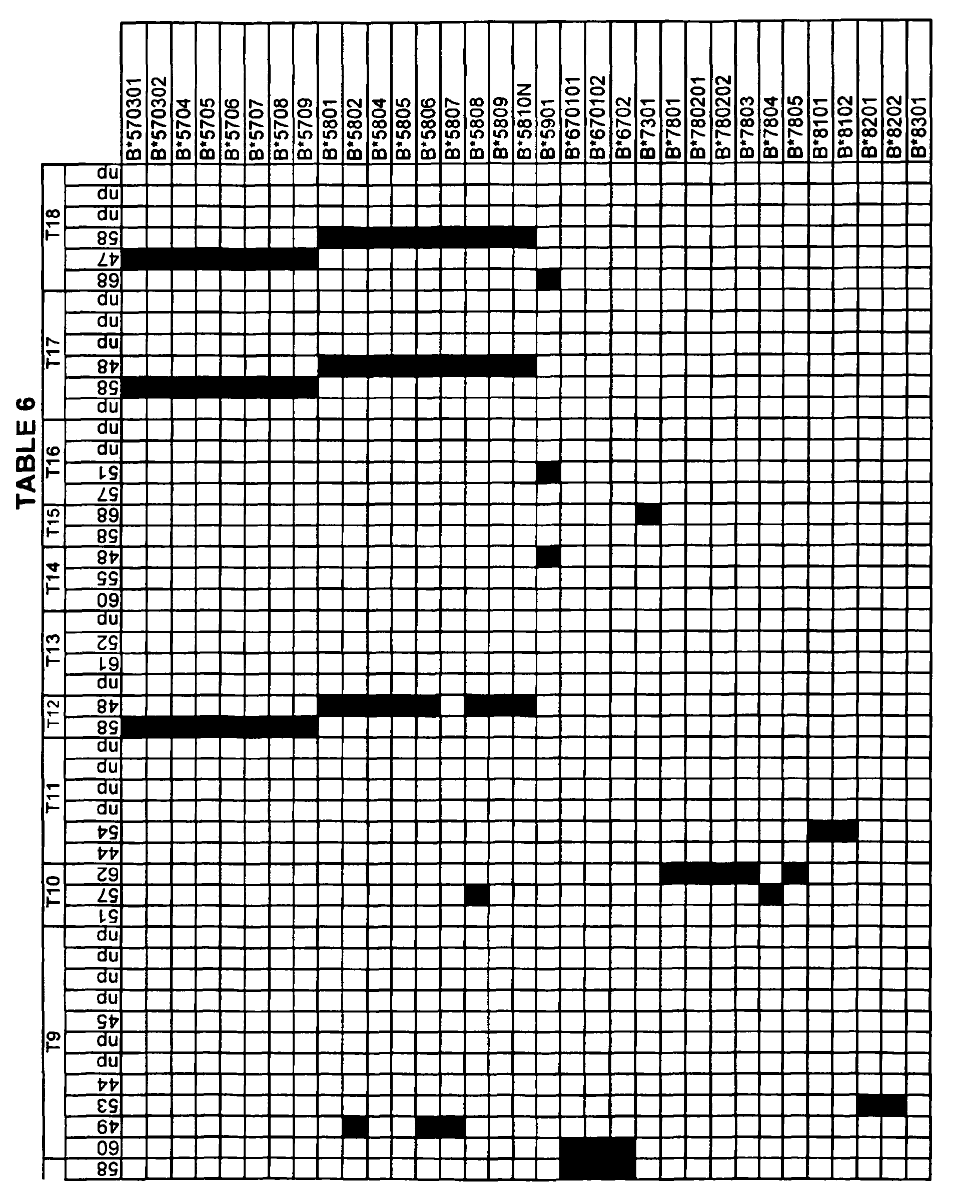

## Claims

1. A method for genotyping the alleles of the human leukocyte antigen B locus (HLA-B) from a nucleic acid sample, which comprises the following steps:
(i) amplifying the nucleic acid of the sample by real-time polymerase chain reaction (PCR) with a battery of containers, the battery comprising the following containers:
container T1: primers consisting of sequences SEQ ID NOs: 15-16 and probes consisting of sequences SEQ ID NOs: 46-47,
container T2: primers consisting of sequences SEQ ID NOs: 15-16 and probes consisting of sequences SEQ ID NOs: 48-49,
container T3: primers consisting of sequences SEQ ID NOs: 15-16 and probes consisting of sequences SEQ ID NOs: 42-43,
container T4: primers consisting of sequences SEQ ID NOs: 15-16 and probes consisting of sequences SEQ ID NOs: 44-45,
container T5: primers consisting of sequences SEQ ID NOs: 15-16 and probes consisting of sequences SEQ ID NOs: 36 and 50,
container T6: primers consisting of sequences SEQ ID NOs: 17-18 and probes consisting of sequences SEQ ID NOs: 40-41, and
container T7: primers consisting of sequences SEQ ID NOs: 18-19 and probes consisting of sequences SEQ ID NOs: 40-41,
wherein the probes are labelled with a fluorescent label;
(ii) detecting fluorescent signals of the probes following the amplification performed in step (i), and analysing the melting temperatures of the probes hybridized to the amplified nucleic acid sequences; and
(iii) comparing the pattern resulting from the combination of all the melting temperatures obtained in step (ii) with each row diclosed in Table 6 corresponding to a pattern of melting temperatures,
so that, when the obtained pattern of melting temperatures matches the pattern of a row of TABLE 6, the allele specified in the first column of the row is determined.

2. The method according to claim 1, wherein the battery of containers additionally comprises one or more of the following containers:
container T8: primers consisting of sequences SEQ ID NOs: 1-3 and probes consisting of sequences SEQ ID NOs: 32-33,
container T9: primers consisting of sequences SEQ ID NOs: 1-3 and probes consisting of sequences SEQ ID NOs: 38-39,
container T10: primers consisting of sequences SEQ ID NOs: 10-12 and probes consisting of sequences SEQ ID NOs: 40-41,
container T11: primers consisting of sequences SEQ ID NOs: 13-14 and probes consisting of sequences SEQ ID NOs: 32-33;
container T13: primers consisting of sequences SEQ ID NOs: 4-7 and probes consisting of sequences SEQ ID NOs: 34-35,
container T14: primers consisting of sequences SEQ ID NOs: 22-23 and probes consisting of sequences SEQ ID NOs: 33 and 51, and
container T15: primers consisting of sequences SEQ ID NOs: 20-21 and probes consisting of sequences SEQ ID NOs: 42-43,
wherein the probes are labelled with a fluorescent label.

3. The method according to any of the claim 1-2, wherein the battery of containers additionally comprises one or more of the following containers:
container T12: primers consisting of sequences SEQ ID NOs: 8-9 and probes consisting of sequences SEQ ID NOs: 36-37,
container T16: primers consisting of sequences SEQ ID NOs: 24-25 and probes consisting of sequences SEQ ID NOs: 46-47,
container T17: primers consisting of sequences SEQ ID NOs: 26-27 and probes consisting of sequences SEQ ID NOs: 36-37, and
container T18: primers consisting of sequences SEQ ID NOs: 15 and 27 and probes consisting of sequences SEQ ID NOs: 36 and 50,
wherein the probes are labelled with a fluorescent label.

4. The method according to any of the claims 1-3, wherein two primers for β-globin gene consisting of SEQ ID NOs: 28-29 are added as a control in step (i).

5. The method according to claim 4, wherein the probes consisting of sequences SEQ ID NOs: 52-53 are also added in order to detect the amplification of the β-globin gene.

6. The method according to any of the claims 1-5, wherein two primers for SCYA4 gene consisting of SEQ ID NOs: 30-31 are added as a control in step (i).

7. The method according to claim 6, wherein the probes consisting of sequences SEQ ID NOs: 54-55 are also added in order to detect the amplification of the SCYA4 gene.

8. The method according to any of the claims 1-7, wherein the probes are fluorescently labelled with fluorochromes of the donor label-acceptor label type.

9. A kit for genotyping the alleles of the human leukocyte antigen B locus (HLA-B) from a nucleic acid sample, which comprises the fluorescence pattern, the primers and the probes as defined in claim 1.

10. The kit according to claim 9, which additionally comprises one or more of the following combinations of primers and probes:
primers consisting of sequences SEQ ID NOs: 1-3 and probes consisting of sequences SEQ ID NOs: 32-33,
primers consisting of sequences SEQ ID NOs: 1-3 and probes consisting of sequences SEQ ID NOs: 38-39,
primers consisting of sequences SEQ ID NOs: 10-12 and probes consisting of sequences SEQ ID NOs: 40-41,
primers consisting of sequences SEQ ID NOs: 13-14 and probes consisting of sequences SEQ ID NOs: 32-33;
primers consisting of sequences SEQ ID NOs: 4-7 and probes consisting of sequences SEQ ID NOs: 34-35,
primers consisting of sequences SEQ ID NOs: 22-23 and probes consisting of sequences SEQ ID NOs: 33 and 51, and
primers consisting of sequences SEQ ID NOs: 20-21 and probes consisting of sequences SEQ ID NOs: 42-43.

11. The kit according to any of the claims 9-10, which additionally comprises one or more of the following combinations of primers and probes:
primers consisting of sequences SEQ ID NOs: 8-9 and probes consisting of sequences SEQ ID NOs: 36-37,
primers consisting of sequences SEQ ID NOs: 24-25 and probes consisting of sequences SEQ ID NOs: 46-47,
primers consisting of sequences SEQ ID NOs: 26-27 and probes consisting of sequences SEQ ID NOs: 36-37, and
primers consisting of sequences SEQ ID NOs: 15 and 27 and probes consisting of sequences SEQ ID NOs: 36 and 50.

12. The kit according to any of the claims 9-11, which additionally comprises two primers for β-globin gene consisting of SEQ ID NOs: 28-29.

13. The kit according to claim 12, which additionally comprises probes consisting of sequences SEQ ID NOs: 52-53 to detect the amplification of the β-globin gene.

14. The kit according to any of the claims 9-13, which additionally comprises two primers for SCYA4 gene consisting of SEQ ID NOs: 30-31.

15. The kit according to claim 14, which additionally comprises probes consisting of sequences SEQ ID NOs: 54-55 to detect the amplification of the SCYA4 gene.

## Patentansprüche

1. Verfahren zur Genotypisierung von Allelen des humanen Leukozyten-Antigen B Locus (HLA-B) aus einer Nukleinsäureprobe, die folgende Schritte umfasst:
(i) Amplifikation der Nukleinsäure der Probe durch Echtzeit-Polymerase-Kettenreaktion (PCR) mit einer Gruppe von Containern, wobei sich die Gruppe aus folgenden Containern zusammensetzt:
Container T1: Primer bestehend aus den Sequenzen mit SEQ-ID-Nrn. 15-16 und Proben bestehend aus den Sequenzen mit SEQ-ID-Nr. 46-47,
Container T2: Primer bestehend aus den Sequenzen mit SEQ-ID-Nrn. 15-16 und Proben bestehend aus den Sequenzen mit SEQ-ID-Nr. 48-49,
Container T3: Primer bestehend aus den Sequenzen mit SEQ-ID-Nrn. 15-16 und Proben bestehend aus den Sequenzen mit SEQ-ID-Nr. 42-43,
Container T4: Primer bestehend aus den Sequenzen mit SEQ-ID-Nrn. 15-16 und Proben bestehend aus den Sequenzen mit SEQ-ID-Nr. 44-45,
Container T5: Primer bestehend aus den Sequenzen mit SEQ-ID-Nrn. 15-16 und Proben bestehend aus den Sequenzen mit SEQ-ID-Nr. 36 und 50,
Container T6: Primer bestehend aus den Sequenzen mit SEQ-ID-Nrn. 17-18 und Proben bestehend aus den Sequenzen mit SEQ-ID-Nr. 40-41 und
Container T7: Primer bestehend aus den Sequenzen mit SEQ-ID-Nrn. 18-19 und Proben bestehend aus den Sequenzen mit SEQ-ID-Nr. 40-41,
wobei die Proben fluoreszenzmarkiert werden;
(ii) Erfassen der Fluoreszenz-Signale der Proben nach der in Schritt (i) durchgeführten Amplifikation und Schmelztemperaturanalyse der auf die Sequenzen von amplifizierter Nukleinsäure hybridisierten Proben; und
(iii) Vergleich des sich aus der Kombination aller Schmelztemperaturen aus Schritt (ii) ergebenden Musters mit jeder der in Tabelle 6 dargestellten Reihen, die einem Muster von Schmelztemperaturen entsprechen,
so dass durch Übereinstimmung des erhaltenen Schmelztemperaturmusters mit einem Muster aus einer der Tabellenreihen das Allel bestimmt wird, das in der ersten Spalte dieser Reihe definiert ist.

2. Verfahren nach Anspruch 1, wobei die Probengruppe zusätzlich einen oder mehrere der folgenden Probencontainer beinhaltet:
Container T8: Primer bestehend aus den Sequenzen mit SEQ-ID-Nrn. 1-3 und Proben bestehend aus den Sequenzen mit SEQ-ID-Nr. 32-33,
Container T9: Primer bestehend aus den Sequenzen mit SEQ-ID-Nrn. 1-3 und Proben bestehend aus den Sequenzen mit SEQ-ID-Nr. 38-39,
Container T10: Primer bestehend aus den Sequenzen mit SEQ-ID-Nrn. 10-12 und
Proben bestehend aus den Sequenzen mit SEQ-ID-Nr. 40-41,
Container T11: Primer bestehend aus den Sequenzen mit SEQ-ID-Nrn. 13-14 und
Proben bestehend aus den Sequenzen mit SEQ-ID-Nr. 32-33;
Container T13: Primer bestehend aus den Sequenzen mit SEQ-ID-Nrn. 4-7 und Proben bestehend aus den Sequenzen mit SEQ-ID-Nr. 34-35,
Container T14: Primer bestehend aus den Sequenzen mit SEQ-ID-Nrn. 22-23 und
Proben bestehend aus den Sequenzen mit SEQ-ID-Nr. 33 und 51 und
Container T15: Primer bestehend aus den Sequenzen mit SEQ-ID-Nrn. 20-21 und
Proben bestehend aus den Sequenzen mit SEQ-ID-Nr. 42-43,
wobei die Proben fluoreszenzmarkiert werden.

3. Verfahren nach einem der Ansprüche 1-2, wobei die Probengruppe zusätzlich einen oder mehrere der folgenden Probencontainer beinhaltet:
Container T12: Primer bestehend aus den Sequenzen mit SEQ-ID-Nrn. 8-9 und Proben bestehend aus den Sequenzen mit SEQ-ID-Nr. 36-37,
Container T16: Primer bestehend aus den Sequenzen mit SEQ-ID-Nrn. 24-25 und Proben bestehend aus den Sequenzen mit SEQ-ID-Nr. 46-47,
Container T17: Primer bestehend aus den Sequenzen mit SEQ-ID-Nrn. 26-27 und Proben bestehend aus den Sequenzen mit SEQ-ID-Nr. 36-37 und
Container T18: Primer bestehend aus den Sequenzen mit SEQ-ID-Nrn. 15 und 27 und Proben bestehend aus den Sequenzen mit SEQ-ID-Nr. 36 und 50,
wobei die Proben fluoreszenzmarkiert werden.

4. Verfahren nach einem der Ansprüche 1-3, wobei zwei Primer für das β-Globin-Gen bestehend aus den SEQ-ID-Nrn. 28-29 zur Kontrolle in Schritt (i) hinzugefügt werden.

5. Verfahren nach Anspruch 4, wobei die Proben bestehend aus den SEQ-ID-Nrn. 52-53 ebenfalls hinzugefügt werden, um die Amplifikation des β-Globin-Gens zu erfassen.

6. Verfahren nach einem der Ansprüche 1-5, wobei zwei Primer für das SCYA4-Gen bestehend aus den SEQ-ID-Nrn. 30-31 zur Kontrolle in Schritt (i) hinzugefügt werden.

7. Verfahren nach Anspruch 6, wobei die Proben bestehend aus den SEQ-ID-Nrn. 54-55 ebenfalls hinzugefügt werden, um die Amplifikation des SCYA4-Gens zu erfassen.

8. Verfahren nach einem der Ansprüche 1-7, wobei die Proben mit Fluorochromen des Typs Spendermarkierung/Empfängermarkierung fluoreszenzmarkiert werden.

9. Kit zur Genotypisierung der Allele des humanen Leukozyten-Antigen B-Locus (HLA-B) aus einer Nukleinsäureprobe, das Fluoreszenzmuster, Primer und Proben wie nach Anspruch 1 definiert umfasst.

10. Kit nach Anspruch 9, das zusätzlich eine oder mehrere der folgenden Kombinationen aus Primern und Proben umfasst:
Primer bestehend aus den Sequenzen mit SEQ-ID-Nrn. 1-3 und Proben bestehend aus den Sequenzen mit SEQ-ID-Nr. 32-33,
Primer bestehend aus den Sequenzen mit SEQ-ID-Nrn. 1-3 und Proben bestehend aus den Sequenzen mit SEQ-ID-Nr. 38-39,
Primer bestehend aus den Sequenzen mit SEQ-ID-Nrn. 10-12 und Proben bestehend aus den Sequenzen mit SEQ-ID-Nr. 40-41,
Primer bestehend aus den Sequenzen mit SEQ-ID-Nrn. 13-14 und Proben bestehend aus den Sequenzen mit SEQ-ID-Nr. 32-33;
Primer bestehend aus den Sequenzen mit SEQ-ID-Nrn. 4-7 und Proben bestehend aus den Sequenzen mit SEQ-ID-Nr. 34-35,
Primer bestehend aus den Sequenzen mit SEQ-ID-Nrn. 22-23 und Proben bestehend aus den Sequenzen mit SEQ-ID-Nr. 33 und 51 und
Primer bestehend aus den Sequenzen mit SEQ-ID-Nrn. 20-21 und Proben bestehend aus den Sequenzen mit SEQ-ID-Nr. 42-43.

11. Kit nach einem der Ansprüche 9-10, das zusätzlich eine oder mehrere der folgenden Kombinationen aus Primern und Proben umfasst:
Primer bestehend aus den Sequenzen mit SEQ-ID-Nrn. 8-9 und Proben bestehend aus den Sequenzen mit SEQ-ID-Nr. 36-37,
Primer bestehend aus den Sequenzen mit SEQ-ID-Nrn. 24-25 und Proben bestehend aus den Sequenzen mit SEQ-ID-Nr. 46-47,
Primer bestehend aus den Sequenzen mit SEQ-ID-Nrn. 26-27 und Proben bestehend aus den Sequenzen mit SEQ-ID-Nr. 36-37 und
Primer bestehend aus den Sequenzen mit SEQ-ID-Nrn. 15 und 27 und Proben bestehend aus den Sequenzen mit SEQ-ID-Nr. 36 und 50.

12. Kit nach einem der Ansprüche 9-11, das zusätzlich zwei Primer für das β-Globin-Gen bestehend aus den SEQ-ID-Nrn. 28-29 umfasst.

13. Kit nach Anspruch 12, das zusätzlich Proben bestehend aus den SEQ-ID-Nrn. 52-53 zur Erfassung der Amplifikation des β-Globin-Gens umfasst.

14. Kit nach einem der Ansprüche 9-13, die zusätzlich zwei Primer für das SCYA4-Gen bestehend aus den SEQ-ID-Nrn. 30-31 umfasst.

15. Kit nach Anspruch 14, das zusätzlich Proben bestehend aus den SEQ-ID-Nrn. 54-55 zur Erfassung der Amplifikation des SCYA4-Gens umfasst.

## Revendications

1. Procédé destiné au génotypage des allèles du locus d'antigène d'histocompatibilité humain B (HLA-B) à partir d'un échantillon d'acide nucléique, qui comprend les étapes suivantes :
(i) une amplification de l'acide nucléique de l'échantillon par réaction d'amplification en chaîne par polymérase (PCR) en temps réel avec une batterie de conteneurs, la batterie comprenant les conteneurs suivants :
conteneur T1 : amorces constituées des séquences SEQ ID No. : 15 - 16 et sondes constituées des séquences SEQ ID No. : 46 - 47,
conteneur T2 : amorces constituées des séquences SEQ ID No. : 15 - 16 et sondes constituées des séquences SEQ ID No. : 48 - 49,
conteneur T3 : amorces constituées des séquences SEQ ID No. : 15 - 16 et sondes constituées des séquences SEQ ID No. : 42 - 43,
conteneur T4 : amorces constituées des séquences SEQ ID No. : 15 - 16 et sondes constituées des séquences SEQ ID No. : 44 - 45,
conteneur T5 : amorces constituées des séquences SEQ ID No. : 15 - 16 et sondes constituées des séquences SEQ ID No. : 36 et 50,
conteneur T6 : amorces constituées des séquences SEQ ID No. : 17 - 18 et sondes constituées des séquences SEQ ID No. : 40 - 41, et
conteneur T7 : amorces constituées des séquences SEQ ID No. : 18 - 19 et sondes constituées des séquences SEQ ID No. : 40 - 41,
dans lequel les sondes sont marquées avec un marqueur fluorescent ;
(ii) une détection de signaux fluorescents des sondes après l'amplification effectuée dans l'étape (i), et une analyse des températures de fusion des sondes hybridées aux séquences d'acide nucléique amplifié ; et
(iii) une comparaison du motif résultant de la combinaison de toutes les températures de fusion obtenues dans l'étape (ii) à chaque ligne décrite dans le Tableau 6 correspondant à un motif de températures de fusion, de sorte que, lorsque le motif obtenu de températures de fusion correspond au motif d'une ligne du TABLEAU 6, l'allèle spécifié dans la première colonne de la ligne est déterminé.

2. Procédé selon la revendication 1, dans lequel la batterie de conteneurs comprend en outre un ou plusieurs des conteneurs suivants :
conteneur T8 : amorces constituées des séquences SEQ ID No. : 1 - 3 et sondes constituées des séquences SEQ ID No. : 32 - 33,
conteneur T9 : amorces constituées des séquences SEQ ID No. : 1 - 3 et sondes constituées des séquences SEQ ID No. : 38 - 39,
conteneur T10 : amorces constituées des séquences SEQ ID No. : 10 - 12 et sondes constituées des séquences SEQ ID No. : 40 - 41,
conteneur T11 : amorces constituées des séquences SEQ ID No. : 13 - 14 et sondes constituées des séquences SEQ ID No. : 32 - 33 ;
conteneur T13 : amorces constituées des séquences SEQ ID No. : 4 - 7 et sondes constituées des séquences SEQ ID No. : 34 - 35,
conteneur T14 : amorces constituées des séquences SEQ ID No. : 22 - 23 et sondes constituées des séquences SEQ I D No. : 33 et 51, et
conteneur T15 : amorces constituées des séquences SEQ ID No. : 20 - 21 et sondes constituées des séquences SEQ ID No. : 42 - 43,
dans lequel les sondes sont marquées avec un marqueur fluorescent ;

3. Procédé selon l'une quelconque des revendications 1 à 2, dans lequel la batterie de conteneurs comprend en outre un ou plusieurs des conteneurs suivants :
conteneur T12 : amorces constituées des séquences SEQ ID No. : 8 - 9 et sondes constituées des séquences SEQ ID No. : 36 - 37,
conteneur T16 : amorces constituées des séquences SEQ ID No. : 24 - 25 et sondes constituées des séquences SEQ ID No. : 46 - 47,
conteneur T17 : amorces constituées des séquences SEQ ID No. : 26 - 27 et sondes constituées des séquences SEQ ID No. : 36 - 37, et
conteneur T18 : amorces constituées des séquences SEQ ID No. : 15 et 27 et sondes constituées des séquences SEQ ID No. : 36 et 50,
dans lequel les sondes sont marquées avec un marqueur fluorescent ;

4. Procédé selon l'une quelconque des revendications 1 à 3, dans lequel deux amorces pour le gène de la β-globine constituées de SEQ ID No. : 28 - 29 sont ajoutées en tant que témoin dans l'étape (i).

5. Procédé selon la revendication 4, dans lequel les sondes constituées des séquences SEQ ID No. : 52 - 53 sont également ajoutées afin de détecter l'amplification du gène de la β-globine.

6. Procédé selon l'une quelconque des revendications 1 à 5, dans lequel deux amorces pour le gène SCYA4 constituées de SEQ ID No. : 30 - 31 sont ajoutées en tant que témoin dans l'étape (i).

7. Procédé selon la revendication 6, dans lequel les sondes constituées des séquences SEQ ID No. : 54 - 55 sont également ajoutées afin de détecter l'amplification du gène SCYA4.

8. Procédé selon l'une quelconque des revendications 1 à 7, dans lequel les sondes sont marquées par fluorescence avec des fluorochromes du type marqueur donneur - marqueur accepteur.

9. Kit destinée au génotypage des allèles du locus d'antigène d'histocompatibilité humain B (HLA-B) à partir d'un échantillon d'acide nucléique, qui comprend le motif de fluorescence, les amorces et les sondes tels que définis dans la revendication 1.

10. Kit selon la revendication 9, qui comprend en outre une ou plusieurs des combinaisons suivantes d'amorces et de sondes :
amorces constituées des séquences SEQ ID No. : 1 - 3 et sondes constituées des séquences SEQ ID No. : 32 - 33,
amorces constituées des séquences SEQ ID No. : 1 - 3 et sondes constituées des séquences SEQ ID No. : 38 - 39,
amorces constituées des séquences SEQ ID No. : 10 - 12 et sondes constituées des séquences SEQ ID No. : 40 - 41,
amorces constituées des séquences SEQ ID No. : 13 - 14 et sondes constituées des séquences SEQ ID No. : 32 - 33 ;
amorces constituées des séquences SEQ ID No. : 4 - 7 et sondes constituées des séquences SEQ ID No. : 34 - 35,
amorces constituées des séquences SEQ ID No. : 22 - 23 et sondes constituées des séquences SEQ ID No. : 33 et 51, et
amorces constituées des séquences SEQ ID No. : 20 - 21 et sondes constituées des séquences SEQ ID No. : 42 - 43.

11. Kit selon l'une quelconque des revendications 9 à 10, qui comprend en outre une ou plusieurs des combinaisons suivantes d'amorces et de sondes :
amorces constituées des séquences SEQ ID No. : 8 - 9 et sondes constituées des séquences SEQ ID No. : 36 - 37,
amorces constituées des séquences SEQ ID No. : 24 - 25 et sondes constituées des séquences SEQ ID No. : 46 - 47,
amorces constituées des séquences SEQ ID No. : 26 - 27 et sondes constituées des séquences SEQ ID No. : 36 - 37, et
amorces constituées des séquences SEQ ID No. : 15 et 27 et sondes constituées des séquences SEQ ID No. : 36 et 50.

12. Kit selon l'une quelconque des revendications 9 à 11, qui comprend en outre deux amorces pour le gène de la β-globine constituées de SEQ ID No. : 28 - 29.

13. Kit selon la revendication 12, qui comprend en outre des sondes constituées des séquences SEQ ID No. : 52 - 53 pour détecter l'amplification du gène de la β-globine.

14. Kit selon l'une quelconque des revendications 9 à 13, qui comprend en outre deux amorces pour le gène SCYA4 constituées de SEQ ID No. : 30 - 31.

15. Kit selon la revendication 14, qui comprend en outre des sondes constituées des séquences SEQ ID No. : 54 - 55 pour détecter l'amplification du gène SCYA4.


### SEQUENCE LISTING

<110> Universitat Autònoma de Barcelona / Banc de Sang i de Teixits
<120> Method and kit for HLA-B genotyping based on real-time PCR
<130> P612EP00 HLA-B
<150> ES200503110
   <151> 2005-12-13
<150> PCT/ES2006/070192
   <151> 2006-12-12
<160> 55
<170> PatentIn version 3.5
<210> 1
   <211> 17
   <212> DNA
   <213> Homo sapiens
<400> 1
   gccagggtct cacaccc 17
<210> 2
   <211> 16
   <212> DNA
   <213> Homo sapiens
<400> 2
   cggcggtcca ggagct 16
<210> 3
   <211> 16
   <212> DNA
   <213> Homo sapiens
<400> 3
   ccgcggtcca ggagct 16
<210> 4
   <211> 17
   <212> DNA
   <213> Homo sapiens
<400> 4
   ggagccccgc ttcatct 17
<210> 5
   <211> 19
   <212> DNA
   <213> Homo sapiens
<400> 5
   ggtcagtctg tgtgttggt 19
<210> 6
   <211> 20
   <212> DNA
   <213> Homo sapiens
<400> 6
   cggtaagtct gtgtgttggt 20
<210> 7
   <211> 19
   <212> DNA
   <213> Homo sapiens
<400> 7
   ggtaagtccg tgtgttggt 19
<210> 8
   <211> 19
   <212> DNA
   <213> Homo sapiens
<400> 8
   aggtatttct acaccgcca 19
<210> 9
   <211> 17
   <212> DNA
   <213> Homo sapiens
<400> 9
   cggtaagtct gcgcgga 17
<210> 10
   <211> 19
   <212> DNA
   <213> Homo sapiens
<400> 10
   gtccagggtc tcacacttg 19
<210> 11
   <211> 17
   <212> DNA
   <213> Homo sapiens
<400> 11
   agccactcca cgcacag 17
<210> 12
   <211> 16
   <212> DNA
   <213> Homo sapiens
<400> 12
   gcccctccac gcacag 16
<210> 13
   <211> 19
   <212> DNA
   <213> Homo sapiens
<400> 13
   gtctcacacc ctccagagc 19
<210> 14
   <211> 15
   <212> DNA
   <213> Homo sapiens
<400> 14
   gcgcgctcca gcttg 15
<210> 15
   <211> 22
   <212> DNA
   <213> Homo sapiens
<400> 15
   gggaggagcg aggggaccsc ag 22
<210> 16
   <211> 17
   <212> DNA
   <213> Homo sapiens
<400> 16
   tmcgtggggg atgggga 17
<210> 17
   <211> 16
   <212> DNA
   <213> Homo sapiens
<400> 17
   cgcctcctcc gcgggc 16
<210> 18
   <211> 23
   <212> DNA
   <213> Homo sapiens
<400> 18
   ggaggccatc cccggcgacc tat 23
<210> 19
   <211> 18
   <212> DNA
   <213> Homo sapiens
<400> 19
   ccctgaacga ggacctga 18
<210> 20
   <211> 17
   <212> DNA
   <213> Homo sapiens
<400> 20
   ggagccccgc ttcatca 17
<210> 21
   <211> 17
   <212> DNA
   <213> Homo sapiens
<400> 21
   ctgtgccttg gccttgc 17
<210> 22
   <211> 17
   <212> DNA
   <213> Homo sapiens
<400> 22
   ccgggtctca cacttgg 17
<210> 23
   <211> 17
   <212> DNA
   <213> Homo sapiens
<400> 23
   agccactcca cgcacgt 17
<210> 24
   <211> 21
   <212> DNA
   <213> Homo sapiens
<400> 24
   cacacagact taccgagaga a 21
<210> 25
   <211> 19
   <212> DNA
   <213> Homo sapiens
<400> 25
   gccatacatc gtctgccaa 19
<210> 26
   <211> 18
   <212> DNA
   <213> Homo sapiens
<400> 26
   ggctacgtgg acgacacc 18
<210> 27
   <211> 20 <212> DNA
   <213> Homo sapiens
<400> 27
   gctctggttg tagtagcgga 20
<210> 28
   <211> 20
   <212> DNA
   <213> Homo sapiens
<400> 28
   acacaactgt gttcactagc 20
<210> 29
   <211> 20
   <212> DNA
   <213> Homo sapiens
<400> 29
   caacttcatc cacgttcacc 20
<210> 30
   <211> 21
   <212> DNA
   <213> Homo sapiens
<400> 30
   gcagaggaag atgcctacca c 21
<210> 31
   <211> 19
   <212> DNA
   <213> Homo sapiens
<400> 31
   ggcagaggaa gatgcaaag 19
<210> 32
   <211> 20
   <212> DNA
   <213> Homo sapiens
<400> 32
   cgcgtataac cagttcgcct 20
<210> 33
   <211> 24
   <212> DNA
   <213> Homo sapiens
<400> 33
   cgacggcaag gattacatcg ccct 24
<210> 34
   <211> 17
   <212> DNA
   <213> Homo sapiens
<400> 34
   gccggaatat tgggacc 17
<210> 35
   <211> 20
   <212> DNA
   <213> Homo sapiens
<400> 35
   gccgtggata gagcaggagg 20
<210> 36
   <211> 20
   <212> DNA
   <213> Homo sapiens
<400> 36
   tgaggttcga cagcgacgcc 20
<210> 37
   <211> 17
   <212> DNA
   <213> Homo sapiens
<400> 37
   gagtccgagg atggcgc 17
<210> 38
   <211> 19
   <212> DNA
   <213> Homo sapiens
<400> 38
   cagaggatgt acggctgcg 19
<210> 39
   <211> 15
   <212> DNA
   <213> Homo sapiens
<400> 39
   cgtggggccg gacgg 15
<210> 40
   <211> 19
   <212> DNA
   <213> Homo sapiens
<400> 40
   ccggtgaggc ggagcagct 19
<210> 41
   <211> 23
   <212> DNA
   <213> Homo sapiens
<400> 41
   agagcctacc tggagggcct gtg 23
<400> 42
   gctacgtgga cgacacccag t 21
<210> 42
   <211> 21
   <212> DNA
   <213> Homo sapiens
<210> 43
   <211> 23
   <212> DNA
   <213> Homo sapiens
<400> 43
   cgtgaggttc gacagcgacg ccg 23
<210> 44
   <211> 18
   <212> DNA
   <213> Homo sapiens
<400> 44 18
   cactgcgatg aagcgggg 18
<210> 45
   <211> 24
   <212> DNA
   <213> Homo sapiens
<400> 45
   gcaactgggt gtcgtccacg tagc 24
<210> 46
   <211> 25
   <212> DNA
   <213> Homo sapiens
<400> 46
   ggaacctgcg cccctactac aacca 25
<210> 47
   <211> 24
   <212> DNA
   <213> Homo sapiens
<400> 47
   agcgaggccg gtgagtgacc ccgg 24
<210> 48
   <211> 21
   <212> DNA
   <213> Homo sapiens
<400> 48
   gtgtgttggt cttgaagatc t 21
<210> 49
   <211> 21
   <212> DNA
   <213> Homo sapiens
<400> 49
   gtgttccggt cccaatactc c 21
<210> 50
   <211> 19
   <212> DNA
   <213> Homo sapiens
<400> 50
   cgagtccgag agaggagcc 19
<210> 51
   <211> 20
   <212> DNA
   <213> Homo sapiens
<400> 51
   gggcatgacc agtacgcctv 20
<210> 52
   <211> 29
   <212> DNA
   <213> Homo sapiens
<400> 52
   aagtctgccg ttactgccct gtggggcaa 29
<210> 53
   <211> 35
   <212> DNA
   <213> Homo sapiens
<400> 53
   caaacagaca ccatggtgca cctgactcct gagga 35
<210> 54
   <211> 26
   <212> DNA
   <213> Homo sapiens
<400> 54
   tcagcacaga cttgcttgct tctttt 26
<210> 55
   <211> 23
   <212> DNA
   <213> Homo sapiens
<400> 55
   tttggaatct gtagaacaag gag 23